# EUROPEAN PATENT APPLICATION

(11) **EP 4 643 877 A1**
(43) Date of publication of application: **05.11.2025**
(21) Application number: 23910820.2
(22) Date of filing: 28.12.2023
(51) Int. Cl.: A61K 39/395, C07K 16/28, A61P 37/00

(54) **COMPOSITION OF CD40-BINDING MOLECULE AND PHARMACEUTICAL USE THEREOF**

(30) Priority: 28.12.2022 CN 202211698820
(71) Applicant: Suzhou Suncadia Biopharmaceuticals Co., Ltd., Suzhou, Jiangsu 215126 (CN); Jiangsu Hengrui Pharmaceuticals Co., Ltd., Lianyungang, Jiangsu 222047 (CN)
(72) Inventor: YANG, Zhen, Suzhou, Jiangsu 215126 (CN); YANG, Xiqin, Suzhou, Jiangsu 215126 (CN); GE, Lingxiao, Suzhou, Jiangsu 215126 (CN); WANG, Hongwei, Suzhou, Jiangsu 215126 (CN); JIANG, Tiantian, Suzhou, Jiangsu 215126 (CN)
(74) Representative: Potter Clarkson
(86) International application number: PCT/CN2023/142688
(87) International publication number: WO 2024/140903

(57) **Abstract**

A composition of a CD40-binding molecule and pharmaceutical use thereof. Specifically, the composition comprises an anti-CD40 antibody or an antigen-binding fragment thereof or an antibody-drug conjugate and a buffer.

## Description

The present application claims priority to Chinese Patent Application No. 202211698820.3 filed on Dec. 28, 2022.

### TECHNICAL FIELD

The present disclosure relates to the field of pharmaceutical formulations, in particular to a pharmaceutical composition comprising a CD40-binding molecule and pharmaceutical use thereof.

### BACKGROUND

CD40, which belongs to the tumor necrosis factor receptor (TNFR) superfamily, is a type I transmembrane glycoprotein localized on the cell membrane surface, has a molecular weight of about 48 kDa, and plays an important role in the immune system. CD40 is expressed in a variety of immune cells, such as B cells, dendritic cells, monocytes, and macrophages, as well as on platelets, and can be expressed on eosinophils and parenchymal cells under certain conditions. The natural ligand for CD40 is CD154 or CD40L, a type II transmembrane protein, the expression of which can be induced on a variety of cell types, including activated CD4+ T cells, NK cells, platelets, and B cells (Pucino V et al., 2020).

CD40L, upon binding to CD40, can recruit TRAFs and mediate downstream signaling through the NF-kB, JNK, and MAPK pathways, producing a variety of cell type-dependent activation outcomes, including activation and proliferation of immune cells, secretion of inflammatory factors and chemokines, and the like. (Vonderheide RH et al., 2007). For example, signaling through these pathways is necessary for several important effector functions of the adaptive immune system, including primary T cell-dependent antibody responses (TDARs), B cell proliferation, germinal center (GC) formation, immunoglobulin (Ig) isotype switching, somatic mutation, and differentiation of memory B cells and plasma cells (Foy TM et al., 1993; Foy TM et al., 1994). In addition to the effects on B cells, CD40 pathway activation provides important signals for DC maturation and function, as well as the survival of monocytes and macrophages and cytokine secretion (Caux, C et al., 1994).

Dysregulation of the CD40 signaling pathway can lead to autoimmune diseases (Kamell JL et al., 2018). The CD40-CD40L signaling pathway has been found to be involved in the function of parenchymal cells in inflamed tissues: activated epithelial cells from sites such as the kidney, salivary glands, and skin that can secrete chemokines are able to respond to CD40. Moreover, the expression levels of CD40 or CD40L are elevated in the lesion sites of patients with atherosclerosis and in preclinical models of atherosclerosis. CD40 can stimulate and induce the expression of matrix-degrading enzymes, promoting the expression of tissue factors in cell types related to the pathogenesis of atherosclerosis, such as endothelial cells, smooth muscle cells, and macrophages (Michel NA et al., 2017). The CD40 pathway up-regulates the production of inflammatory factors such as IL-1, IL-6, and IL-8, as well as adhesion molecules including intercellular adhesion molecule-1 (ICAM-1), E-selectin, and vascular cell adhesion molecule (VCAM). The CD40/CD40L interaction has also been used to prevent graft rejection. In a study of renal allografts in cynomolgus monkeys, the use of chimeric anti-CD40 antagonist ch5D12 demonstrated that CD40 antagonism was sufficient to improve the condition and extend the average survival time to over 100 days. When ch5D12 was combined with an anti-CD86 antibody and given only at the start of the allograft study, followed by extended treatment with cyclosporine, an average survival time of over 4 years was achieved, suggesting that such a combination can potentially induce immune tolerance (Haanstra et al., 2005). Numerous preclinical studies provide evidence for the key role of the CD40/CD40L interaction in promoting T cell-dependent immune responses. Therefore, blocking CD40 signaling is considered a suitable and desirable therapeutic strategy for suppressing pathogenic autoimmune responses in diseases such as rheumatoid arthritis, systemic lupus erythematosus, and Sjögren's syndrome. Currently, no anti-CD40 antibodies have been approved as treatments for such diseases. Thus, there is still an urgent need in the art for therapeutic agents that can intervene in the CD40-CD40L interaction and block CD40 signaling.

The patent application WO2023274201 filed by the applicants provides an anti-CD40 antibody with a novel structure, which is capable of specifically binding to CD40 and can be used for intervening in or treating diseases related to the CD40 signaling pathway.

Antibody drugs are an important class of biological drugs with large molecular weights and complex structures, and are prone to degradation and aggregation due to physical or chemical influences during production, storage, and use, leading to reduced activity or even loss of efficacy. Therefore, it is of great significance to develop an excellent antibody formulation.

### SUMMARY

The present disclosure provides a pharmaceutical composition comprising a CD40-binding molecule, and the composition has excellent stability.

The present disclosure provides a pharmaceutical composition comprising a CD40-binding molecule and a buffer, wherein the buffer is selected from the group consisting of a tris(hydroxymethyl)aminomethane (Tris) buffer, an acetate buffer, a succinate buffer, a phosphate buffer, a histidine salt buffer, an acetate buffer, a citrate buffer, a tartrate buffer, a fumarate buffer, a glycylglycine buffer, and a citrate buffer. In certain embodiments, the buffer is selected from the group consisting of an acetate buffer, a citrate buffer, a histidine salt buffer, and a phosphate buffer. In certain embodiments, the buffer is a histidine salt buffer. In certain embodiments, the histidine salt buffer is selected from the group consisting of a histidine-hydrochloride buffer and a histidine-acetate buffer. In certain embodiments, the buffer is a histidine-hydrochloride buffer. In certain embodiments, the buffer is a histidine-histidine hydrochloride buffer.

### Anti-CD40 Antibody or Antigen-Binding Fragment Thereof

In one aspect, the CD40-binding molecule of the present disclosure is an anti-CD40 antibody or an antigen-binding fragment thereof.

In one aspect, the present disclosure provides a pharmaceutical composition comprising an anti-CD40 antibody or an antigen-binding fragment thereof, and the composition has excellent stability.

The present disclosure provides a pharmaceutical composition comprising an anti-CD40 antibody or an antigen-binding fragment thereof and a buffer, wherein the buffer is selected from the group consisting of a tris(hydroxymethyl)aminomethane (Tris) buffer, an acetate buffer, a succinate buffer, a phosphate buffer, a histidine salt buffer, an acetate buffer, a citrate buffer, a tartrate buffer, a fumarate buffer, a glycylglycine buffer, and a citrate buffer. In certain embodiments, the buffer is selected from the group consisting of an acetate buffer, a citrate buffer, a histidine salt buffer, and a phosphate buffer. In certain embodiments, the buffer is a histidine salt buffer. In certain embodiments, the histidine salt buffer is selected from the group consisting of a histidine-hydrochloride buffer and a histidine-acetate buffer. In certain embodiments, the buffer is a histidine-hydrochloride buffer. In certain embodiments, the buffer is a histidine-histidine hydrochloride buffer.

In the aforementioned pharmaceutical composition of the present disclosure, the anti-CD40 antibody and the antigen-binding fragment thereof comprise:
a heavy chain HCDR1, a heavy chain HCDR2, and a heavy chain HCDR3, comprising the sequences set forth in SEQ ID NOs: 3, 4, and 5, respectively; and/or a light chain LCDR1, comprising the sequence set forth in SEQ ID NO: 6; a light chain LCDR2, comprising the sequence set forth in SEQ ID NO: 7; and a light chain LCDR3, comprising the sequence QGGYWTSTSNFGX₉X₁₀ (SEQ ID NO: 19), wherein X₉ is selected from the group consisting of N, S, T, and Q, and X₁₀ is selected from the group consisting of V and G.

In certain embodiments, the aforementioned anti-CD40 antibody or antigen-binding fragment thereof comprises:
a heavy chain HCDR1, a heavy chain HCDR2, and a heavy chain HCDR3, comprising the sequences set forth in SEQ ID NOs: 3, 4, and 5, respectively; a light chain LCDR1, comprising the sequence set forth in SEQ ID NO: 6; a light chain LCDR2, comprising the sequence set forth in SEQ ID NO: 7; and a light chain LCDR3, comprising the sequence set forth in any one of SEQ ID NOs: 15-18.

In certain embodiments, the aforementioned anti-CD40 antibody or antigen-binding fragment thereof comprises a heavy chain variable region (VH) and a light chain variable region (VL), wherein:
the VH comprises a HCDR1, a HCDR2, and a HCDR3 in a VH set forth in SEQ ID NO: 1, and the VL comprises a LCDR1, a LCDR2, and a LCDR3 in a VL set forth in SEQ ID NO: 2;
wherein the CDRs are defined according to the Kabat, IMGT, Chothia, AbM, or Contact numbering scheme. In certain embodiments, the CDRs are defined according to the Kabat numbering scheme. In certain embodiments, the aforementioned anti-CD40 antibody or antigen-binding fragment thereof is a recombinant antibody.

In certain embodiments, the aforementioned anti-CD40 antibody or antigen-binding fragment thereof is a rabbit antibody, a chimeric antibody, a humanized antibody, a human antibody, or an antigen-binding fragment thereof.

In certain embodiments, when the aforementioned anti-CD40 antibody or antigen-binding fragment thereof is a humanized antibody, heavy chain framework regions are derived from IGHV2-26*01, IGHV4-30-4*02, IGHV4-4*08, and IGHJ1*01, and/or light chain framework regions are derived from IGkV1-13*02, IGkV1-9*01, IGkV1-6*01, and IGKJ4*01. For example, heavy chain framework regions FR1-FR3 are derived from IGHV2-26*01, IGHV4-30-4*02, or IGHV4-4*08, and a heavy chain framework region FR4 is derived from IGHJ1*01; light chain framework regions FR1-FR3 are derived from IGkV1-13*02, IGkV1-9*01, or IGkV1-6*01, and a light chain framework region FR4 is derived from IGKJ4*01.

In certain embodiments, the aforementioned anti-CD40 antibody or antigen-binding fragment thereof comprises a VH and a VL, wherein
A-1) the amino acid sequence of the VH is set forth in SEQ ID NO: 1 or has at least 90% identity thereto, and the amino acid sequence of the VL is set forth in SEQ ID NO: 2 or has at least 90% identity thereto;
A-2) the amino acid sequence of the VH is set forth in SEQ ID NO: 13 or has at least 90% identity thereto, and the amino acid sequence of the VL is set forth in any one of SEQ ID NOs: 9-12 or has at least 90% identity thereto;
A-3) the amino acid sequence of the VH is set forth in SEQ ID NO: 14 or has at least 90% identity thereto, and the amino acid sequence of the VL is set forth in any one of SEQ ID NOs: 9-12 or has at least 90% identity thereto.

In certain embodiments, the aforementioned anti-CD40 antibody or antigen-binding fragment thereof is an IgG antibody or an antigen-binding fragment thereof, e.g., an IgG1, IgG2, IgG2, or IgG4 antibody or an antigen-binding fragment thereof, e.g., an IgG1 antibody having the mutation N297A or an antigen-binding fragment thereof, e.g., an IgG1 antibody having one of L234A, L235A, M252Y, S254T, and T256E, or any combination thereof, or an antigen-binding fragment thereof.

In certain embodiments, the antigen-binding fragment of the aforementioned anti-CD40 antibody is a Fab, an Fv, an sFv, a Fab', a F(ab')₂, a linear antibody, a single-chain antibody, an scFv, an sdAb, an sdFv, a nanobody, a peptibody, a domain antibody, and a multispecific antibody (bispecific antibody, diabody, triabody, and tetrabody, tandem di-scFv, tandem tri-scFv), for example, an scFv, Fv, Fab, or Fab' fragment.

In certain embodiments, the full-length amino acid sequence of a heavy chain of the antigen-binding fragment of the aforementioned anti-CD40 antibody is set forth in SEQ ID NO: 20 or 22 or has at least 90% identity thereto, and the full-length amino acid sequence of a light chain is set forth in SEQ ID NO: 21 or has at least 90% identity thereto.

As described above, "at least 90% identity" includes, for example, at least 91%, at least 92%, at least 93%, at least 94%, at least 95%, at least 96%, at least 97%, at least 98%, and at least 99% identity. In certain embodiments, the heavy chain variable region of the aforementioned anti-CD40 antibody or antigen-binding fragment thereof has 0 to 10 (1, 2, 3, 4, 5, 6, 7, 8, 9, or 10) amino acid alterations, and the light chain variable region has 0 to 10 (1, 2, 3, 4, 5, 6, 7, 8, 9, or 10) amino acid alterations. In some specific embodiments, the amino acid alterations are conservative replacements, substitutions, or modifications, and/or deletions or additions that do not affect function.

### Antibody-Drug Conjugate

In another aspect, the CD40-binding molecule in the pharmaceutical composition of the present disclosure is an antibody-drug conjugate comprising an anti-CD40 antibody or an antigen-binding fragment thereof, wherein the anti-CD40 antibody or the antigen-binding fragment thereof is any one of the anti-CD40 antibodies or antigen-binding fragments thereof of the present disclosure.

The present disclosure provides a pharmaceutical composition comprising an antibody-drug conjugate and a buffer, wherein the antibody-drug conjugate comprises any one of the anti-CD40 antibodies or antigen-binding fragments thereof of the present disclosure, and the buffer is selected from the group consisting of a tris(hydroxymethyl)aminomethane (Tris) buffer, an acetate buffer, a succinate buffer, a phosphate buffer, a histidine salt buffer, an acetate buffer, a citrate buffer, a tartrate buffer, a fumarate buffer, a glycylglycine buffer, and a citrate buffer. In certain embodiments, the buffer is selected from the group consisting of an acetate buffer, a citrate buffer, a histidine salt buffer, and a phosphate buffer. In certain embodiments, the buffer is a histidine salt buffer. In certain embodiments, the histidine salt buffer is selected from the group consisting of a histidine-hydrochloride buffer and a histidine-acetate buffer. In certain embodiments, the buffer is a histidine-hydrochloride buffer. In certain embodiments, the buffer is a histidine-histidine hydrochloride buffer. In certain embodiments, the aforementioned anti-CD40 antibody or antigen-binding fragment thereof comprises:
a heavy chain HCDR1, a heavy chain HCDR2, and a heavy chain HCDR3, comprising the sequences set forth in SEQ ID NOs: 3, 4, and 5, respectively; and a light chain LCDR1, comprising the sequence set forth in SEQ ID NO: 6; a light chain LCDR2, comprising the sequence set forth in SEQ ID NO: 7; and a light chain LCDR3, comprising the sequence QGGYWTSTSNFGX₉X₁₀ (SEQ ID NO: 19), wherein X₉ is selected from the group consisting of N, S, T, and Q, and X₁₀ is selected from the group consisting of V and G.

In certain embodiments, the aforementioned anti-CD40 antibody or antigen-binding fragment thereof comprises:
a heavy chain HCDR1, a heavy chain HCDR2, and a heavy chain HCDR3, comprising the sequences set forth in SEQ ID NOs: 3, 4, and 5, respectively; a light chain LCDR1, comprising the sequence set forth in SEQ ID NO: 6; a light chain LCDR2, comprising the sequence set forth in SEQ ID NO: 7; and a light chain LCDR3, comprising the sequence set forth in any one of SEQ ID NOs: 15-18.

In certain embodiments, the aforementioned anti-CD40 antibody or antigen-binding fragment thereof comprises a heavy chain variable region (VH) and a light chain variable region (VL), wherein:
the VH comprises a HCDR1, a HCDR2, and a HCDR3 in a VH set forth in SEQ ID NO: 1, and the VL comprises a LCDR1, a LCDR2, and a LCDR3 in a VL set forth in SEQ ID NO: 2;
wherein the CDRs are defined according to the Kabat, IMGT, Chothia, AbM, or Contact numbering scheme. In certain embodiments, the CDRs are defined according to the Kabat numbering scheme. In certain embodiments, the aforementioned anti-CD40 antibody or antigen-binding fragment thereof is a recombinant antibody.

In certain embodiments, the aforementioned anti-CD40 antibody or antigen-binding fragment thereof is a rabbit antibody, a chimeric antibody, a humanized antibody, a human antibody, or an antigen-binding fragment thereof.

In certain embodiments, when the aforementioned anti-CD40 antibody or antigen-binding fragment thereof is a humanized antibody, heavy chain framework regions are derived from IGHV2-26*01, IGHV4-30-4*02, IGHV4-4*08, and IGHJ1*01, and/or light chain framework regions are derived from IGkV1-13*02, IGkV1-9*01, IGkV1-6*01, and IGKJ4*01. For example, heavy chain framework regions FR1-FR3 are derived from IGHV2-26*01, IGHV4-30-4*02, or IGHV4-4*08, and a heavy chain framework region FR4 is derived from IGHJ1*01; light chain framework regions FR1-FR3 are derived from IGkV1-13*02, IGkV1-9*01, or IGkV1-6*01, and a light chain framework region FR4 is derived from IGKJ4*01.

In certain embodiments, the aforementioned anti-CD40 antibody or antigen-binding fragment thereof comprises a VH and a VL, wherein
A-1) the amino acid sequence of the VH is set forth in SEQ ID NO: 1 or has at least 90% identity thereto, and the amino acid sequence of the VL is set forth in SEQ ID NO: 2 or has at least 90% identity thereto;
A-2) the amino acid sequence of the VH is set forth in SEQ ID NO: 13 or has at least 90% identity thereto, and the amino acid sequence of the VL is set forth in any one of SEQ ID NOs: 9-12 or has at least 90% identity thereto;
A-3) the amino acid sequence of the VH is set forth in SEQ ID NO: 14 or has at least 90% identity thereto, and the amino acid sequence of the VL is set forth in any one of SEQ ID NOs: 9-12 or has at least 90% identity thereto.

In certain embodiments, the aforementioned anti-CD40 antibody or antigen-binding fragment thereof is an IgG antibody or an antigen-binding fragment thereof, e.g., an IgG1, IgG2, IgG2, or IgG4 antibody or an antigen-binding fragment thereof, e.g., an IgG1 antibody having the mutation N297A or an antigen-binding fragment thereof, e.g., an IgG1 antibody having one of L234A, L235A, M252Y, S254T, and T256E, or any combination thereof, or an antigen-binding fragment thereof.

In certain embodiments, the antigen-binding fragment of the aforementioned anti-CD40 antibody is a Fab, an Fv, an sFv, a Fab', a F(ab')₂, a linear antibody, a single-chain antibody, an scFv, an sdAb, an sdFv, a nanobody, a peptibody, a domain antibody, and a multispecific antibody (bispecific antibody, diabody, triabody, and tetrabody, tandem di-scFv, tandem tri-scFv), for example, an scFv, Fv, Fab, or Fab' fragment.

In certain embodiments, the full-length amino acid sequence of a heavy chain of the antigen-binding fragment of the aforementioned anti-CD40 antibody is set forth in SEQ ID NO: 20 or 22 or has at least 90% identity thereto, and the full-length amino acid sequence of a light chain is set forth in SEQ ID NO: 21 or has at least 90% identity thereto.

As described above, "at least 90% identity" includes, for example, at least 91%, at least 92%, at least 93%, at least 94%, at least 95%, at least 96%, at least 97%, at least 98%, and at least 99% identity. In certain embodiments, the heavy chain variable region of the aforementioned anti-CD40 antibody or antigen-binding fragment thereof has 0 to 10 (1, 2, 3, 4, 5, 6, 7, 8, 9, or 10) amino acid alterations, and the light chain variable region has 0 to 10 (1, 2, 3, 4, 5, 6, 7, 8, 9, or 10) amino acid alterations. In some specific embodiments, the amino acid alterations are conservative replacements, substitutions, or modifications, and/or deletions or additions that do not affect function.

In certain embodiments, the antibody-drug conjugate of the present disclosure has a structure represented by formula (I):

Ab-(L-D)ₖ (I);

wherein Ab is any one of the aforementioned anti-CD40 antibodies or antigen-binding fragments thereof;
D is selected from the group consisting of a glucocorticoid receptor agonist, a glucocorticoid, a calcineurin inhibitor, and a target of rapamycin (mTOR) kinase inhibitor;
L is a linker covalently linking Ab to D;
k is an integer or decimal of 1-20 (including 1, 2, 3, 4, 5, 6, 7, 8, 9, 10, 11, 12, 13, 14, 15, 16, 17, 18, 19, 20, or any decimal or integer between any two of the aforementioned values). In certain embodiments, k is 4±0.4, 4±0.5, 4±0.6, or 4±0.8. In certain embodiments, k is 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0.

In certain embodiments, D is a glucocorticoid receptor agonist, for example, selected from the group consisting of a glucocorticoid receptor agonist with any structure disclosed in WO2022166779A1. In certain embodiments, D is a glucocorticoid, such as budesonide and ciclesonide.

In certain embodiments, D is a calcineurin inhibitor (CAI), such as tacrolimus, voclosporin, and cyclosporine A (CsA).

In certain embodiments, D is a target of rapamycin (mTOR) kinase inhibitor, such as rapamycin, everolimus, and temsirolimus.

WO2022166779A1 disclosed an antibody-drug conjugate of a glucocorticoid receptor agonist, the content of which is incorporated herein in its entirety, including the structure of the antibody-drug conjugate and the preparation method therefor.

In certain embodiments, the antibody-drug conjugate of the present disclosure has a structure represented by formula (I):

Ab-(L-D)ₖ (I);

wherein Ab is any one of the aforementioned anti-CD40 antibodies or antigen-binding fragments thereof;
D is a glucocorticoid;
L is a linker covalently linking Ab to D;
k is an integer or decimal selected from the group consisting of 1-20 (including 1, 2, 3, 4, 5, 6, 7, 8, 9, 10, 11, 12, 13, 14, 15, 16, 17, 18, 19, 20, or any value between any two of the values).

WO2023143351A1 disclosed a drug conjugate of a glucocorticoid, the content of which is incorporated herein in its entirety, including the structure of the antibody-drug conjugate and the preparation method therefor.

In certain embodiments, D is represented by the following formula:

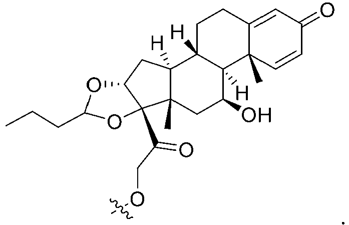

In certain embodiments, k is any value between 1 and 10. k may be an integer or a decimal.

In certain embodiments, the antibody-drug conjugate (ADC) of the present disclosure is represented by the following formula: 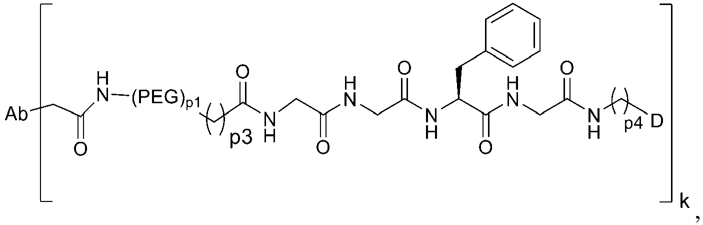 wherein k is selected from the group consisting of 1 to 10 and may be an integer or a decimal; p1 is selected from the group consisting of 2, 4, 6, and 8; p3 and p4 are each independently selected from the group consisting of 0, 1, and 2;

In certain embodiments, the antibody-drug conjugate (ADC) of the present disclosure is selected from the group consisting of: 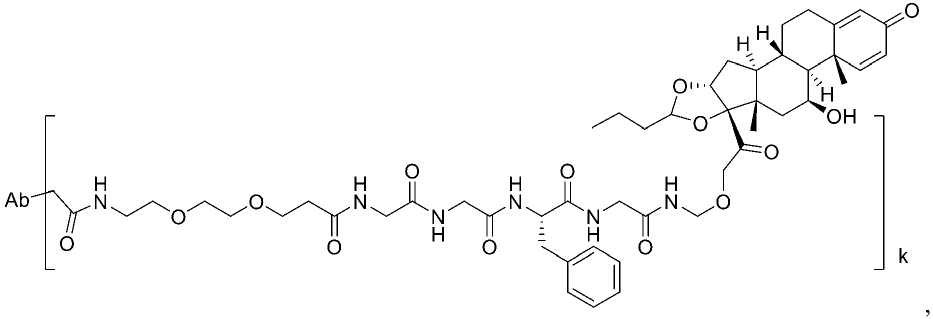 and 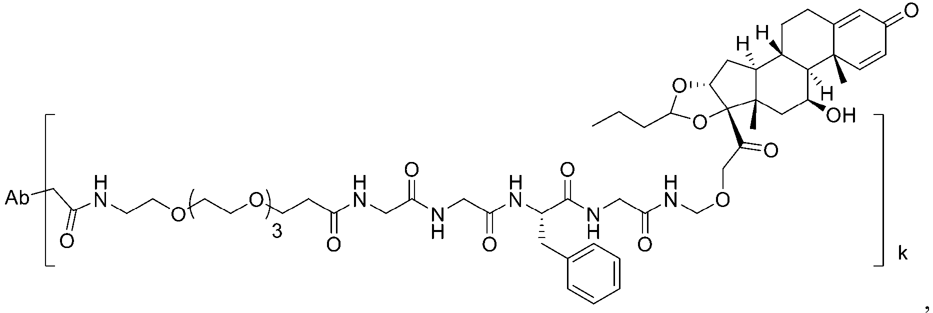 wherein k is an integer or decimal of 1-10 (e.g., 1, 2, 3, 4, 5, 6, 7, 8, 9, 10, or any decimal or integer between any two of the aforementioned values). In some embodiments, k is an integer or decimal of 3-5. In some embodiments, k is 4±0.4, 4±0.5, 4±0.6, or 4±0.8. In some embodiments, k is 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0. In certain embodiments, k is any value between 2 and 5.

In certain embodiments, the antibody-drug conjugate (ADC) of the present disclosure is shown below: 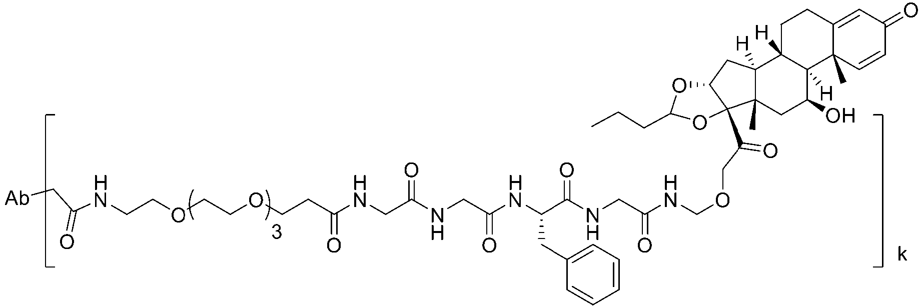 wherein k is an integer or decimal of 1-10 (e.g., 1, 2, 3, 4, 5, 6, 7, 8, 9, 10, or any decimal or integer between any two of the aforementioned values). In some embodiments, k is an integer or decimal of 2-5. In some embodiments, k is 4±0.4, 4±0.5, 4±0.6, or 4±0.8. In some embodiments, k is 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0.

In certain embodiments, the antibody-drug conjugate (ADC) of the present disclosure is shown below: 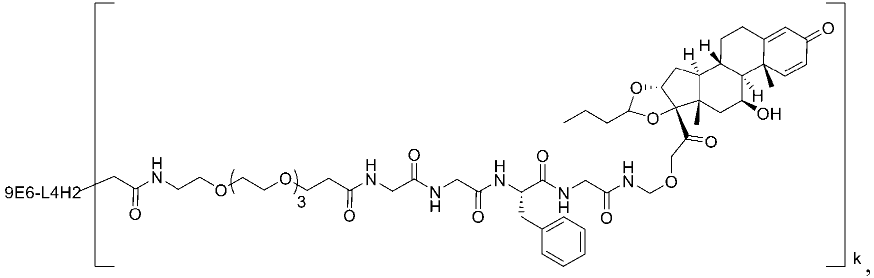
wherein 9E6-L4H2 is an anti-CD40 antibody comprising: a heavy chain and a light chain comprising the amino acid sequences set forth in SEQ ID NOs: 20 and 21, respectively;
wherein k is an integer or decimal selected from the group consisting of 1-10. In certain embodiments, k is an integer or decimal from 2 to 5. In certain embodiments, k is 4±0.4, 4±0.5, 4±0.6, or 4±0.8. In certain embodiments, k is about 3.0, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, or about 5.0.

In certain embodiments, the buffer in the pharmaceutical composition has a pH of 4-8, e.g., 4.5-7.5, 4.5-7, 4.5-6.8, 4.5-6.5, 4.5-6.3, 4.5-6, 5-7.2, 5-7.1, 5-7, 5-6.9, 5-6.8, 5-6.7, 5-6.6, 5-6.5, 5-6.4, 5-6.3, 5-6.2, 5-6.1, 5-6, 5.5-7, 5.5-6.9, 5.5-6.8, 5.5-6.7, 5.5-6.6, 5.5-6.5, 5.5-6.4, 5.5-6.3, 5.5-6.2, 5.5-6.1, 5.5-6, 5.6-7, 5.6-6.9, 5.6-6.8, 5.6-6.7, 5.6-6.6, 5.6-6.5, 5.6-6.4, 5.6-6.3, 5.6-6.2, 5.6-6.1, or 6-6.8.

In certain embodiments, the buffer in the pharmaceutical composition has a pH of 4.5-7.5. In certain embodiments, the buffer in the pharmaceutical composition has a pH of 5-7.2. In certain embodiments, the buffer in the pharmaceutical composition has a pH of 5.5-7. In certain embodiments, the buffer has a pH of 5.6-6.6. In certain embodiments, the buffer in the pharmaceutical composition has a pH of about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, or about 7.0. In certain embodiments, the buffer in the pharmaceutical composition has a pH of about 5.6, about 5.7, about 5.8, about 5.9, about 6.0, about 6.1, about 6.2, about 6.3, about 6.4, about 6.5, or about 6.6. In certain embodiments, the buffer in the pharmaceutical composition has a pH of about 6.0, about 6.1, about 6.2, about 6.3, about 6.4, about 6.5, or about 6.6.

In certain embodiments, there is a difference of no more than ±0.5 between the pH of the pharmaceutical composition and the pH of the buffer that the pharmaceutical composition comprises. In certain embodiments, the pharmaceutical composition has a pH of 4-8, e.g., 4.5-7.5, 4.5-7, 4.5-6.8, 4.5-6.5, 4.5-6.3, 4.5-6, 5-7.2, 5-7.1, 5-7, 5-6.9, 5-6.8, 5-6.7, 5-6.6, 5-6.5, 5-6.4, 5-6.3, 5-6.2, 5-6.1, 5-6, 5.5-7, 5.5-6.9, 5.5-6.8, 5.5-6.7, 5.5-6.6, 5.5-6.5, 5.5-6.4, 5.5-6.3, 5.5-6.2, 5.5-6.1, 5.5-6, 5.6-7, 5.6-6.9, 5.6-6.8, 5.6-6.7, 5.6-6.6, 5.6-6.5, 5.6-6.4, 5.6-6.3, 5.6-6.2, 5.6-6.1, or 6-6.8. In certain embodiments, the pharmaceutical composition has a pH of 4.5-7.5. In certain embodiments, the pharmaceutical composition has a pH of 5-7.2. In certain embodiments, the pharmaceutical composition has a pH of 5.5-7. In certain embodiments, the buffer has a pH of 5.6-6.6. In certain embodiments, the pharmaceutical composition has a pH of about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, or about 7.0. In certain embodiments, the pharmaceutical composition has a pH of about 5.6, about 5.7, about 5.8, about 5.9, about 6.0, about 6.1, about 6.2, about 6.3, about 6.4, about 6.5, or about 6.6. In certain embodiments, the pharmaceutical composition has a pH of about 6.0, about 6.1, about 6.2, about 6.3, about 6.4, about 6.5, or about 6.6.

In certain embodiments, the buffer in the pharmaceutical composition is at a concentration of 1-50 mM, e.g., 5-45 mM, 5-40 mM, 5-35 mM, 5-30 mM, 5-25 mM, 5-20 mM, 10-40 mM, 10-35 mM, 10-30 mM, 10-25 mM, 10-20 mM, 15-35 mM, 15-30 mM, 15-25 mM, or 15-20 mM. In certain embodiments, the buffer in the pharmaceutical composition is at a concentration of 5-40 mM. In certain embodiments, the buffer in the pharmaceutical composition is at a concentration of 10-30 mM. In certain embodiments, the buffer in the pharmaceutical composition is at a concentration of 15-25 mM. In certain embodiments, the buffer in the pharmaceutical composition is at a concentration of about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM, or about 30 mM. In certain embodiments, the buffer in the pharmaceutical composition is at a concentration of about 20 mM.

In certain embodiments, the pharmaceutical composition further comprises a stabilizer selected from the group consisting of one or more of an amino acid or a pharmaceutically acceptable salt thereof and a sugar. In certain embodiments, the amino acid is selected from the group consisting of one or more of proline, aspartic acid, glutamic acid, lysine, arginine, glycine, and histidine. In certain embodiments, the amino acid is selected from the group consisting of one or more of proline and arginine or pharmaceutically acceptable salts thereof. In certain embodiments, the sugar is selected from the group consisting of one or more of glucose, sucrose, maltose, and trehalose. In certain embodiments, the stabilizer is selected from the group consisting of one or more of arginine or a pharmaceutically acceptable salt thereof, proline or a pharmaceutically acceptable salt thereof, trehalose, and sucrose. In certain embodiments, the stabilizer is selected from the group consisting of one or more of sucrose and trehalose. In certain embodiments, the stabilizer is proline or a pharmaceutically acceptable salt thereof.

In certain embodiments, sucrose in the pharmaceutical composition is at a concentration of 0.1%-30% w/v, e.g., 0.5%-25% w/v, 0.5%-20% w/v, 0.5%-15% w/v, 0.5%-10% w/v, 1%-20% w/v, 1%-15% w/v, 1%-10% w/v, 5%-15% w/v, or 5%-10% w/v. In certain embodiments, sucrose in the pharmaceutical composition is at a concentration of 0.5%-20% w/v. In certain embodiments, sucrose in the pharmaceutical composition is at a concentration of 1%-15% w/v. In certain embodiments, sucrose in the pharmaceutical composition is at a concentration of 5%-10% w/v. In certain embodiments, sucrose in the pharmaceutical composition is at a concentration of about 1% w/v, about 2% w/v, about 3% w/v, about 4% w/v, about 5% w/v, about 5.5% w/v, about 6% w/v, about 6.5% w/v, about 7% w/v, about 7.5% w/v, about 8% w/v, about 8.5% w/v, about 9% w/v, about 9.5% w/v, about 10% w/v, about 11% w/v, about 12% w/v, about 13% w/v, about 14% w/v, or about 15% w/v. In certain embodiments, sucrose in the pharmaceutical composition is at a concentration of about 7.5% w/v, about 8% w/v, or about 9% w/v.

In certain embodiments, trehalose in the pharmaceutical composition is at a concentration of 0.1%-30% w/v, e.g., 0.5%-25% w/v, 0.5%-20% w/v, 0.5%-15% w/v, 0.5%-10% w/v, 1%-20% w/v, 1%-15% w/v, 1%-10% w/v, 5%-15% w/v, or 5%-10% w/v. In certain embodiments, trehalose in the pharmaceutical composition is at a concentration of 0.5%-20% w/v. In certain embodiments, trehalose in the pharmaceutical composition is at a concentration of 1%-15% w/v. In certain embodiments, sucrose in the pharmaceutical composition is at a concentration of 5%-10% w/v. In certain embodiments, trehalose in the pharmaceutical composition is at a concentration of about 1% w/v, about 2% w/v, about 3% w/v, about 4% w/v, about 5% w/v, about 5.5% w/v, about 6% w/v, about 6.5% w/v, about 7% w/v, about 7.5% w/v, about 8% w/v, about 8.5% w/v, about 9% w/v, about 9.5% w/v, about 10% w/v, about 11% w/v, about 12% w/v, about 13% w/v, about 14% w/v, or about 15% w/v. In certain embodiments, trehalose in the pharmaceutical composition is at a concentration of about 7.5% w/v.

In certain embodiments, proline or a pharmaceutically acceptable salt thereof in the pharmaceutical composition is at a concentration of 0.01%-20% w/v, e.g., 0.05%-20% w/v, 0.05%-15% w/v, 0.05%-10% w/v, 0.05%-5% w/v, 0.1%-20% w/v, 0.1%-15% w/v, 0.1%-10% w/v, 0.1%-5% w/v, 0.5%-20% w/v, 0.5%-15% w/v, 0.5%-10% w/v, 0.5%-5% w/v, 1%-20% w/v, 1%-15% w/v, 1%-10% w/v, or 1%-5% w/v. In certain embodiments, proline or a pharmaceutically acceptable salt thereof in the pharmaceutical composition is at a concentration of 0.01 %-20% w/v. In certain embodiments, proline or a pharmaceutically acceptable salt thereof in the pharmaceutical composition is at a concentration of 0.1%-15% w/v. In certain embodiments, proline or a pharmaceutically acceptable salt thereof in the pharmaceutical composition is at a concentration of 0.5%-10% w/v. In certain embodiments, proline or a pharmaceutically acceptable salt thereof in the pharmaceutical composition is at a concentration of 1%-5% w/v. In certain embodiments, proline or a pharmaceutically acceptable salt thereof in the pharmaceutical composition is at a concentration of about 0.5% w/v, 1% w/v, 1.5% w/v, 2% w/v, 2.5% w/v, 3% w/v, 3.5% w/v, 4% w/v, 4.5% w/v, 5% w/v, 5.5% w/v, 6% w/v, 6.5% w/v, 7% w/v, 7.5% w/v, 8% w/v, 8.5% w/v, 9% w/v, 9.5% w/v, or 10% w/v. In certain embodiments, proline or a pharmaceutically acceptable salt thereof in the pharmaceutical composition is at a concentration of about 1% w/v, 1.5% w/v, 2% w/v, 2.5% w/v, 3% w/v, 3.5% w/v, 4% w/v, 4.5% w/v, or 5% w/v. In certain embodiments, proline or a pharmaceutically acceptable salt thereof in the pharmaceutical composition is at a concentration of about 2.5% w/v.

In certain embodiments, arginine or a salt thereof in the pharmaceutical composition is at a concentration of 1-100 nM, e.g., 10-90 nM, 10-80 nM, 10-70 nM, 10-60 nM, 10-55 nM, 20-80 nM, 20-70 nM, 20-60 nM, 20-55 nM, 30-70 nM, 30-60 nM, 30-55 nM, 40-60 nM, or 40-55 nM. In certain embodiments, arginine or a salt thereof in the pharmaceutical composition is at a concentration of 20-80 nM. In certain embodiments, arginine or a salt thereof in the pharmaceutical composition is at a concentration of 30-70 nM. In certain embodiments, arginine or a salt thereof in the pharmaceutical composition is at a concentration of 40-60 nM. In certain embodiments, arginine or a salt thereof in the pharmaceutical composition is at a concentration of about 40 nM, about 41 nM, about 42 nM, about 43 nM, about 44 nM, about 45 nM, about 46 nM, about 47 nM, about 48 nM, about 49 nM, about 50 nM, about 51 nM, about 52 nM, about 53 nM, about 54 nM, about 55 nM, about 56 nM, about 57 nM, about 58 nM, about 59 nM, or about 60 nM. In certain embodiments, arginine or a salt thereof in the pharmaceutical composition is at a concentration of 50 nM.

In certain embodiments, the stabilizer in the pharmaceutical composition is sucrose and arginine or a pharmaceutically acceptable salt thereof, wherein sucrose is at a concentration of 0.5%-20% w/v, and arginine or a salt thereof is at a concentration of 20-80 nM. In certain embodiments, the stabilizer in the pharmaceutical composition is sucrose and arginine or a pharmaceutically acceptable salt thereof, wherein sucrose is at a concentration of 1%-15% w/v, and arginine or a salt thereof is at a concentration of 30-70 nM. In certain embodiments, the stabilizer in the pharmaceutical composition is sucrose and arginine or a pharmaceutically acceptable salt thereof, wherein sucrose is at a concentration of 5%-10% w/v, and arginine or a salt thereof is at a concentration of 40-60 nM. In certain embodiments, the stabilizer in the pharmaceutical composition is sucrose and arginine or a pharmaceutically acceptable salt thereof, wherein sucrose is at a concentration of about 7.5% w/v or about 8% w/v, and arginine or a salt thereof is at a concentration of 50 nM. In certain embodiments, arginine or a pharmaceutically acceptable salt thereof is arginine hydrochloride.

In certain embodiments, the pharmaceutical composition of the present disclosure further comprises a surfactant. In certain embodiments, the surfactant is an ionic or nonionic surfactant. In certain embodiments, the surfactant is selected from the group consisting of one or more of polysorbate, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocamidopropyl-betaine, linoleamidopropyl-betaine, myristamidopropyl-betaine, palmidopropyl-betaine, isostearamidopropyl-betaine, myristamidopropyl-dimethylamine, palmidopropyl-dimethylamine, isostearamidopropyl-dimethylamine, sodium methyl cocoyl, sodium methyl oleyl taurate, polyethylene glycol, polypropylene glycol, and copolymers of ethylene and propylene glycol. In certain embodiments, the surfactant is a polysorbate. In certain embodiments, the surfactant is polysorbate 20 or polysorbate 80. In certain embodiments, the surfactant is polysorbate 80.

In certain embodiments, the surfactant in the pharmaceutical composition is at a concentration of 0.001%-1% w/v, e.g., 0.005%-1% w/v, 0.005%-0.8% w/v, 0.005%-0.6% w/v, 0.005%-0.4% w/v, 0.005%-0.2% w/v, 0.005%-0.1% w/v, 0.005%-0.09% w/v, 0.005%-0.08% w/v, 0.005%-0.07% w/v, 0.005%-0.06% w/v, 0.005%-0.05% w/v, 0.008%-0.5% w/v, 0.008%-0.4% w/v, 0.008%-0.3% w/v, 0.008%-0.2% w/v, 0.008%-0.1% w/v, 0.008%-0.09% w/v, 0.008%-0.08% w/v, 0.008%-0.07% w/v, 0.008%-0.06% w/v, 0.008%-0.05% w/v, 0.01%-0.1% w/v, 0.01%-0.09% w/v, 0.01%-0.08% w/v, 0.01%-0.07% w/v, 0.01%-0.06% w/v, or 0.01%-0.05%w/v. In certain embodiments, the surfactant in the pharmaceutical composition is at a concentration of 0.005%-1% w/v. In certain embodiments, the surfactant in the pharmaceutical composition is at a concentration of 0.008%-0.2% w/v. In certain embodiments, the surfactant in the pharmaceutical composition is at a concentration of 0.01%-0.1% w/v. In certain embodiments, the surfactant in the pharmaceutical composition is at a concentration of about 0.001% w/v, about 0.002% w/v, about 0.003% w/v, about 0.004% w/v, about 0.005% w/v, about 0.006% w/v, about 0.007% w/v, about 0.008% w/v, about 0.009% w/v, about 0.01% w/v, about 0.02% w/v, about 0.03% w/v, about 0.04% w/v, about 0.05% w/v, about 0.06% w/v, about 0.07% w/v, about 0.08% w/v, about 0.09% w/v, or about 0.1% w/v. In certain embodiments, arginine or a salt thereof in the pharmaceutical composition is at a concentration of about 0.02% w/v.

In certain embodiments, the anti-CD40 antibody or the antigen-binding fragment thereof in the pharmaceutical composition is at a concentration of 0.1-500 mg/mL, e.g., 0.1-400 mg/mL, 0.1-350 mg/mL, 0.1-300 mg/mL, 0.1-250 mg/mL, 0.1-200 mg/mL, 0.1-190 mg/mL, 0.1-180 mg/mL, 0.1-170 mg/mL, 0.1-160 mg/mL, 0.1-150 mg/mL, 0.1-145 mg/mL, 0.1-140 mg/mL, 0.1-135 mg/mL, 0.1-130 mg/mL, 0.1-125 mg/mL, 0.1-120 mg/mL, 0.1-115 mg/mL, 0.1-110 mg/mL, 0.1-105 mg/mL, 0.1-100 mg/mL, 0.1-95 mg/mL, 0.1-90 mg/mL, 0.1-85 mg/mL, 0.1-80 mg/mL, 1-300 mg/mL, 1-250 mg/mL, 1-240 mg/mL, 1-230 mg/mL, 1-220 mg/mL, 1-210 mg/mL, 1-200 mg/mL, 1-190 mg/mL, 1-180 mg/mL, 1-170 mg/mL, 1-160 mg/mL, 1-150 mg/mL, 1-145 mg/mL, 1-140 mg/mL, 1-135 mg/mL, 1-130 mg/mL, 1-125 mg/mL, 1-120 mg/mL, 1-115 mg/mL, 1-110 mg/mL, 1-105 mg/mL, 1-100 mg/mL, 1-95 mg/mL, 1-90 mg/mL, 1-85 mg/mL, 1-80 mg/mL, 10-250 mg/mL, 10-240 mg/mL, 10-230 mg/mL, 10-220 mg/mL, 10-210 mg/mL, 10-200 mg/mL, 10-190 mg/mL, 10-180 mg/mL, 10-170 mg/mL, 10-160 mg/mL, 10-150 mg/mL, 10-145 mg/mL, 10-140 mg/mL, 10-135 mg/mL, 10-132 mg/mL, 10-130 mg/mL, 10-125 mg/mL, 10-120 mg/mL, 10-115 mg/mL, 10-110 mg/mL, 10-105 mg/mL, 10-100 mg/mL, 10-95 mg/mL, 10-90 mg/mL, 10-85 mg/mL, 10-80 mg/mL, 15-200 mg/mL, 15-190 mg/mL, 15-180 mg/mL, 15-170 mg/mL, 15-160 mg/mL, 15-150 mg/mL, 15-145 mg/mL, 15-140 mg/mL, 15-132 mg/mL, 15-135 mg/mL, 15-130 mg/mL, 15-125 mg/mL, 15-120 mg/mL, 15-115 mg/mL, 15-110 mg/mL, 15-105 mg/mL, 15-100 mg/mL, 15-95 mg/mL, 15-90 mg/mL, 15-85 mg/mL, 15-80 mg/mL, 20-200 mg/mL, 20-190 mg/mL, 20-180 mg/mL, 20-170 mg/mL, 20-160 mg/mL, 20-150 mg/mL, 20-145 mg/mL, 20-140 mg/mL, 20-135 mg/mL, 20-130 mg/mL, 20-125 mg/mL, 20-120 mg/mL, 20-115 mg/mL, 20-110 mg/mL, 20-105 mg/mL, 20-100 mg/mL, 20-95 mg/mL, 20-90 mg/mL, 20-85 mg/mL, 20-80 mg/mL, 30-200 mg/mL, 30-190 mg/mL, 30-180 mg/mL, 30-170 mg/mL, 30-160 mg/mL, 30-150 mg/mL, 30-145 mg/mL, 30-140 mg/mL, 30-135 mg/mL, 30-130 mg/mL, 30-125 mg/mL, 30-120 mg/mL, 30-115 mg/mL, 30-110 mg/mL, 30-105 mg/mL, 30-100 mg/mL, 30-95 mg/mL, 30-90 mg/mL, 30-85 mg/mL, 30-80 mg/mL, 40-90 mg/mL, 40-85 mg/mL, 40-80 mg/mL, 50-200 mg/mL, 50-190 mg/mL, 50-180 mg/mL, 50-170 mg/mL, 50-160 mg/mL, 50-150 mg/mL, 50-145 mg/mL, 50-140 mg/mL, 50-135 mg/mL, 50-130 mg/mL, 50-125 mg/mL, 50-120 mg/mL, 50-115 mg/mL, 50-110 mg/mL, 50-105 mg/mL, 50-100 mg/mL, 50-95 mg/mL, 50-90 mg/mL, 50-85 mg/mL, 50-80 mg/mL, 85-200 mg/mL, 85-190 mg/mL, 85-180 mg/mL, 85-170 mg/mL, 85-160 mg/mL, or 85-150 mg/mL. In certain embodiments, the anti-CD40 antibody or the antigen-binding fragment thereof is at a concentration of 1-200 mg/mL. In certain embodiments, the anti-CD40 antibody or the antigen-binding fragment thereof is at a concentration of 10-150 mg/mL. In certain embodiments, the anti-CD40 antibody or the antigen-binding fragment thereof is at a concentration of 20-100 mg/mL. In certain embodiments, the anti-CD40 antibody or the antigen-binding fragment thereof is at a concentration of 1-300 mg/mL. In certain embodiments, the anti-CD40 antibody or the antigen-binding fragment thereof is at a concentration of 10-250 mg/mL. In certain embodiments, the anti-CD40 antibody or the antigen-binding fragment thereof is at a concentration of 20-200 mg/mL. In certain embodiments, the anti-CD40 antibody or the antigen-binding fragment thereof is at a concentration of 15-130 mg/mL or 15-132 mg/mL. In certain embodiments, the anti-CD40 antibody or the antigen-binding fragment thereof is at a concentration of about 5 mg/mL, about 6 mg/mL, about 7 mg/mL, about 8 mg/mL, about 9 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, about 105 mg/mL, about 110 mg/mL, about 115 mg/mL, about 120 mg/mL, about 130 mg/mL, about 140 mg/mL, about 150 mg/mL, about 160 mg/mL, about 165 mg/mL, about 170 mg/mL, about 175 mg/mL, about 180 mg/mL, about 185 mg/mL, 190 mg/mL, or about 200 mg/mL. In certain embodiments, the anti-CD40 antibody or the antigen-binding fragment thereof is at a concentration of about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, or about 190 mg/mL. In certain embodiments, the anti-CD40 antibody or the antigen-binding fragment thereof is at a concentration of about 50 mg/mL, about 80 mg/mL, or about 150 mg/mL.

In certain embodiments, the antibody-drug conjugate in the pharmaceutical composition is at a concentration of 0.1-300 mg/mL, e.g., 1-200 mg/mL, 1-190 mg/mL, 1-180 mg/mL, 1-170 mg/mL, 1-160 mg/mL, 1-150 mg/mL, 1-145 mg/mL, 1-140 mg/mL, 1-135 mg/mL, 1-130 mg/mL, 1-120 mg/mL, 1-110 mg/mL, 1-100 mg/mL, 1-90 mg/mL, 1-80 mg/mL, 1-70 mg/mL, 1-60 mg/mL, 5-150 mg/mL, 5-140 mg/mL, 5-130 mg/mL, 5-120 mg/mL, 5-110 mg/mL, 5-100 mg/mL, 5-90 mg/mL, 5-80 mg/mL, 5-70 mg/mL, 5-60 mg/mL, 10-130 mg/mL, 10-120 mg/mL, 10-110 mg/mL, 10-100 mg/mL, 10-90 mg/mL, 10-80 mg/mL, 10-70 mg/mL, or 10-60 mg/mL. In certain embodiments, the antibody-drug conjugate is at a concentration of 1-150 mg/mL. In certain embodiments, the antibody-drug conjugate is at a concentration of 5-120 mg/mL. In certain embodiments, the antibody-drug conjugate is at a concentration of about 20-100 mg/mL. In certain embodiments, the antibody-drug conjugate is at a concentration of 10-100 mg/mL. In certain embodiments, the antibody-drug conjugate is at a concentration of about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, or about 100 mg/mL. In certain embodiments, the antibody-drug conjugate is at a concentration of about 20 mg/mL, about 30 mg/mL, about 40 mg/mL, about 50 mg/mL, about 60 mg/mL, about 70 mg/mL, or about 80 mg/mL. In certain embodiments, the antibody-drug conjugate is at a concentration of about 30 mg/mL, about 40 mg/mL, or about 50 mg/mL.

In certain embodiments, the present disclosure provides a pharmaceutical composition comprising an anti-CD40 antibody or an antigen-binding fragment thereof (e.g., 9E6(L4H2) with heavy and light chain sequences of SEQ ID NOs: 20 and 21, respectively), and the pharmaceutical composition comprises or is any one of the following groups A) to F):
A) the anti-CD40 antibody or the antigen-binding fragment thereof;
   a histidine-hydrochloride buffer, e.g., histidine-histidine hydrochloride;
   a polysorbate, e.g., preferably polysorbate 80;
   sucrose, trehalose, and/or arginine or a pharmaceutically acceptable salt thereof;
B) the anti-CD40 antibody or the antigen-binding fragment thereof;
   a histidine-hydrochloride buffer, e.g., histidine-histidine hydrochloride;
   a polysorbate, e.g., preferably polysorbate 80;
   proline or a pharmaceutically acceptable salt thereof;
C) the anti-CD40 antibody or the antigen-binding fragment thereof;
   a histidine-hydrochloride buffer, e.g., histidine-histidine hydrochloride;
   a polysorbate, e.g., preferably polysorbate 80;
   sucrose;
D) any one of A)-C) described above, wherein the buffer has a pH of 4.5-7.5, e.g., 5-7.2, 5.5-7, or 5.6-6.6;
E) any one of A)-D) described above, wherein the pharmaceutical composition further comprises water for injection;
F) any one of A)-E) described above, wherein the pharmaceutical composition has a pH of 4.5-7.5, e.g., 5-7.2, 5.5-7, or 5.6-6.6.

In certain embodiments, the present disclosure provides a pharmaceutical composition comprising an antibody-drug conjugate, and the pharmaceutical composition comprises or is any one of the following groups A) to H):
A) the antibody-drug conjugate;
   a histidine-hydrochloride buffer, e.g., histidine-histidine hydrochloride;
   a polysorbate, e.g., preferably polysorbate 80;
   sucrose, trehalose, and/or arginine or a pharmaceutically acceptable salt thereof;
B) the antibody-drug conjugate;
   a histidine-hydrochloride buffer, e.g., histidine-histidine hydrochloride;
   a polysorbate, e.g., preferably polysorbate 80;
   proline or a pharmaceutically acceptable salt thereof;
C) the antibody-drug conjugate;
   a histidine-hydrochloride buffer, e.g., histidine-histidine hydrochloride;
   a polysorbate, e.g., preferably polysorbate 80;
   sucrose;
D) the antibody-drug conjugate;
   a histidine-hydrochloride buffer, e.g., histidine-histidine hydrochloride;
   a polysorbate, e.g., preferably polysorbate 80;
   trehalose;
E) any one of A)-D) described above, wherein the antibody-drug conjugate has the following structure: 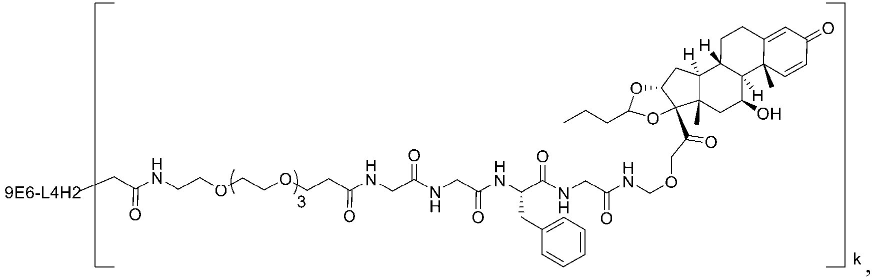
   wherein 9E6-L4H2 is an anti-CD40 antibody comprising: a heavy chain and a light chain comprising the amino acid sequences set forth in SEQ ID NOs: 20 and 21, respectively;
   wherein k is an integer or decimal selected from the group consisting of 1-10; for example, k is an integer or decimal of 2-5; for example, k is 4±0.4, 4±0.5, 4±0.6, or 4±0.8; for example, k is about 3.0, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, or about 5.0;
F) any one of A)-E) described above, wherein the buffer has a pH of 4.5-7.5, e.g., 5-7.2, 5.5-7, or 5.6-6.6;
G) any one of A)-F) described above, wherein the pharmaceutical composition further comprises water for injection;
H) any one of A)-G) described above, wherein the pharmaceutical composition has a pH of 4.5-7.5, e.g., 5-7.2, 5.5-7, or 5.6-6.6.

In certain embodiments, the present disclosure provides a pharmaceutical composition comprising an anti-CD40 antibody or an antigen-binding fragment thereof (e.g., 9E6(L4H2) with heavy and light chain sequences of SEQ ID NOs: 20 and 21, respectively), and the pharmaceutical composition comprises or is any one of the following groups 1) to 21):
1) 0.1-300 mg/mL anti-CD40 antibody or antigen-binding fragment thereof;
   1-50 mM histidine-hydrochloride buffer, e.g., histidine-histidine hydrochloride;
   0.001%-1% w/v polysorbate, e.g., polysorbate 80;
   0.1%-30% w/v sucrose;
   and the composition having a pH of 4.5-7.5;
2) 1-200 mg/mL anti-CD40 antibody or antigen-binding fragment thereof;
   5-40 mM histidine-hydrochloride buffer, e.g., histidine-histidine hydrochloride;
   0.005%-1% w/v polysorbate, e.g., polysorbate 80;
   0.5%-20% w/v sucrose;
   and the composition having a pH of 5-7.2;
3) 10-150 mg/mL anti-CD40 antibody or antigen-binding fragment thereof;
   10-30 mM histidine-hydrochloride buffer, e.g., histidine-histidine hydrochloride;
   0.008%-0.2% w/v polysorbate, e.g., polysorbate 80;
   1%-15% w/v sucrose;
   and the composition having a pH of 5.5-7;
4) 15-132 mg/mL or 20-100 mg/mL anti-CD40 antibody or antigen-binding fragment thereof;
   15-25 mM histidine-hydrochloride buffer, e.g., histidine-histidine hydrochloride;
   0.01%-0.1% w/v polysorbate, e.g., polysorbate 80;
   5%-10% w/v sucrose;
   and the composition having a pH of 5.6-6.6;
5) about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, about 110 mg/mL, about 120 mg/mL, or about 130 mg/mL anti-CD40 antibody or antigen-binding fragment thereof;
   15-25 mM histidine-hydrochloride buffer, e.g., histidine-histidine hydrochloride;
   0.01%-0.1% w/v polysorbate, e.g., polysorbate 80;
   5%-10% w/v sucrose;
   and the composition having a pH of 5.6-6.6;
6) 20-100 mg/mL anti-CD40 antibody or antigen-binding fragment thereof;
   about 15 mM, about 20 mM, or about 25 mM histidine-hydrochloride buffer, e.g., histidine-histidine hydrochloride;
   0.01%-0.1% w/v polysorbate, e.g., polysorbate 80;
   5%-10% w/v sucrose;
   and the composition having a pH of 5.6-6.6;
7) 20-100 mg/mL anti-CD40 antibody or antigen-binding fragment thereof;
   15-25 mM histidine-hydrochloride buffer, e.g., histidine-histidine hydrochloride;
   about 0.01% w/v, about 0.02% w/v, about 0.03% w/v, about 0.04% w/v, about 0.05% w/v, about 0.06% w/v, or about 0.07% w/v polysorbate, e.g., polysorbate 80;
   5%-10% w/v sucrose;
   and the composition having a pH of 5.6-6.6;
8) 20-100 mg/mL anti-CD40 antibody or antigen-binding fragment thereof;
   15-25 mM histidine-hydrochloride buffer, e.g., histidine-histidine hydrochloride;
   0.01%-0.1% w/v polysorbate, e.g., polysorbate 80;
   about 5% w/v, about 5.5% w/v, about 6% w/v, about 6.5% w/v, about 7% w/v, about 7.5% w/v, about 8% w/v, about 8.5% w/v, about 9% w/v, about 9.5% w/v, or about 10% w/v sucrose;
   and the composition having a pH of 5.6-6.6;
9) 20-100 mg/mL anti-CD40 antibody or antigen-binding fragment thereof;
   15-25 mM histidine-hydrochloride buffer, e.g., histidine-histidine hydrochloride;
   0.01%-0.1% w/v polysorbate, e.g., polysorbate 80;
   5%-10% w/v sucrose;
   and the composition having a pH of about 5.6, about 5.7, about 5.8, about 5.9, about 6.0, about 6.1, about 6.2, about 6.3, about 6.4, about 6.5, or about 6.6;
10) 0.1-500 mg/mL anti-CD40 antibody or antigen-binding fragment thereof;
   1-50 mM histidine-hydrochloride buffer, e.g., histidine-histidine hydrochloride;
   0.001%-1% w/v polysorbate, e.g., polysorbate 80;
   0.01%-20% w/v proline or a pharmaceutically acceptable salt thereof;
   and the composition having a pH of 4.5-7.5;
11) 1-300 mg/mL anti-CD40 antibody or antigen-binding fragment thereof;
   5-40 mM histidine-hydrochloride buffer, e.g., histidine-histidine hydrochloride;
   0.005%-1% w/v polysorbate, e.g., polysorbate 80;
   0.1%-15% w/v proline or a pharmaceutically acceptable salt thereof;
   and the composition having a pH of 5-7.2;
12) 10-250 mg/mL anti-CD40 antibody or antigen-binding fragment thereof;
   10-30 mM histidine-hydrochloride buffer, e.g., histidine-histidine hydrochloride;
   0.008%-0.2% w/v polysorbate, e.g., polysorbate 80;
   0.5%-10% w/v proline or a pharmaceutically acceptable salt thereof;
   and the composition having a pH of 5.5-7;
13) 20-200 mg/mL anti-CD40 antibody or antigen-binding fragment thereof;
   15-25 mM histidine-hydrochloride buffer, e.g., histidine-histidine hydrochloride;
   0.01%-0.1% w/v polysorbate, e.g., polysorbate 80;
   1%-5% w/v proline or a pharmaceutically acceptable salt thereof;
   and the composition having a pH of 5.6-6.6;
14) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL anti-CD40 antibody or antigen-binding fragment thereof;
   15-25 mM histidine-hydrochloride buffer, e.g., histidine-histidine hydrochloride;
   0.01%-0.1% w/v polysorbate, e.g., polysorbate 80;
   1%-5% w/v proline or a pharmaceutically acceptable salt thereof;
   and the composition having a pH of 5.6-6.6;
15) 20-200 mg/mL anti-CD40 antibody or antigen-binding fragment thereof;
   about 15 mM, about 20 mM, or about 25 mM histidine-hydrochloride buffer, e.g., histidine-histidine hydrochloride;
   0.01%-0.1% w/v polysorbate, e.g., polysorbate 80;
   1%-5% w/v proline or a pharmaceutically acceptable salt thereof;
   and the composition having a pH of 5.6-6.6;
16) 20-200 mg/mL anti-CD40 antibody or antigen-binding fragment thereof;
   15-25 mM histidine-hydrochloride buffer, e.g., histidine-histidine hydrochloride;
   about 0.01% w/v, about 0.02% w/v, about 0.03% w/v, about 0.04% w/v, about 0.05% w/v, about 0.06% w/v, or about 0.07% w/v polysorbate, e.g., polysorbate 80;
   1%-5% w/v proline or a pharmaceutically acceptable salt thereof;
   and the composition having a pH of 5.6-6.6;
17) 20-200 mg/mL anti-CD40 antibody or antigen-binding fragment thereof;
   15-25 mM histidine-hydrochloride buffer, e.g., histidine-histidine hydrochloride;
   0.01%-0.1% w/v polysorbate, e.g., polysorbate 80;
   about 1% w/v, about 1.5% w/v, about 2% w/v, about 2.5% w/v, about 3% w/v, about 3.5% w/v, about 4% w/v, about 4.5% w/v, or about 5% w/v proline or a pharmaceutically acceptable salt thereof;
   and the composition having a pH of 5.6-6.6;
18) 20-200 mg/mL anti-CD40 antibody or antigen-binding fragment thereof;
   15-25 mM histidine-hydrochloride buffer, e.g., histidine-histidine hydrochloride;
   0.01%-0.1% w/v polysorbate, e.g., polysorbate 80;
   1%-5% w/v proline or a pharmaceutically acceptable salt thereof;
   and the composition having a pH of about 5.6, about 5.7, about 5.8, about 5.9, about 6.0, about 6.1, about 6.2, about 6.3, about 6.4, about 6.5, or about 6.6;
19) the pharmaceutical composition according to any one of the aforementioned 6)-9), wherein the anti-CD40 antibody or the antigen-binding fragment thereof is at a concentration of 15-130 mg/mL or 15-132 mg/mL;
20) the pharmaceutical composition according to any one of the aforementioned 15)-18), wherein the anti-CD40 antibody or the antigen-binding fragment thereof is at a concentration of 30-190 mg/mL;
21) the pharmaceutical composition according to any one of the aforementioned 1)-20), wherein the pharmaceutical composition further comprises water for injection.

In certain embodiments, the present disclosure provides a pharmaceutical composition comprising an antibody-drug conjugate, and the pharmaceutical composition comprises or is any one of the following groups 1) to 6):
1) 0.1-300 mg/mL antibody-drug conjugate;
   1-50 mM histidine-hydrochloride buffer, e.g., histidine-histidine hydrochloride;
   0.001%-1% w/v polysorbate, e.g., polysorbate 80;
   0.1%-30% w/v sucrose or trehalose;
   and the composition having a pH of 4.5-7.5;
2) 1-150 mg/mL antibody-drug conjugate;
   5-40 mM histidine-hydrochloride buffer, e.g., histidine-histidine hydrochloride;
   0.005%-1% w/v polysorbate, e.g., polysorbate 80;
   0.5%-20% w/v sucrose or trehalose;
   and the composition having a pH of 5-7.2;
3) 5-120 mg/mL antibody-drug conjugate;
   10-30 mM histidine-hydrochloride buffer, e.g., histidine-histidine hydrochloride;
   0.008%-0.2% w/v polysorbate, e.g., polysorbate 80;
   1%-15% w/v sucrose or trehalose;
   and the composition having a pH of 5.5-7;
4) 10-100 mg/mL antibody-drug conjugate;
   15-25 mM histidine-hydrochloride buffer, e.g., histidine-histidine hydrochloride;
   0.01%-0.1% w/v polysorbate, e.g., polysorbate 80;
   5%-10% w/v sucrose or trehalose;
   and the composition having a pH of 5.6-6.6;
5) any one of the aforementioned 1)-4), wherein the antibody-drug conjugate has the following structure: 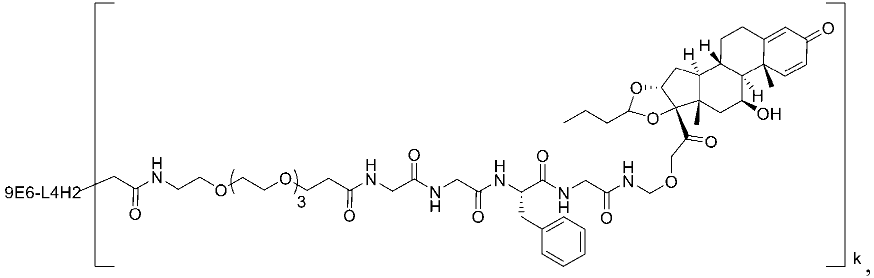
   wherein 9E6-L4H2 is an anti-CD40 antibody comprising: a heavy chain and a light chain comprising the amino acid sequences set forth in SEQ ID NOs: 20 and 21, respectively;
   wherein k is an integer or decimal selected from the group consisting of 1-10; for example, k is an integer or decimal of 3-5; for example, k is 4±0.4, 4±0.5, 4±0.6, or 4±0.8; for example, k is about 3.0, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, or about 5.0;
6) the pharmaceutical composition according to any one of the aforementioned 1)-5), wherein the pharmaceutical composition further comprises water for injection.

In certain embodiments, the present disclosure provides a pharmaceutical composition comprising an anti-CD40 antibody or an antigen-binding fragment thereof (e.g., 9E6(L4H2) with heavy and light chain sequences of SEQ ID NOs: 20 and 21, respectively), and the pharmaceutical composition comprises or is any one of the following groups 1) to 19):
1) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 7.5% w/v sucrose;
   and the composition having a pH of about 6.0;
2) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 7.5% w/v sucrose;
   and the composition having a pH of about 6.1;
3) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 7.5% w/v sucrose;
   and the composition having a pH of about 6.2;
4) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 7.5% w/v sucrose;
   and the composition having a pH of about 6.3;
5) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 7.5% w/v sucrose;
   and the composition having a pH of about 6.5;
6) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 7.5% w/v sucrose;
   and the composition having a pH of about 6.6;
7) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 8% w/v sucrose;
   and the composition having a pH of about 6.0;
8) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 8% w/v sucrose;
   and the composition having a pH of about 6.1;
9) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 8% w/v sucrose;
   and the composition having a pH of about 6.2;
10) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 8% w/v sucrose;
   and the composition having a pH of about 6.3;
11) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 8% w/v sucrose;
   and the composition having a pH of about 6.5;
12) about 50 mg/mL, about 70 mg/mL, about 80 mg/mL, about 90 mg/mL, about 100 mg/mL, about 110 mg/mL, about 120 mg/mL, or about 130 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 8% w/v sucrose;
   and the composition having a pH of about 6.6;
13) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 2.5% w/v proline;
   and the composition having a pH of about 6.0;
14) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 2.5% w/v proline;
   and the composition having a pH of about 6.1;
15) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 2.5% w/v proline;
   and the composition having a pH of about 6.2;
16) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 2.5% w/v proline;
   and the composition having a pH of about 6.3;
17) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 2.5% w/v proline;
   and the composition having a pH of about 6.5;
18) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL anti-CD40 antibody or antigen-binding fragment thereof;
   about 20 mM histidine-hydrochloride buffer, e.g., histidine-histidine hydrochloride;
   about 0.02% w/v polysorbate 80;
   about 2.5% w/v proline;
   and the composition having a pH of about 6.6;
19) the pharmaceutical composition according to any one of the aforementioned 1)-18), wherein the pharmaceutical composition further comprises water for injection.

In certain embodiments, the present disclosure provides a pharmaceutical composition comprising an antibody-drug conjugate, and the pharmaceutical composition comprises or is any one of the following groups 1) to 8):
1) about 20 mg/mL, about 30 mg/mL, about 40 mg/mL, about 50 mg/mL, about 60 mg/mL, about 70 mg/mL, or about 80 mg/mL antibody-drug conjugate;
   about 20 mM histidine-hydrochloride buffer, e.g., histidine-histidine hydrochloride;
   about 0.02% w/v polysorbate 80;
   about 7.5% w/v or about 9% w/v sucrose;
   and the composition having a pH of about 6.0;
2) about 20 mg/mL, about 30 mg/mL, about 40 mg/mL, about 50 mg/mL, about 60 mg/mL, about 70 mg/mL, or about 80 mg/mL antibody-drug conjugate;
   about 20 mM histidine-hydrochloride buffer, e.g., histidine-histidine hydrochloride;
   about 0.02% w/v polysorbate 80;
   about 7.5% w/v or about 9% w/v sucrose;
   and the composition having a pH of about 6.3;
3) about 20 mg/mL, about 30 mg/mL, about 40 mg/mL, about 50 mg/mL, about 60 mg/mL, about 70 mg/mL, or about 80 mg/mL antibody-drug conjugate;
   about 20 mM histidine-hydrochloride buffer, e.g., histidine-histidine hydrochloride;
   about 0.02% w/v polysorbate 80;
   about 7.5% w/v or about 9% w/v sucrose;
   and the composition having a pH of about 6.6;
4) about 20 mg/mL, about 30 mg/mL, about 40 mg/mL, about 50 mg/mL, about 60 mg/mL, about 70 mg/mL, or about 80 mg/mL antibody-drug conjugate;
   about 20 mM histidine-hydrochloride buffer, e.g., histidine-histidine hydrochloride;
   about 0.02% w/v polysorbate 80;
   about 7.5% w/v trehalose;
   and the composition having a pH of about 6.0;
5) about 20 mg/mL, about 30 mg/mL, about 40 mg/mL, about 50 mg/mL, about 60 mg/mL, about 70 mg/mL, or about 80 mg/mL antibody-drug conjugate;
   about 20 mM histidine-hydrochloride buffer, e.g., histidine-histidine hydrochloride;
   about 0.02% w/v polysorbate 80;
   about 7.5% w/v trehalose;
   and the composition having a pH of about 6.3;
6) about 20 mg/mL, about 30 mg/mL, about 40 mg/mL, about 50 mg/mL, about 60 mg/mL, about 70 mg/mL, or about 80 mg/mL antibody-drug conjugate;
   about 20 mM histidine-hydrochloride buffer, e.g., histidine-histidine hydrochloride;
   about 0.02% w/v polysorbate 80;
   about 7.5% w/v trehalose;
   and the composition having a pH of about 6.6;
7) any one of the aforementioned 1)-6), wherein the antibody-drug conjugate has the following structure: 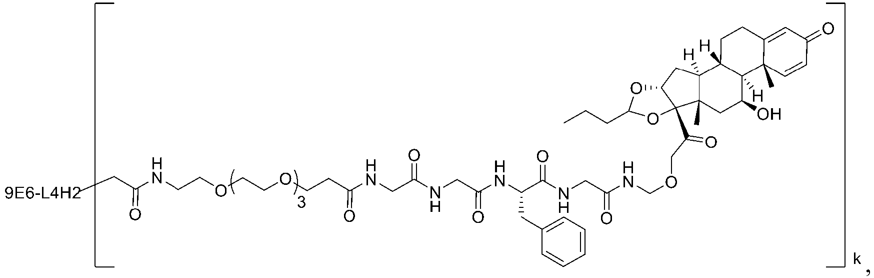
   wherein 9E6-L4H2 is an anti-CD40 antibody comprising: a heavy chain and a light chain comprising the amino acid sequences set forth in SEQ ID NOs: 20 and 21, respectively;
   wherein k is an integer or decimal selected from the group consisting of 1-10; for example, k is an integer or decimal of 3-5; for example, k is 4±0.4, 4±0.5, 4±0.6, or 4±0.8; for example, k is about 3.0, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, or about 5.0;
   8) the pharmaceutical composition according to any one of the aforementioned 1)-7), wherein the pharmaceutical composition further comprises water for injection.

In certain embodiments, the pharmaceutical composition of the present disclosure has a viscosity of less than or equal to 30 cp, e.g., less than or equal to 25 cp or 20 cp. In certain embodiments, the viscosity of the pharmaceutical compositions of the present disclosure is suitable for injection.

The present disclosure further provides a method for preparing the aforementioned pharmaceutical composition, which comprises a step of mixing the anti-CD40 antibody or the antigen-binding fragment thereof with a buffer. In certain embodiments, the buffer is a histidine-hydrochloride buffer (e.g., histidine-histidine hydrochloride).

In certain embodiments, provided is the pharmaceutical composition according to any one of the above, wherein the pharmaceutical composition is a liquid formulation. In some embodiments, the solvent of the liquid formulation is water, normal saline, or glucose solution.

The present disclosure further provides a freeze-dried formulation, wherein the freeze-dried formulation is capable of forming the pharmaceutical composition according to any one of the above upon reconstitution.

The present disclosure further provides a freeze-dried formulation, wherein the freeze-dried formulation is obtained by lyophilizing the pharmaceutical composition according to any one of the above.

The present disclosure provides a reconstituted solution, wherein the reconstituted solution is prepared by reconstituting the aforementioned freeze-dried formulation. In certain embodiments, the reconstituted solution is selected from the group consisting of, but not limited to, water for injection, normal saline, or glucose solution.

The present disclosure further provides an article of manufacture, which comprises a container containing the aforementioned pharmaceutical composition, the aforementioned freeze-dried formulation, or the aforementioned reconstituted solution. In certain embodiments, the container is a tubular injection vial made of neutral borosilicate glass. In certain embodiments, the article of manufacture comprises a package insert.

The present disclosure provides use of the aforementioned pharmaceutical composition, a pharmaceutical composition prepared by the aforementioned method, the aforementioned freeze-dried formulation, or the aforementioned reconstituted solution in a medicament for treating or alleviating an autoimmune disease or graft-versus-host disease, or alleviating graft rejection.

In certain embodiments, provided are a method for ameliorating or treating autoimmune diseases or inflammatory diseases, and related pharmaceutical use, the method comprising administering to a subject the aforementioned pharmaceutical composition, a pharmaceutical composition prepared by the aforementioned method, the aforementioned freeze-dried formulation, or the aforementioned reconstituted solution.

In certain embodiments, provided are a method for treating CD40-related disorders and diseases, and related pharmaceutical use; in certain embodiments, provided are a method for inhibiting the growth or differentiation of cells of CD40-related disorders, and related pharmaceutical use; in certain embodiments, provided are a method for inhibiting the growth and/or differentiation of cells that express the antigen human CD40, and related pharmaceutical use; in certain embodiments, provided are a method for producing an antibody that inhibits B cells in a subject, and related pharmaceutical use; in certain embodiments, provided are a method for treating immune disorders and diseases, and related pharmaceutical use. The above methods all comprise administering to a subject or a cell the aforementioned pharmaceutical composition, a pharmaceutical composition prepared by the aforementioned method, the aforementioned freeze-dried formulation, or the aforementioned reconstituted solution.

In certain embodiments, provided are a method for inducing peripheral B cell depletion, and related pharmaceutical use, the method comprising administering to a subject the aforementioned pharmaceutical composition, a pharmaceutical composition prepared by the aforementioned method, the aforementioned freeze-dried formulation, or the aforementioned reconstituted solution.

In certain embodiments, provided are a method for treating or alleviating a disease or condition, and related pharmaceutical use, the method comprising administering to a subject in need thereof the aforementioned pharmaceutical composition, a pharmaceutical composition prepared by the aforementioned method, the aforementioned freeze-dried formulation, or the aforementioned reconstituted solution, wherein the disease or condition may or may not be related to CD40, and includes: rheumatoid arthritis, systemic lupus erythematosus, lupus nephritis, autoimmune demyelinating diseases (e.g., multiple sclerosis and allergic encephalomyelitis), endocrine ophthalmopathy, uveoretinitis, systemic lupus erythematosus, myasthenia gravis, Graves' disease, glomerulonephritis, autoimmune liver disease, inflammatory bowel diseases (e.g., Crohn's disease or ulcerative colitis), anaphylaxis, allergic reaction, Sjögren's syndrome, type I diabetes, primary biliary cirrhosis, Wegener's granulomatosis, fibromyalgia, polymyositis, dermatomyositis, inflammatory myositis, multiple endocrine failure, Schmidt's syndrome, autoimmune uveitis, Addison's disease, adrenalitis, thyroiditis, Hashimoto's thyroiditis, autoimmune thyroid disease, pernicious anemia, gastric atrophy, chronic hepatitis, lupoid hepatitis, atherosclerosis, subacute cutaneous lupus erythematosus, hypoparathyroidism, Dressler's syndrome, autoimmune thrombocytopenia, idiopathic thrombocytopenic purpura, hemolytic anemia, pemphigus vulgaris, pemphigus, dermatitis herpetiformis, alopecia arcata, pemphigoid, scleroderma, progressive systemic sclerosis, CREST syndrome (calcinosis, Raynaud's phenomenon, esophageal dysmotility, sclerodactyly, and telangiectasia), male and female autoimmune infertility, ankylosing spondolytis, ulcerative colitis, mixed connective tissue disease, polyarteritis nodosa, systemic necrotizing vasculitis, atopic dermatitis, atopic rhinitis, Goodpasture's syndrome, Chagas' disease, sarcoidosis, rheumatic fever, asthma, recurrent abortion, anti-phospholipid syndrome, farmer's lung, erythema multiforme, post cardiotomy syndrome, Cushing's syndrome, autoimmune chronic active hepatitis, bird fancier's lung, toxic epidermal necrolysis, Alport's syndrome, alveolitis, allergic alveolitis, fibrosing alveolitis, interstitial lung disease, erythema nodosum, pyoderma gangrenosum, transfusion reaction, Takayasu's arteritis, polymyalgia rheumatica, temporal arteritis, schistosomiasis, giant cell arteritis, ascariasis, aspergillosis, Sampter's syndrome, eczema, lymphomatoid granulomatosis, Behcet's disease, Caplan's syndrome, Kawasaki's disease, dengue, encephalomyelitis, endocarditis, endomyocardial fibrosis, endophthalmitis, erythema elevatum diutinum, psoriasis, erythroblastosis fetalis, eosinophilic fasciitis, Shulman's syndrome, Felty's syndrome, filariasis, cyclitis, chronic cyclitis, heterochronic cyclitis, Fuch's cyclitis, IgA nephropathy, Henoch-Schonlein purpura, graft-versus-host disease, graft rejection, cardiomyopathy, Eaton-Lambert syndrome, relapsing polychondritis, cryoglobulinemia, Waldenström's macroglobulinemia, Evan's syndrome, acute respiratory distress syndrome, pulmonary inflammation, osteoporosis, delayed type hypersensitivity, and autoimmune gonadal failure. Examples are Sjögren's syndrome, multiple sclerosis, and systemic lupus erythematosus.

In certain embodiments, provided are a method for treating diseases related to B lymphocytes (e.g., systemic lupus erythematosus, Goodpasture's syndrome, rheumatoid arthritis, and type I diabetes), Th1-lymphocytes (e.g., rheumatoid arthritis, multiple sclerosis, psoriasis, Sjögren's syndrome, Hashimoto's disease, Grave's disease, primary biliary cirrhosis, Wegener's granulomatosis, tuberculosis, or graft-versus-host disease), or Th2-lymphocytes (e.g., atopic dermatitis, systemic lupus erythematosus, atopic asthma, rhinoconjunctivitis, allergic rhinitis, Omenn's syndrome, systemic sclerosis, or chronic graft-versus-host disease), and related pharmaceutical use, the method comprising administering to a subject in need thereof the aforementioned pharmaceutical composition, a pharmaceutical composition prepared by the aforementioned method, the aforementioned freeze-dried formulation, or the aforementioned reconstituted solution.

In certain embodiments, provided are a method for treating a tumor or cancer, and related pharmaceutical use, the method comprising administering to a subject in need thereof an effective amount of the aforementioned anti-CD40 antibody or antigen-binding fragment thereof, wherein the tumor or cancer may or may not be related to CD40 expression.

In certain embodiments, provided is a method for treating or alleviating graft-versus-host disease or graft rejection, comprising administering to a subject in need thereof the aforementioned pharmaceutical composition, a pharmaceutical composition prepared by the aforementioned method, the aforementioned freeze-dried formulation, or the aforementioned reconstituted solution, and tacrolimus.

In certain embodiments, provided is use of the aforementioned pharmaceutical composition, a pharmaceutical composition prepared by the aforementioned method, the aforementioned freeze-dried formulation, or the aforementioned reconstituted solution in the manufacture of a medicament for treating or alleviating graft-versus-host disease or graft rejection, including use in combination with tacrolimus. In certain embodiments, provided is use of tacrolimus in the manufacture of a medicament for treating or alleviating graft-versus-host disease or graft rejection, including use in combination with the aforementioned pharmaceutical composition, a pharmaceutical composition prepared by the aforementioned method, the aforementioned freeze-dried formulation, or the aforementioned reconstituted solution.

In certain embodiments, provided are a method for treating or alleviating graft-versus-host disease or graft rejection by using the aforementioned anti-CD40 antibody or antigen-binding fragment thereof in combination with tacrolimus, and use of said combination in the manufacture of a medicament for treating or alleviating graft-versus-host disease or graft rejection.

In certain embodiments, the graft described above is a solid organ graft, such as a kidney graft, a liver graft, a heart graft, a lung graft, a pancreas graft, a small intestine graft, or a composite tissue graft. In certain embodiments, the graft described above refers to grafting one selected from the group consisting of an allogeneic cell, a xenogeneic cell, an allogeneic tissue, a xenogeneic tissue, an allogeneic organ, and a xenogeneic organ.

In certain embodiments, the aforementioned pharmaceutical composition, a pharmaceutical composition prepared by the aforementioned method, the aforementioned freeze-dried formulation, or the aforementioned reconstituted solution inhibits or reverses the rejection of the tissue graft by the graft acceptor, or prolongs or preserves the function of the tissue grafted into the graft acceptor, or restores the function of the damaged graft tissue in the graft acceptor.

### Definitions of Terms

In order to facilitate the understanding of the present disclosure, certain technical and scientific terms are specifically defined below. Unless otherwise specifically defined herein, all other technical and scientific terms used herein have the meanings generally understood by those of ordinary skill in the art to which the present disclosure pertains.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to".

"CD40" and "CD40 antigen" refer to an approximately 48 kD glycoprotein expressed on the surface of normal and neoplastic B cells, which acts as a receptor for signals involved in cellular proliferation and differentiation (Ledbetter et al., 1987, J. Immunol. 138:788-785). A cDNA molecule encoding CD40 has been isolated from a library prepared from the Burkitt lymphoma cell line Raji (Stamenkovic et al., 1989, EMBO J. 8:1403). Sequence information can be found in Table 2 of the present disclosure. A cell that endogenously expresses CD40 is any cell characterized by the surface expression of CD40, including but not limited to, normal and neoplastic B cells, interdigitating cells, basal epithelial cells, carcinoma cells, macrophages, endothelial cells, follicular dendritic cells, tonsil cells, and bone marrow-derived plasma cells.

"Antibody" is used in the broadest sense and encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. An antibody may refer to an immunoglobulin that is a four-peptide-chain structure formed by linking two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity determining regions (CDRs). Each of the light chain variable regions (VLs) and the heavy chain variable regions (VHs) consists of 3 CDRs and 4 FRs arranged from the amino-terminus to the carboxy-terminus in the following order: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3.

For the determination or definition of "CDRs", the deterministic depiction of CDRs and identification of residues comprising antigen-binding sites of the antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues do not significantly affect the antigen binding. As used herein, a CDR may refer to a CDR defined by any method known in the art, including combinations of methods. The correspondence between the various numbering schemes is well known to those skilled in the art, and examples are shown in Table 1 below.

**Table 1. The relationships between CDR numbering schemes**

| CDR | I MGT | Kabat | Ab M | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues of the VL and VH regions of the antibody or the antigen-binding fragment of the present disclosure accord with the known Kabat numbering scheme in terms of number and positions.

"Monoclonal antibody" or "mAb" refers to an antibody obtained from a population of substantially homogeneous antibodies, i.e., the individual antibodies included in the population are identical except for possible naturally-occurring mutations that may be present in minor amounts. Monoclonal antibodies are highly specific, being directed against a single antigenic site. Furthermore, in contrast to polyclonal antibody preparations, which typically include different antibodies directed against different determinants (epitopes), each monoclonal antibody is directed against a single determinant on the antigen. The modifier "monoclonal" indicates the characteristics of an antibody obtained from a population of substantially homogeneous antibodies, and is not to be construed as requiring production of the antibody by any particular method.

The term "rabbit antibody" herein refers to a monoclonal antibody directed against human CD40 or an epitope thereof prepared according to the knowledge and skill in the art. In the preparation, a test rabbit is injected with the CD40 antigen, and then antibodies expressed with the desired sequences or functional properties are isolated. In one specific embodiment of the present disclosure, the rabbit anti-human CD40 antibody or the antigen-binding fragment thereof may further comprise a light chain constant region of a rabbit κ or λ chain or a variant thereof, or further comprise a heavy chain constant region of a rabbit IgG1, IgG2, IgG3 or IgG4 or a variant thereof.

The term "fully human antibody" includes antibodies having variable and constant regions derived from human germline immunoglobulin sequences. The fully human antibody of the present disclosure may include amino acid residues not encoded by human germline immunoglobulin sequences (e.g., mutations introduced by random or site-specific mutagenesis *in vitro* or by somatic mutations *in vivo*). However, the term "fully human antibody" does not include antibodies in which CDR sequences derived from the germline of another mammalian species (such as rabbits) have been grafted into human framework sequences (i.e., "humanized antibody").

The term "humanized antibody", also known as a CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity.

The term "chimeric antibody" refers to an antibody obtained by fusing variable regions of an antibody of a first species to constant regions of an antibody of a second species, which can reduce an immune response induced by the antibody of the first species. As an example, the chimeric antibody is established by firstly establishing rabbits secreting a rabbit specific monoclonal antibody, isolating the antibody, then cloning a constant region gene of fully human antibody as required, linking the rabbit variable region gene and the human constant region gene into a chimeric gene, inserting the chimeric gene into a human vector, and finally expressing chimeric antibody molecules in a eukaryotic industrial system or prokaryotic industrial system. The constant region of the fully human antibody may be selected from the group consisting of the heavy chain constant regions of human IgG1, IgG2, IgG3 and IgG4 or variants thereof, preferably comprising human IgG1 or IgG4 heavy chain constant regions, or IgG1 mutated at amino acids without ADCC (antibody-dependent cell-mediated cytotoxicity) toxicity.

The term "antigen-binding fragment" includes a single-chain antibody (i.e., full-length heavy and light chains); a Fab, a modified Fab, a Fab', a modified Fab', an F(ab')2, an Fv, a Fab-Fv, a Fab-dsFv, a single domain antibody (e.g., VH or VL or VHH), an scFv, a bivalent or trivalent or tetravalent antibody, a Bis-scFv, a diabody, a tribody, a triabody, a tetrabody and an epitope-binding fragment of any one of the above (see, e.g., Holliger and Hudson, 2005, Nature Biotech. 23(9): 1126-1136; Adair and Lawson, 2005, Drug Design Reviews-Online 2(3), 209-217). Methods for producing and preparing such antibody fragments are well known in the art (see, e.g., Verma et al., 1998, Journal of Immunological Methods, 216, 165-181). Fab-Fv was first disclosed in WO2009/040562, and its disulfide-stabilized form Fab-dsFv was first disclosed in WO2010/035012. The antigen-binding fragment of the present disclosure also includes Fab and Fab' fragments described in WO2005/003169, WO2005/003170, and WO2005/003171. Multivalent antibodies may comprise multiple specificities (e.g., bispecificities) or may be monospecific (see, e.g., WO92/22583 and WO05/113605), and an example of the latter is Tri-Fab (or TFM) described in WO 92/22583.

The term "bind to CD40" refers to being able to interact with CD40 or an epitope thereof, wherein the CD40 or the epitope thereof may be derived from humans. The term "antigen-binding site" herein refers to a discontinuous three-dimensional spatial site on an antigen that is recognized by the antibody or the antigen-binding fragment of the present disclosure.

The term "antigen" refers to a molecule used for immunization of an immunocompetent vertebrate to produce an antibody that recognizes the antigen or to screen an expression library (e.g., particularly phage, yeast or ribosome display library). In the present disclosure, the antigen is defined in a broader sense, and includes a target molecule that is specifically recognized by the antibody, and a portion or a mimic of a molecule used in an immunization process for producing the antibody or in library screening for selecting the antibody. For the antibody of the present disclosure that binds to human CD40, monomers and polymers (e.g., dimers, trimers, etc.) of human CD40, and truncated variants and other variants of human CD40 are all referred to as antigens.

The term "epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described in the present disclosure, such as X-ray crystallography and two-dimensional nuclear magnetic resonance.

The term "specific binding" or "selective binding" refers to binding of an antibody to an epitope on a predetermined antigen. Generally, an antibody binds to a predetermined antigen or epitope thereof with an equilibrium dissociation constant (K_{D}) of about less than 10⁻⁷ M or even less and with an affinity that is at least twice as high as its affinity for binding to a non-specific antigen other than the predetermined antigen or the epitope thereof (or non-specific antigens other than closely related antigens, e.g., BSA, etc.), when determined by surface plasmon resonance (SPR) techniques in an instrument using human CD40 or an epitope thereof as an analyte and the antibody as a ligand. The term "antigen-recognizing antibody" is used interchangeably herein with the term "specifically bound antibody".

"Binding affinity" or "affinity" is used herein as a measure of the strength of a non-covalent interaction between two molecules (e.g., an antibody or a portion thereof and an antigen). The binding affinity between two molecules can be quantified by determining the dissociation constant (K_{D}). KD can be determined by measuring the kinetics of complex formation and dissociation using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods and can be calculated by methods such as those described by Caceci et al (1984, Byte 9:340-362) even for complex mixtures. For example, K_{D} can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA, and flow cytometry, as well as other assays exemplified elsewhere in the present disclosure. The binding kinetics and binding affinity of the antibody can also be evaluated by standard assays known in the art, such as surface plasmon resonance (SPR), e.g., by using the Biacore^{™} system or KinExA. The binding affinities associated with different molecular interactions, e.g., the binding affinities of different antibodies for a given antigen, can be compared by comparing the K_{D} values of antibody/antigen complexes. Similarly, the specificity of an interaction can be evaluated by determining and comparing the K_{D} value for the interaction of interest (e.g., a specific interaction between an antibody and an antigen) with the K_{D} value for an interaction not of interest (e.g., a control antibody known not to bind to CD40).

The term "conservative replacement" refers to replacement by another amino acid residue having similar properties to the original amino acid residue. For example, lysine, arginine, and histidine have similar properties in that they have basic side chains, and aspartic acid and glutamic acid have similar properties in that they have acidic side chains. Additionally, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties. "Inhibition" and "blocking" are used interchangeably and encompass both partial and complete inhibition/blocking. Inhibition/blocking of CD40 preferably reduces or alters the normal level or type of activity that occurs when CD40 binding occurs without inhibition or blocking. Inhibition and blocking are also intended to include any measurable decrease in CD40 binding affinity when in contact with an anti-CD40 antibody as compared to CD40 not in contact with an anti-CD40 antibody. "Inhibition of growth" (e.g., involving cells) is intended to include any measurable reduction in cell growth.

"Agonistic activity", "agonist activity" or "agonism" refers to the function as an agonist. The binding of an agonist to a cell receptor initiates a reaction or activity that is similar to or the same as that initiated by the receptor's natural ligand. For example, a CD40 agonist can induce any or all of the following responses: cell proliferation and/or differentiation; up-regulation of intercellular adhesion via such molecules as ICAM-1, E-selectin, and VCAM; secretion of pro-inflammatory cytokines such as IL-1, IL-6, IL-8, IL-12, and TNF; signal transduction through the CD40 receptor by such pathways as TRAF (e.g., TRAF2 and/or TRAF3), MAP kinases such as NIK (NF-κB inducing kinase), 1-κB kinases (IKKα/β), transcription factor NF-κB, Ras and the MEK/ERK pathway, the PI3K/Akt pathway, and the P38 MAPK pathway; transduction of anti-apoptotic signals by such molecules as XIAP, Mcl-1, and BCLx; B and/or T cell memory generation: B cell antibody production; B cell isotype switching; up-regulation of cell surface expression of MHC class II and CD80/86, and the like. "Antagonistic activity", "antagonist activity" or "antagonism" refers to the function of a substance as an antagonist. For example, an antagonist of CD40 can prevent or reduce any of the responses induced by the binding of the CD40 receptor to an agonist ligand, particularly CD40L. The antagonist may reduce any one or more of the responses induced by agonist binding by 5%, 10%, 15%, 20%, 25%, 30%, 35%, preferably 40%, 45%, 50%, 55%, 60%, more preferably 70%, 80%, 85%, and most preferably 90%, 95%, 99% or 100%. Methods for measuring anti-CD40 antibody and CD40-ligand binding specificity and antagonistic activity are known to those skilled in the art and include, but are not limited to, standard competitive binding assays, assays for monitoring immunoglobulin secretion by B cells, B cell proliferation assays, Banchereau-like B cell proliferation assays, T cell helper assays for antibody production, co-stimulation assays for B cell proliferation, and assays for up-regulation of B cell activation markers.

Methods for producing and purifying antibodies and antigen-binding fragments are well known in the prior art and can be found in, for example, "Antibodies: A Laboratory Manual", Cold Spring Harbor Press (chapters 5-8 and 15). For example, mice or rabbits can be immunized with human CD40 or a fragment thereof, and the resulting antibodies can be renatured and purified, and amino acid sequencing can be performed by conventional methods. Likewise, antigen-binding fragments can be prepared by conventional methods. The antibody or the antigen-binding fragment described herein is genetically engineered to contain one or more human FR regions in the non-human CDR regions. Human FR germline sequences are available from the ImMunoGeneTics (IMGT) website. Antibodies can be competitively screened for binding to the same epitope using conventional techniques known to those skilled in the art. For example, competition and cross-competition studies can be performed to obtain antibodies that compete or cross-compete with one another for binding to an antigen. A high-throughput method for obtaining antibodies that bind to the same epitope based on their cross-competition is described in International Patent Publication No. WO03/48731. Therefore, an antibody and an antigen-binding fragment thereof that compete for binding to the same epitope on CD40 with the antibody molecule of the present disclosure can be obtained by conventional techniques known to those skilled in the art.

The term "disorder" is any condition that would benefit from treatment with a humanized anti-CD40 antibody of the present disclosure. This includes chronic and acute disorders or diseases. Non-limiting examples of disorders to be treated by the present disclosure include cancer, hematological malignancies, benign and malignant tumors, leukemias and lymphoid malignancies and inflammatory, angiogenic, and autoimmune and immunological disorders.

The term "CD40-related disorder" or "CD40-related disease" refers to a condition in which modification or elimination of cells expressing CD40 is indicated. These cells include CD40-expressing cells demonstrating abnormal proliferation or CD40-expressing cells that are associated with cancerous or malignant growth. More specific examples of cancers that demonstrate abnormal expression of the CD40 antigen include B lymphoblastoid cells, Burkitt's lymphoma, multiple myeloma, T cell lymphomas, Kaposi's sarcoma, osteosarcoma, epidermal and endothelial tumors, pancreatic cancer, lung cancer, breast cancer, ovarian cancer, colon cancer, prostate cancer, head and neck cancer, skin cancer (melanoma), bladder cancer, and renal cancer. Such disorders include, but are not limited to, leukemias, lymphomas (including B cell lymphoma and non-Hodgkin lymphoma), multiple myeloma, Waldenstrom's macroglobulinemia; solid tumors, including sarcomas, such as osteosarcoma, Ewing sarcoma, malignant melanoma, adenocarcinoma (including ovarian adenocarcinoma), Kaposi's sarcoma/Kaposi's tumor and squamous cell carcinoma. "CD40-related disorders" also include diseases and disorders of the immune system, such as autoimmune disorders and inflammatory disorders. Such conditions include, but are not limited to, rheumatoid arthritis (RA), systemic lupus erythematosus (SLE), scleroderma, Sjögren's syndrome, multiple sclerosis, psoriasis, inflammatory bowel diseases (e.g., ulcerative colitis and Crohn's disease), pulmonary inflammation, asthma, and idiopathic thrombocytopenic purpura (ITP).

The term "arrests the growth of" or "growth inhibition" refers to inhibiting the growth or proliferation of a cell, especially a neoplastic cell type expressing the CD40 antigen. Thus, growth inhibition, for example, significantly reduces the percentage of neoplastic cells in S phase.

"Homology" or "identity" refers to sequence similarity between two polynucleotide sequences or between two polypeptides. When positions in two compared sequences are occupied by identical nucleotides or amino acid monomer subunits, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. In general, when two sequences are aligned, a comparison is performed to obtain the maximum homology percentage.

"Cell", "cell line", and "cell culture" are used interchangeably, and all such designations include their progeny. It should also be understood that all progeny may not be precisely identical in DNA content due to intentional or unintentional mutations. Mutant progeny with identical function or biological activity as those screened in the initially transformed cells are included.

"Optional" or "optionally" means that the event or circumstance subsequently described may, but does not necessarily, occur, and this description includes instances where the event or circumstance occurs or does not occur. For example, "optionally comprising 1-3 antibody heavy chain variable regions" means that the antibody heavy chain variable region of a particular sequence may, but does not necessarily, exist.

The "CD40-binding molecule" of the present disclosure is interpreted in a maximized sense, and includes the anti-CD40 antibody or the antigen-binding fragment thereof of the present disclosure, and any protein capable of achieving binding to CD40 is within the scope of the term. For example, a CD40-binding protein may comprise one or more effector molecules, e.g., in the form of a conjugate. The "effector molecule" alone may be therapeutically active (e.g., have anti-tumor activity or immune-activating or inhibiting activity) or have a detecting function, and may be in any form, such as biologically active proteins (e.g. enzymes), other antibodies or antibody fragments, synthetic or naturally occurring polymers, polynucleotides and fragments thereof (e.g., DNA and RNA and fragments thereof), radionuclides (particularly radioiodides), radioisotopes, chelated metals, nanoparticles and reporter groups (e.g., fluorescent compounds) or compounds that can be detected by NMR or ESR spectroscopy. Conjugation of the effector molecule to the anti-CD40 antibody or the antigen-binding fragment thereof of the present disclosure can be achieved by conventional methods. The term "antibody-drug conjugate" means that a ligand is linked to a biologically active drug by a stable linker unit. In the present disclosure, "antibody-drug conjugate" (ADC) means that a monoclonal antibody or an antibody fragment is linked to a drug molecule by a stable linker unit, wherein the antibody or the antibody fragment, through a specific group therein (such as an interchain disulfide bond), can bind to the drug molecule comprising a linker.

The term "drug loading" refers to the average number of drug molecules loaded per antibody-drug conjugate molecule in a population of antibody-drug conjugates, and may also be represented as the ratio of the number of drug molecules to the number of antibody molecules. The drug loading may range from 1 to 20, preferably from 1 to 10 drug molecules (D) linked per antibody (Ab). In an embodiment of the present disclosure, the drug loading is represented as k; illustratively, the drug loading may be an average of 1, 2, 3, 4, 5, 6, 7, 8, 9, 10, 11, 12, 13, 14, 15, 16, 17, 18, 19, 20, or a numerical value between any two of these numerical values, preferably an average of 1-10, and more preferably an average of 1-8, or 2-8, or 2-7, or 3-8, or 3-7, or 3-6, or 4-7, or 4-6, or 4-5. The average number of drug molecules per ADC molecule after the coupling reaction can be characterized by conventional methods such as UV/visible spectroscopy, mass spectrometry, an ELISA assay, a monoclonal antibody molecular size variant assay (CE-SDS), and HPLC.

The monoclonal antibody molecular size variant assay (CE-SDS) of the present disclosure may be used for quantitatively determining the purity of a recombinant monoclonal antibody product by adopting capillary electrophoresis-sodium dodecyl sulfate (CE-SDS) ultraviolet assay based on the molecular weight under reduced and non-reduced conditions and according to a capillary electrophoresis method (Chinese Pharmacopoeia 0542, 2015 Edition).

In one embodiment of the present disclosure, the drug molecule is coupled to the N-terminal amino of the ligand and/or ε-amino of the lysine residue through a linker unit, and generally, the number of drug molecules that can be coupled to the antibody in the coupling reaction will be less than the theoretical maximum.

The loading of the antibody-drug conjugate can be controlled by the following non-limiting methods, including:
(1) controlling a molar ratio of a linking reagent to a monoclonal antibody,
(2) controlling reaction time and temperature, and
(3) selecting different reaction reagents.

The term "about" or "approximately" as used herein means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprise" may mean a range of up to ±20%; for example, a pH of about 5.5 means a pH of 5.5±1.1. Furthermore, particularly for biological systems or processes, the term may mean up to an order of magnitude or up to 5-fold of a numerical value. Unless otherwise stated, when a specific value is provided in the present application and claims, the meaning of "about" or "substantially comprise" should be assumed to be within an acceptable error range for that specific value.

"Buffer" refers to a buffer that resists changes in pH by the action of its acid-base conjugate components. Examples of buffers that control the pH in an appropriate range include tris(hydroxymethyl)aminomethane (Tris), acetate, succinate, gluconate, histidine salt, oxalate, lactate, phosphate, citrate, tartrate, fumarate, glycylglycine, and other organic acid buffers.

"Citrate buffer" is a buffer comprising citrate ions. Examples of citrate buffers include citric acid-sodium citrate, citric acid-potassium citrate, citric acid-calcium citrate, citric acid-magnesium citrate, and the like. A preferred citrate buffer is a citric acid-sodium citrate buffer.

"Histidine salt buffer" is a buffer comprising histidine ions. Examples of histidine salt buffers include a histidine-hydrochloride buffer, a histidine-acetate buffer, a histidine-phosphate buffer, a histidine-sulfate buffer, and the like, and the histidine-hydrochloride buffer or histidine-acetate buffer is preferred. The histidine-acetate buffer is prepared from histidine and acetic acid, and the histidine-hydrochloride buffer is prepared from histidine and histidine hydrochloride, or histidine and hydrochloric acid.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. Preferably, the phosphate buffer is disodium hydrogen phosphate-sodium dihydrogen phosphate. "Tris(hydroxymethyl)aminomethane buffer" is a buffer comprising tris(hydroxymethyl) aminomethane (Tris). Examples of tris(hydroxymethyl)aminomethane buffers include a tris(hydroxymethyl)aminomethane-hydrochloric acid buffer ((Tris-HCl), a tris(hydroxymethyl) aminomethane-acetic acid buffer (Tris-AA), a tris(hydroxymethyl)aminomethane-succinic acid buffer (Tris-SA), a tris(hydroxymethyl)aminomethane-citric acid buffer (Tris-CA), and the like.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Pharmaceutical composition" refers to a mixture comprising one or more of the antibodies described herein and other chemical components, for example, physiological/pharmaceutically acceptable carriers and excipients. The purpose of the pharmaceutical composition is to maintain the stability of the active ingredient and promote the administration to an organism, which facilitates the absorption of the active ingredient, thereby exerting biological activity.

In the present disclosure, "pharmaceutical composition" and "formulation" are not mutually exclusive.

Unless otherwise stated, the solvent in the pharmaceutical composition described herein in solution form is water.

"Freeze-dried formulation" refers to a formulation or a pharmaceutical composition obtained by lyophilizing a pharmaceutical composition or a formulation in liquid or solution form *in vacuo*.

The pharmaceutical composition described herein can achieve a stable effect, that is, the anti-CD40 antibody or the antigen-binding fragment thereof in the pharmaceutical composition substantially retains its physical and/or chemical stability and/or biological activity after storage; for example, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical formulation is one in which no significant change is observed under the following conditions: storage at refrigeration temperature (2-8 °C) for at least 3 months, at least 6 months, at least 1 year, at least 2 years, or at most 2 years. In addition, stable liquid formulations include liquid formulations that exhibit desirable features after storage at 25 °C for periods including 1 month, 3 months, and 6 months, or storage at 40 °C for periods including 1 month. Typical acceptable criteria for stability are as follows: typically, no more than about 10%, for example, no more than about 5%, of antibody monomers are degraded as measured by SEC-HPLC. The pharmaceutical formulation is colorless, or clear to slightly opalescent, by visual analysis. The concentration, pH, and osmolality of the formulation have a change of no more than ±10%. Typically, clippings of no more than about 10%, for example, no more than about 5%, are observed. Typically, aggregation of no more than about 10%, for example, no more than about 5%, is formed.

An antibody "retains its physical stability" in a pharmaceutical formulation if it shows no significant increase in aggregation, precipitation and/or denaturation upon visual inspection of color and/or clarity, or as determined by UV light scattering, size exclusion chromatography (SEC), and dynamic light scattering (DLS). Changes in protein conformation can be assessed by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed proteins. Degradation processes that often change the chemical structure of proteins include hydrolysis or clipping (assessed by methods such as size exclusion chromatography and SDS-PAGE), oxidation (assessed by methods such as peptide mapping in combination with mass spectrometry or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

A GLP-1 analog "retains its biological activity" in a pharmaceutical formulation if the biological activity of an antibody at a given time is within a predetermined range of the biological activity exhibited during the preparation of the pharmaceutical formulation. The biological activity of an antibody can be determined, for example, by an antigen-binding assay.

"Administrating", "giving", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administering", "giving", and "treating" can refer to, for example, therapeutic, pharmacokinetic, diagnostic, research, and experimental methods. The treatment of cells comprises contacting a reagent with cells and contacting the reagent with a fluid, where the fluid is in contact with the cells. "Administering", "giving", and "treating" also refer to treating, e.g., cells by reagents, diagnosis, binding compositions or by another cell *in vitro* and *ex vivo*. "Treating", when applied to humans, veterinary, or research subjects, refers to therapeutic treatment, preventive or prophylactic measures, and research and diagnostic applications.

"Treatment" refers to administering, either internally or externally, a therapeutic agent, for example, comprising any one of the pharmaceutical compositions of the present disclosure, to a patient with one or more symptoms of a disease on which the therapeutic agent is known to have a therapeutic effect. Generally, the therapeutic agent is administered in an amount effective to alleviate one or more symptoms of the disease in the patient or population being treated to induce regression of such symptoms or to inhibit the development of such symptoms to any clinically measurable degree. The amount of therapeutic agent effective to alleviate any particular disease symptom (also known as a "therapeutically effective amount") may vary depending on a variety of factors, such as the disease state, age, and weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although an embodiment of the present disclosure (for example, a treatment method or an article of manufacture) may not be effective in alleviating every target disease symptom, it should alleviate the target disease symptom in a statistically significant number of patients as determined by any statistical testing methods known in the art, such as Student t-test, chi-square test, Mann and Whitney's U test, Kruskal-Wallis test (H test), Jonckheere-Terpstra test, and Wilcoxon test.

### The equipment and methods used in the detection are shown below:

### Appearance:

A visual method was used. The sample vial was wiped clean, and the color, clarity, and visible foreign matter of the sample were observed on a clarity tester under a white background and a black background at an illumination intensity of 1000-1500 1x.

Instrument for appearance examination: Jingtuo Instrument YB-2A clarity analyzer.

### pH value:

Potentiometry was used. 100 µL of a sample was taken, and the pH value was measured by selecting a micro pH meter calibrated by a standard solution.

pH measurement instrument: Mettler Toledo, model: S210.

### SEC molecular exclusion chromatography:

This is an analytical method that separates a solute based on the relative relationship between the pore size of the gel pores and the coil size of the polymer sample molecule. SEC monomer content percentage = A monomer/A total × 100% (A monomer represents the peak area of the main peak monomer in the sample, and A total represents the sum of all peak areas).

Instrument for SEC determination: Agilent 1260-Bio; chromatographic column: Waters, XBrige BEH200Å SEC (300 × 7.8 mm, 3.5 µm)

### NR-CE capillary gel electrophoresis:

This is a type of electrophoresis in which a gel is moved into a capillary as a support medium and a method that achieves separation under a certain voltage based on the molecular weight of the sample. Non-reduced CE purity percentage = A main peak/A total × 100% (A main peak represents the peak area of the main peak in the sample, and A total represents the sum of all peak areas).

Instrument for CE determination: Sciex model PA800 plus.

### icIEF imaged capillary isoelectric focusing electrophoresis:

This is a technique for separation according to the difference of isoelectric points (pI) of proteins. icIEF main peak content percentage = main peak area/total area × 100% (total area represents the sum of areas of acidic, main, and basic peaks).

Manufacturer of instrument for **icIEF** determination: Protein Simple, model: Muarice.

### Protein concentration determination:

Instrument for protein concentration determination: ultraviolet-visible spectrophotometer; model: Nano Drop 2000; optical path length: 1 mm.

### Melting temperature (Tm) and aggregation temperature (Tagg):

Tm is the temperature at which 50% of the components in a protein are denatured during the heating process; Tagg is the temperature at which the protein aggregates during the heating process. The sample was loaded into a Uni tube, and the analysis was run with a thermal ramp from 25 °C to 95 °C. Instrument for Tm and Taag analysis: Uncle, instrument manufacturer: Unchained.

### Viscosity measurement:

The viscosity of a sample was measured using a rheometer. 35 µL of a sample was loaded into a measurement cell, and the measurement cell was inserted into the instrument. Pressure was then applied to push the samples through microfluidic channels, and the speed of movement of the samples in each channel was tracked by the software to obtain their viscosity.

Measurement instrument: Honybun, instrument manufacturer: Unchained.

### Exemplary antibody pharmaceutical composition (formulation) preparation process

Step 1: The anti-CD40 antibody was obtained by cell culture and purification; the obtained anti-CD40 antibody was exchanged by ultrafiltration/concentration (UF/DF), and was prepared with a buffer, a stabilizer, and/or a surfactant into a formulation stock solution containing the anti-CD40 antibody; the formulation stock solution was sterilized and filtered through a 0.22 µm filter; and the filtrate was collected.

Step 2: The filling amount (the target filling amount was 2.835 mL/vial) was adjusted, and vials were selected for filling; and at the start of filling, during filling, and at the end of filling, samples were taken and examined for filling volume difference.

Step 3: Plugging was performed, the capping machine was started, aluminum caps were put on, and capping was performed.

Step 4: Visual inspection was performed to confirm that the products have no defects, such as inaccurate filling amount and poor appearance. Carton labels were printed. Cartons were folded. The products were packed in the cartons. The carton labels were put on the cartons.

### BRIEF DESCRIPTION OF THE DRAWINGS

FIGs. 1A to 1B: the activity of CD40 antagonistic antibodies in a reporter gene system. FIGs. 1A and 1B show the related results for 9E6-L4H2 and 2F12-L4H2, respectively. FIGs. 1A and 1B both use a human IgG1 isotype as a negative control and CFZ533 as a positive control.
FIGs. 2A to 2B: the inhibitory activity of CD40 antagonistic antibodies in a B cell activation assay system. FIG. 2A shows the percent inhibition of CD19+CD69+ cells by 9E6-L4H2 and 2F12-L4H2. FIG. 2B shows the MFI inhibition of CD19+CD69+ cells by 9E6-L4H2 and 2F12-L4H2. FIGs. 2A- 2B both use a human IgG1 isotype as a negative control and CFZ533 as a positive control.
FIGs. 3A to 3D: the inhibitory activity of CD40 antagonistic antibodies in a DC cell activation assay system. FIG. 3A shows the MFI inhibition of CD11C+CD80+ cells by 9E6-L4H2 and 2F12-L4H2.
FIG. 3B shows the MFI inhibition of CD11C+CD86+ cells by 9E6-L4H2 and 2F12-L4H2. FIG. 3C shows the inhibition of IL-12/23 p40 by 9E6-L4H2 and 2F12-L4H2. FIG. 3D shows the inhibition of TNFα by 9E6-L4H2 and 2F12-L4H2. FIGs. 3A-3D all use a human IgG1 isotype as a negative control and CFZ533 as a positive control.
FIG. 4: the endogenous agonistic activity of the CD40 antagonistic antibodies 9E6-L4H2 and 2F12-L4H2 in a B cell activation assay system, with a human IgG1 isotype, CFZ533 and the agonistic anti-CD40 antibody 9E5-SELFNS as controls.
FIGs. 5A to 5B: the activity of CD40 antagonistic antibodies in a mouse T cell-dependent humoral immune response model. FIG. 5A is a flowchart. FIG. 5B is a graph showing the results of the tests on days 7, 14, 21 and 28.
FIGs. 6A to 6B: the activity of CD40 antagonistic antibodies in a mouse skin graft rejection model. FIG. 6A is a flowchart. FIG. 6B shows skin graft survival rates (%) and skin graft scores.
FIG. 7: the activity of combinations of CD40 antagonistic antibodies with tacrolimus (FK506) in a mouse skin graft rejection model. A in FIG. 7 shows the skin graft survival rates (%) for 9E6-L4H2 (10 mpk) and its combination with tacrolimus (FK506). B in FIG. 7 shows the skin graft survival rates (%) for 2F12-L4H2 (10 mpk) and its combination with tacrolimus (FK506). C in FIG. 7 shows the skin graft scores for 9E6-L4H2 (10 mpk) and its combination with tacrolimus (FK506). D in FIG. 7 shows the skin graft scores for 2F12-L4H2 (10 mpk) and its combination with tacrolimus (FK506).
FIG. 8: the PK results of the CD40 antagonistic antibodies 9E6-L4H2 and 2F12-L4H2 in human CD40 transgenic mice, with CFZ533 as a control.
FIG. 9A: the inhibitory activity of Fc-mutation CD40 antagonistic antibodies in a B cell activation assay system. FIG. 9A shows the MFI inhibition of CD19+CD69+ cells by 9E6-L4H2 and 9E6-L4H2-AAYTE.
FIGs. 10A to 10B: the inhibitory activity of Fc-mutation CD40 antagonists in a DC cell activation assay system. FIG. 10A shows the inhibition of IL-12/23 p40 by 9E6-L4H2 and 9E6-L4H2-AAYTE.
FIG. 10B shows the inhibition of TNFα by 9E6-L4H2 and 9E6-L4H2-AAYTE.

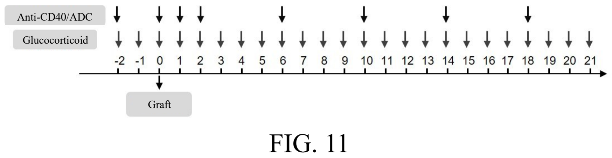

FIG. 11: a flow chart of the anti-CD40-ADC in a mouse skin graft rejection experiment

### DETAILED DESCRIPTION

The present disclosure is further described with reference to the following examples; however, these examples are not intended to limit the scope of the present disclosure.

Experimental procedures without specific conditions indicated in the examples or test examples of the present disclosure are generally conducted under conventional conditions, or under conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific origins indicated were commercially available conventional reagents.

Methods for preparing and purifying the anti-CD40 antibodies in the present application are described in the international patent application No. WO2023274201, which is incorporated herein by reference in its entirety.

### Example 1. CD40 Immunizing Antigens, Sequences for Antigen Screening and Preparation

The his-tagged human CD40 (h-CD40-his) recombinant protein (Cat. # CD0-H5228), the mouse Fc-tagged human CD40 (h-CD40-mFc) recombinant protein (Cat. # CD0-H525a), the his- and biotin-tagged human CD40 (hCD40-his-avi) recombinant protein (Cat. # CD0-H82E8), and the his-tagged cynomolgus monkey CD40 (cyno-CD40-his) recombinant protein (Cat. # CD0-C52H6) were all purified commercial protein reagents purchased from Acrobiosystems Inc., and the sources of their respective sequences are shown in Table 2. The protein reagents can be used in the experiments of the following examples.

**Table 2. Sources of amino acid sequences of recombinant proteins**

| Name | Start and end of amino acid sequence | GenBank accession No. |
|---|---|---|
| h-CD40-his | Glu21-Arg193 | P25942-1 |
| h-CD40- mFc | Glu21-Arg193 | P25942-1 |
| h-CD40-his-avi | Glu21-Arg193 | P25942-1 |
| cyno-CD40-his | Glu21-Arg193 | G7PG38 |

### Example 2. Screening for Anti-CD40 Rabbit Monoclonal Antibody and Preparation of Human-Rabbit Chimeric Antibody

Anti-human CD40 monoclonal antibodies were produced by immunizing 2 New Zealand white rabbits. The immunizing antigen was a His-tagged human CD40 recombinant protein (h-CD40-his, prepared into 1 µg/µL with phosphate buffer). Emulsification with Freund's adjuvants: complete Freund's adjuvant (CFA) was used for the first immunization, and incomplete Freund's adjuvant (IFA) was used for the boost immunizations. For each immunization, a multiple-site subcutaneous injection of 400 µg of the antigen was used. The injections for immunization were performed on days 0, 7, 20 and 41. Blood was collected on days 27 and 48 and tested, and antibody titers in rabbit serum were determined by ELISA and FACS. Rabbits in which the antibody titers were high in serum and tended to stabilize were selected, and each was intravenously injected with 400 µg of an antigen solution prepared with phosphate buffer on day 63 for boost immunization. On day 67, the spleens of these rabbits were collected, and a biotin-tagged CD40 antigen was added. Labeled monoclonal B cells were sorted into a 96-well plate using a flow cytometer. After 14 days of culture, supernatants were collected and screened by ELISA and FACS for clones that could bind to human CD40, cynomolgus monkey CD40 and Raji cells (a tumor cell line that expresses human CD40), and a total of 28 strains of B cell monoclones were obtained. The RNAs of these monoclonal cells were extracted and reverse-transcribed, and after PCR amplification, the products were sent to a sequencing company for sequencing. Finally, the sequences of 28 rabbit antibodies were obtained. These antibodies were screened by affinity and activity assays (see Examples 2-3), and a monoclonal antibody was obtained. Its heavy and light chain variable region sequences are shown in Table 3, and the CDR sequences are shown in Table 4.

**Table 3. Variable region sequences of anti-CD40 rabbit monoclonal antibody**

| No. | Heavy chain variable region and light chain variable region sequences | |
|---|---|---|
| 9E6 | VH | QSVRESEGGLVKPTDTLTLTCTVSGFSLSSYD MSWVRQAPGNGLEWIGAIGGAGGTYYASWAKSRSTITRNTNLNTVTLK |
| | | MSSLTAADTATYFCARGWTRLDLWGQGTLVTVSS (SEQ ID NO:1) |
| | VL | ADIVLTQTPSPVSGAVGGTVTIKCQASEDISSNLAWYQQKPGQPPKLLIFPASNLASG VSSRFKGSGSGTEFTLTISDLECADAATYYCQGGYWTSTSNFGNGFGGGTEVVVK (SEQ ID NO:2) |

| | | |
|---|---|---|
| (Note: The CDR regions of heavy and light chain variable regions are underlined and determined using the Kabat numbering scheme) | | |

**Table 4. CDR regions of anti-CD40 rabbit monoclonal antibody (using the Kabat numbering scheme)**

| No. | Heavy chain CDR | | Light chain CDR | |
|---|---|---|---|---|
| 9E6 | HCDR1 | SYD MS (SEQ ID NO:3) | LCDR1 | QASEDISSNLA (SEQ ID NO:6) |
| | HCDR2 | AIGGAGGTYYASWAKS (SEQ ID NO:4) | LCDR2 | PASNLAS (SEQ ID NO:7) |
| | HCDR3 | GWTRLDL (SEQ ID NO:5) | LCDR3 | QGGYWTSTSNFGNG (SEQ ID NO:8) |

| | | | | |
|---|---|---|---|---|
| The obtained variable region sequences were connected to a human antibody IgG1 constant region (with the mutation N297A, according to the Eu numbering scheme) sequence and a human kappa chain constant region sequence, respectively, to obtain human-rabbit chimeric antibody sequences. The sequences of the chimeric antibody were inserted into expression vectors by molecular cloning, and a human-rabbit chimeric antibody was obtained using a HEK293 cell expression system. | | | | |

### Example 3. Binding of Human-Rabbit Chimeric Anti-CD40 Antibodies to Raji Cells

Raji cells are of a tumor cell line that overexpresses human CD40. 2E5 Raji cells were inoculated onto a 96-well plate. 100 µL of a test antibody was added, with the highest final concentration of 100 nM. The antibody was 5-fold diluted to 8 concentrations. The plate was incubated at 4 °C for 1 h. The plate was washed once with a washing buffer, and an AF647-conjugated anti-human IgG antibody (Jackson Immunoresearch Laborotories, Cat. # 205-609-088) was added at a dilution ratio of 1:500. The plate was incubated at 4 °C for 30 min. The plate was washed once with the washing buffer, and fluorescence intensity was measured using a flow cytometer. The binding EC₅₀ values of the anti-CD40 antibodies for CD40 were calculated. The anti-CD40 antagonistic antibody CFZ533 (i.e., iscalimab, Nova) was used as a positive control, and the heavy and light chain variable region sequences were derived from sequence 5 and sequence 2 of US8277810B, respectively.

The results in Table 5 show that 9E6 has a stronger binding EC₅₀ than CFZ533.

**Table 5. FACS binding EC₅₀ of human-rabbit chimeric anti-CD40 antibodies for Raji cells (nM)**

| No. | Binding EC₅₀ (nM) |
|---|---|
| 9E6 | 0.608 |
| CFZ533 | 2.410 |

### Example 4. Reporter Gene Cell Activity of Human-Rabbit Chimeric Anti-CD40 Antibodies

HEK-Blue CD40L cells were purchased from Invivogen (Cat. # hkb-cd40). The cells were stably transfected with the human CD40 gene and the NF-kB-mediated SEAP genome. The activation level of the CD40 signaling pathway can be characterized by detecting the secreted SEAP in the supernatant using QUANTI-Blue, the substrate of SEAP. In this experiment, the *in vitro* antagonist activity of anti-CD40 antibodies was assessed according to IC₅₀ by detecting the activation of HEK-Blue CD40L cells by CD40L. HEK-Blue CD40L cells were cultured in a DMEM medium containing 10% FBS, 100 µg/mL Zeocin, and 30 µg/mL Blasticidin, and passaged 2-3 times in a week at a ratio of 1:5 or 1: 10. During passaging, the medium was pipetted off, and the cell layer was rinsed with 5 mL of 0.25% pancreatin. Then the pancreatin was pipetted off, the cells were digested in an incubator for 3-5 min, and then a fresh medium was added to resuspend cells. 100 µL of cell suspension was added to a 96-well cell culture plate at a density of 5 × 10^5 cells/mL. The medium was DMEM containing 10% FBS, 100 µg/mL Zeocin, and 30 µg/mL Blasticidin. Only 100 µL of sterile water was added to the peripheral wells of the 96-well plate. The plate was incubated in an incubator for 24 h (37 °C, 5% CO₂). After cell adhesion, serially diluted test antibodies were added at 100 µL/well, and the plate was incubated at 37 °C for 30 min. 25 ng/mL CD40L (R&D, Cat. # 2706-CL) and 2 µg/mL anti-His antibody (R&D, Cat. # MAB050) were added, and the plate was incubated in an incubator for 20-24 h (37 °C, 5% CO2). 20 µL of cell supernatant was taken from each well to a new 96-well flat-bottomed plate, and 180 µL of QUANTI-Blue substrate solution was added. The plate was incubated in the dark in an incubator for 1-3 h. The absorbance at 630 nm was measured with a microplate reader (Thermo MultiSkanFc), and IC₅₀ values were calculated and used to assess the *in vitro* cell activity of the anti-CD40 antibodies.

See Table 6. The results show that 9E6 has reporter gene cell inhibitory activity IC₅₀ similar to or slightly stronger than CFZ533.

**Table 6. Reporter gene cell activity IC₅₀ of anti-CD40 antibodies (nM)**

| No. | IC₅₀ (nM) |
|---|---|
| 9E6 | 0.2206 |
| CFZ533 | 0.2715 |

### Example 5. Humanization of Anti-CD40 Antibody

The sequences of the heavy and light chain variable regions of the rabbit antibody 9E6 were compared with the GermLine database of antibodies to obtain a human germline template with high homology. The human germline template information for the humanization of the final antibody is shown in Table 7.

**Table 7. Human germline template information for the humanization of antibody**

| No. | Heavy chain variable region | | Light chain variable region | |
|---|---|---|---|---|
| | FR1-FR3 | FR4 | FR1-FR3 | FR4 |
| 9E6 | IGHV4-30-4*02 | IGHJ1*01 | IGkV1-9*01 | IGKJ4*01 |

The CDR regions of the rabbit antibody were grafted to the selected human germline template to replace the human germline variable regions and recombined with the corresponding human IgG constant regions (preferably, the heavy chain was IgG1 with the mutation N297A and the light chain was κ). Then on the basis of the three-dimensional structures of the rabbit antibody, back mutation was performed on the embedded residues, the residues that directly interact with the CDR regions, and the residues that greatly affect the conformations of VL and VH, and mutation was performed on potential post-translational modification risk sites to obtain the final humanized molecules. By way of example, the humanized light and heavy chain variable region sequences corresponding to 9E6 are shown (see Table 8), where L represents the light chain, H represents the heavy chain, and the numbers following L and H represent different versions of the humanized sequences containing different back mutations.

**Table 8. Heavy and light chain variable region sequences of the humanized 9E6**

| Heavy and light chain variable region sequences | | Sequence No. |
|---|---|---|
| 9E6-L1 | DIQLTQSPSFLSASVGDRVTITCQASEDISSNLAWYQQKPGKPPKLLI FPASNLASGVPSRFSGSGSGTEFTLTISSLQPEDFATYYCQGGYWTST SNFGNVFGGGTKVEIK | SEQ ID NO:9 |
| 9E6-L2 | DIQLTQSPSFLSASVGDRVTITCQASEDISSNLAWYQQKPGKPPKLLI FPASNLASGVPSRFSGSGSGTEFTLTISSLQPEDFATYYCQGGYWTST SNFGSGFGGGTKVEIK | SEQ ID NO:10 |
| 9E6-L3 | DIQLTQSPSFLSASVGDRVTITCQASEDISSNLAWYQQKPGKPPKLLI FPASNLASGVPSRFSGSGSGTEFTLTISSLQPEDFATYYCQGGYWTST SNFGTGFGGGTKVEIK | SEQ ID NO:11 |
| 9E6-L4 | DIQLTQSPSFLSASVGDRVTITCQASEDISSNLAWYQQKPGKPPKLLI FPASNLASGVPSRFSGSGSGTEFTLTISSLQPEDFATYYCQGGYWTST SNFGQGFGGGTKVEIK | SEQ ID NO:12 |
| 9E6-H1 | EVQLQESGPGLVKPSDTLSLTCTVSGFSLSSYD MSWIRQPPGKGLEWIGAIGGAGGTYYASWAKSRVTISVDTSLNQVS LKLSSVTAADTAVYYCARGWTRLDLWGQGTLVTVSS | SEQ ID NO:13 |
| 9E6-H2 | QVQVQESGPGLVKPSDTLSLTCTVSGFSLSSYD MSWIRQPPGKGLEWIGAIGGAGGTYYASWAKSRVTISVDTSLNQVS LKLSSVTAADTAVYYCARGWTRLDLWGQGTLVTVSS | SEQ ID NO:14 |

| | | |
|---|---|---|
| (The CDRs defined by the Kabat numbering scheme are underlined.) | | |

During the humanization of 9E6, the following LCDR3s were obtained:
>LCDR3 of 9E6-L1
   QGGYWTSTSNFGNV (SEQ ID NO: 15)
>LCDR3 of 9E6-L2
   QGGYWTSTSNFGSG (SEQ ID NO: 16)
>LCDR3 of 9E6-L3
   QGGYWTSTSNFGTG (SEQ ID NO: 17)
>LCDR3 of 9E6-L4
   QGGYWTSTSNFGQG (SEQ ID NO: 18)
>General formula for LCDR3s of 9E6-Ls
   QGGYWTSTSNFGX₉X₁₀ (SEQ ID NO: 19), wherein X₉ is selected from the group consisting of N, S, T, and Q, and X₁₀ is selected from the group consisting of V and G.

The various versions of light and heavy chains of each of the rabbit antibodies were combined in pairs, expressed and purified. The names are combinations of the heavy chain variable region number and the light chain variable region number. For example, "9E6-L1H2" refers to an antibody with SEQ ID NO: 9 as the VH and SEQ ID NO: 13 as the VL. The light and heavy chain constant regions used the human IgG1 constant region (with the mutation N297A, according to the Eu numbering scheme) sequence and the human kappa chain constant region sequence, respectively.

By way of example, the sequences of the humanized molecule 9E6-L4H2 (using the 9E6-L4 light chain and the 9E6-H2 heavy chain) are shown below, where the light and heavy chain constant regions used the human IgG1 constant region (with the mutation N297A, according to the Eu numbering scheme) sequence and the human kappa chain constant region sequence, respectively.
>9E6-L4H2 HC: 
QVQVQESGPGLVKPSDTLSLTCTVSGFSLSSYD
MSWIRQPPGKGLEWIGAIGGAGGTYYASWAKSRVTISVDTSLNQVSLKLSSVTAADTAVYY
CARGWTRLDLWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDKKVEPKS
CDKTHTCPPCPAPELLGGPSVFLFPPKPKDTL
MISRTPEVTCVVVDVSHEDPEVKFNWYVDGVEVHNAKTKPREEQYASTYRVVSVLTVLHQ
DWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQ
QGNVFSCSV MHEALHNHYTQKSLSLSPGK SEQ ID NO: 20
>9E6-L4H2 LC:

DIQLTQSPSFLSASVGDRVTITCQASEDISSNLAWYQQKPGKPPKLLIFPASNLASGVPSRFSG
SGSGTEFTLTISSLQPEDFATYYCQGGYWTSTSNFGQGFGGGTKVEIKRTVAAPSVFIFPPSDE
QLKSGTASVVCLLNNFYPREAKVQWKVDNALQSGNSQESVTEQDSKDSTYSLSSTLTLSK
ADYEKHKVYACEVTHQGLSSPVTKSFNRGEC SEQ ID NO: 21

The heavy chain constant region of the above antibody was engineered. The antibody used the human IgG1 constant region and contained 5 point mutations: L234A, L235A, M252Y, S254T, and T256E (according to the Eu numbering scheme), and the light chain constant region and the light and heavy chain variable regions were unchanged. The newly produced antibody was 9E6-L4H2-AAYTE, the complete heavy chain sequence of which is shown below:
>9E6-L4H2-AAYTE HC:

QVQVQESGPGLVKPSDTLSLTCTVSGFSLSSYD
MSWIRQPPGKGLEWIGAIGGAGGTYYASWAKSRVTISVDTSLNQVSLKLSSVTAADTAVYY
CARGWTRLDLWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDKKVEPKS
CDKTHTCPPCPAPEAAGGPSVFLFPPKPKDTLYITREPEVTCVVVDVSHEDPEVKFNWYVD
GVEVHNAKTKPREEQYNSTYRVVSVLTVLHQDWLNGKEYKCKVSNKALPAPIEKTISKAK
GQPREPQVYTLPPSREE
MTKNQVSLTCLVKGFYPSDIAVEWESNGQPENNYKTTPPVLDSDGSFFLYSKLTVDKSRWQ
QGNVFSCSV MHEALHNHYTQKSLSLSPGK SEQ ID NO: 22

The heavy and light chain constant regions are underlined.

Exemplary IgG Fcs are shown below:
> IgG1-Fc(N297A) 
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPSVF
LFPPKPKDTL
MISRTPEVTCVVVDVSHEDPEVKFNWYVDGVEVHNAKTKPREEQYASTYRVVSVLTVLHQ
DWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQ
QGNVFSCSV MHEALHNHYTQKSLSLSPGK SEQ ID NO: 23
> IgG1-Fc(AAYTE)

ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPEAAGGPSVF
LFPPKPKDTLYITREPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQ
QGNVFSCSV MHEALHNHYTQKSLSLSPGK SEQ ID NO: 24

### Example 6. Binding of Humanized Anti-CD40 Antibodies to Raji Cells

Raji cells highly express human CD40 on the cell surface and thus can be used to detect the binding properties of CD40 antagonistic antibodies to the cell surface human CD40.

Raji cells were plated at 1.5E5 cells/well, and various concentrations of CD40 antagonistic antibodies were added. The plate was incubated at 4 °C for 1 h. After two washes with FACS buffer (PBS + 2% FBS), a secondary antibody (Alexa Flour488 conjugated anti-human IgG (H+L) antibody) was added, and the plate was incubated at 4 °C for 0.5 h. After two washes with FACS buffer, the fluorescence intensity on the cell surface was measured by flow cytometry (BD FACS Celesta).

According to the results shown in Table 9, 9E6-L4H2 and CFZ533 have similar abilities to bind to Raji cell surface human CD40.

**Table 9. EC₅₀ results for the FACS binding of humanized anti-CD40 antibodies to Raji cells**

| Antibody No. | Binding EC₅₀ (nM) |
|---|---|
| 9E6 | 0.895 |
| 9E6-L1H1 | 1.083 |
| 9E6-L1H2 | 1.048 |
| 9E6-L2H1 | 1.993 |
| 9E6-L2H2 | 1.660 |
| 9E6-L3H1 | 1.065 |
| 9E6-L3H2 | 1.148 |
| 9E6-L4H1 | 0.928 |
| 9E6-L4H2 | 0.870 |
| CFZ533 | 1.009 |

### Example 7. Assays for Affinities of Anti-CD40 Antibodies for Human CD40 and Cynomolgus Monkey CD40

The test antibody was affinity-captured on an anti-human Fc chip, and a series of concentrations of His-tagged human or cynomolgus monkey CD40 antigen were allowed to flow over the chip surface. Reaction signals were detected in real time using a Biacore instrument, and thus association and dissociation curves were obtained. The buffer used in the experiment was HBS-EP + 10× buffer solution (Cat. # BR-1006-69, GE) diluted to 1× (pH 7.4) with D. I. Water. Fitting was performed on the data obtained from the experiment using a (1:1) Binding model to obtain affinity values. See Table 10.

9E6-L4H2 has a similar binding constant for human CD40 to CFZ533, and 9E6-L4H2 has a stronger binding affinity for cynomolgus monkey CD40.

**Table 10. SPR affinities of CD40 antagonistic antibodies for CD40 of different species**

| Mobile phase | Stationary phase | Ka (1/ Ms) | Kd (1/s) | KD (M) |
|---|---|---|---|---|
| Human CD40 | 9E6-L4H2 | 4.13E+05 | 2.21E-04 | 5.36E-10 |
| | CFZ533 | 6.88E+05 | 1.41E-04 | 2.05E-10 |
| Cynomolgus monkey CD40 | 9E6-L4H2 | 5.14E+05 | 6.23E-05 | 1.21E-10 |

### Example 8. Reporter Gene Cell Activity of Anti-CD40 Antibodies

HEK-Blue CD40L cells were purchased from Invivogen (Cat. # hkb-cd40). The cells were stably transfected with the human CD40 gene and the NF-kB-mediated SEAP genome. The activation level of the CD40 signaling pathway can be characterized by measuring the amount of secreted SEAP in the supernatant using QUANTI-Blue, the substrate of SEAP. In the assays, the inhibitory effects of the CD40 antagonistic antibodies on the CD40L-induced HEK-Blue CD40L cell activation were measured, and the *in vitro* cell activity of the CD40 antagonistic antibodies was assessed based on IC₅₀.

HEK-Blue CD40L cells were cultured in a DMEM medium containing 10% FBS, 100 µg/mL Normocin, 100 µg/mL Zeocin, and 30 pg/mL Blasticidin, with 2-3 passages a week. HEK-Blue CD40L cells were plated at 5E4 cells/well into a 96-well cell culture plate (the medium was DMEM containing 10% FBS and 100 µg/mL Normocin) and incubated overnight. After cell adhesion, serially diluted test antibodies were added at 100 µL/well, and the plate was incubated at 37 °C for 1 h. CD40L-his (R&D, 2706-CL-025) and anti-His antibody (R&D, MAB050-500) were added, and the plate was incubated overnight. After cell centrifugation, 20 µL of the cell supernatant was transferred to a new 96-well white plate, and 180 µL of QUANTI-Blue substrate solution was added. The plate was incubated in the dark for 15 min. The absorbance at 620 nm was measured with an Envision microplate reader, and IC₅₀ values were calculated and used to assess the *in vitro* cell activity of the CD40 antagonistic antibodies.

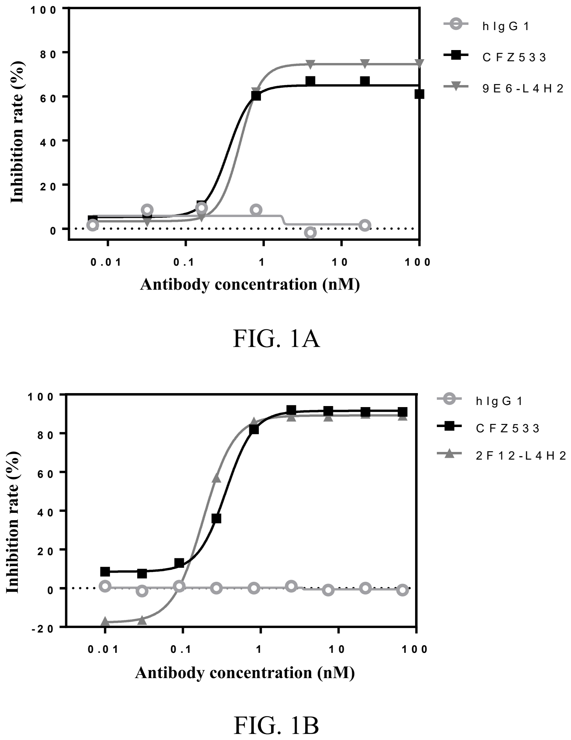

According to the results shown in Table 11 and FIGs. 1A to 1B, 9E6-L4H2 and CFZ533 have similar inhibitory activity against the reporter gene system, and 2F12-L4H2 has better inhibitory activity than CFZ533. 2F12-L4H2 was another anti-CD40 antibody obtained by screening in the present application.

**Table 11. IC₅₀ for the inhibition of CD40 reporter gene cell activity by anti-CD40 antibodies**

| | Antibody No. | IC₅₀ (nM) |
|---|---|---|
| Experiment 1 | 2F12-L4H2 | 0.18 |
| | CFZ533 | 0.36 |
| Experiment 2 | 9E6-L4H2 | 0.50 |
| | CFZ533 | 0.35 |

### Example 9. Inhibitory Activity of Anti-CD40 Antibodies in B Cell Activation Assay System

CD40 is highly expressed in B cells, and CD40L, upon binding to CD40, can induce B cell activation and up-regulate the expression of a series of activation markers. CD40 antagonistic antibodies block the binding of CD40L to CD40, thereby eliminating the immune activation process of B cells.

Human PBMCs were plated at 2E5 cells/well and 50 µL/well into a 96-well cell culture plate (the medium was RPMI-1640 containing 10% FBS and 1% penicillin-streptomycin). Serially diluted test antibodies were added at 50 µL/well, and the cells were co-incubated at 37 °C in 5% CO₂ for 0.5 h. CD40L-his and an anti-His antibody were added to each well for overnight stimulation. The following day, the plate was centrifuged, the supernatant was removed, and the cells were washed twice with FACS buffer and stained at room temperature for 15 min with 100 µL of Fixable viability dye EF780 (Invitrogen, 65086514) diluted at 1:1000. The cells were washed twice and then blocked at room temperature for 10 min with 100 µL of human Fc blocker (BD, 564220) diluted at 1:200. After centrifugation, the cells were incubated at 4 °C for 0.5 h with flow cytometry antibodies (PerCP/Cyanine5.5 anti-human CD19 (Biolegend, 302230), and APC anti-human CD69 (Biolegend, 310910)) diluted at 1:200. After centrifugation, the supernatant was removed, the cells were washed twice with FACS buffer and then resuspended in 200 µL of PBS, and the fluorescence intensity on the cell surface was measured by a flow cytometer (BD FACS Celesta).

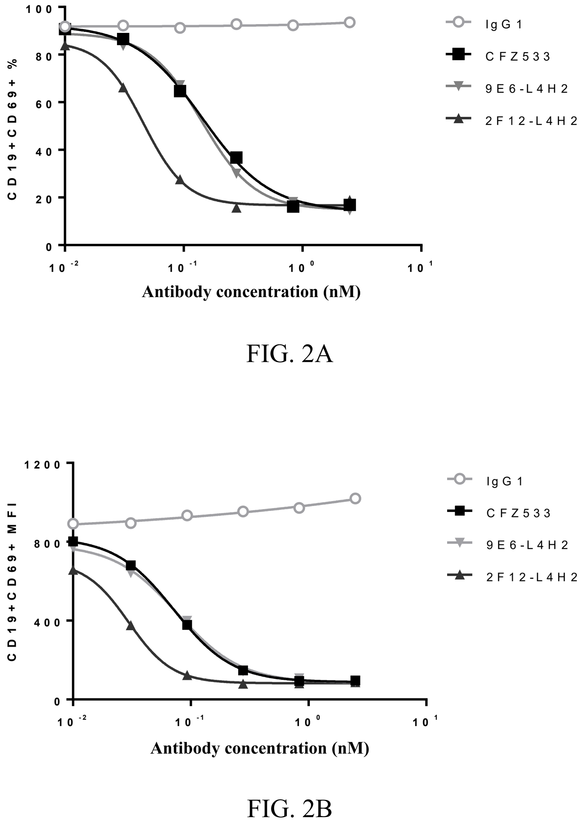

According to the results shown in Table 12 and FIGs. 2A to 2B, 9E6-L4H2 has similar B cell activation inhibitory activity to CFZ533. 2F12-L4H2 has greater B cell inhibitory activity than CFZ533. 2F12-L4H2 was another anti-CD40 antibody obtained by screening in the present application.

**Table 12. Inhibitory activity of humanized anti-CD40 antibodies in B cell activation assay system**

| | CD19+CD69+% | | CD19+CD69+ MFI | |
|---|---|---|---|---|
| Antibody No. | IC₅₀ (nM) | Maximum inhibition rate | IC₅₀ (nM) | Maximum inhibition rate |
| CFZ533 | 0.14 | 95% | 0.07 | 110% |
| 9E6-L4H2 | 0.14 | 98% | 0.07 | 110% |
| 2F12-L4H2 | 0.04 | 93% | 0.03 | 111% |

After antibody addition, the CD19+CD69+ signal was lower than the background, probably because the background activation of B cells was inhibited to some extent (in addition to the inhibition of the CD40L-induced signal).

### Example 10. Inhibitory Activity of Anti-CD40 Antibodies in DC Cell Activation Assay System

CD40 is highly expressed on dendritic cells (DCs), and CD40L, upon binding to CD40, can induce DC activation, up-regulate the expression of multiple activation markers on the DC cell surface, and promote the secretion of multiple inflammatory factors from DCs, further amplifying immune responses. CD40 antagonistic antibodies block the binding of CD40L to CD40, thereby eliminating the immune activation process of DC cells.

Monocytes were sorted and enriched from fresh primary human peripheral blood PBMCs using the EsaySepTM human CD14 sorting kit (Stemcell, 19359), and differentiated for 6 days with RPMI-1640 medium (10% FBS and 1% penicillin-streptomycin), 50 ng/mL IL-4 (PeproTech, 200-04), and 50 ng/mL GM-CSF (PeproTech, 300-03). On day 7, the differentiated DC cells were plated at 1E5/well into a 96-well cell culture plate. Serially diluted test antibodies were added to the wells, and the cells were co-incubated at 37 °C in 5% CO₂ for 0.5 h. CD40L-his and an anti-His antibody were then added to each well at final concentrations. After 48 h of culture, the level of DC cell activation was measured by flow cytometry: after centrifugation, the supernatant was removed, and the cells were washed twice with FACS buffer (PBS + 2% FBS) and stained at room temperature for 15 min with 100 µL of Fixable viability dye EF780 (Invitrogen, 65086514) diluted at 1:1000. The cells were washed twice and then blocked at room temperature for 10 min with 100 µL of human Fc blocker (BD, 564220) diluted at 1:200. After centrifugation, the cells were incubated at 4 °C for 0.5 h with flow cytometry antibodies (Alexa Fluor^{®} 700 anti-human CD11c (Biolegend, 337220), Brilliant Violet 421^{™} anti-human CD80 (Biolegend, 305221), and APC anti-human CD86 (Biolegend, 305412)) diluted at 1:200. After centrifugation, the supernatant was removed, the cells were washed twice with FACS buffer and then resuspended in 200 µL of PBS, and the fluorescence intensity on the cell surface was measured by a flow cytometer (BD FACS Celesta).

In addition, the level of cytokine secretion in the supernatant was measured after 24 h (TNFα, Cisbio, 62HTNFAPEG) and 48 h (IL-12/23 p40, Novus, VAL121) of culture.

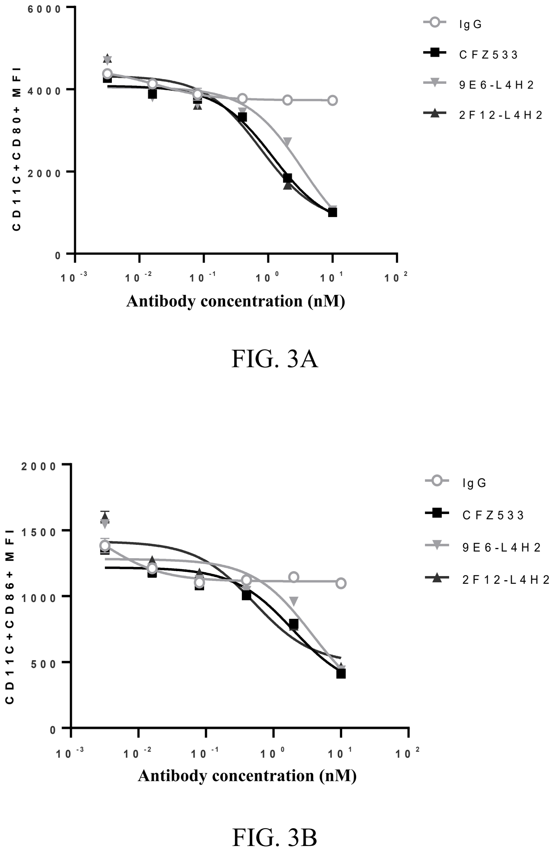

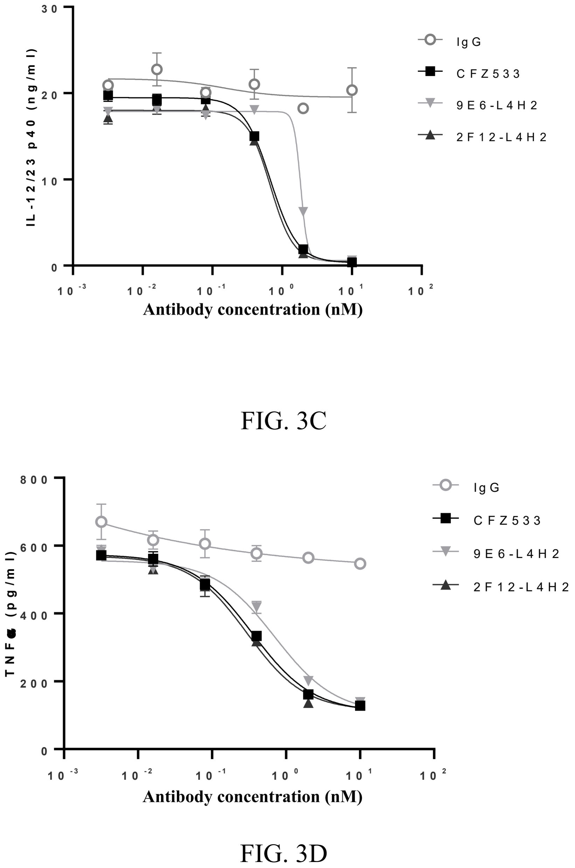

According to the results shown in Table 13 and FIGs. 3A to 3D, 9E6-L4H2 and 2F12-L4H2 have similar DC cell inhibitory activity to the control antibody CFZ533. 2F12-L4H2 was another anti-CD40 antibody obtained by screening in the present application.

**Table 13. Inhibitory activity of humanized anti-CD40 antibodies in DC cell activation assay system**

| Antibody No. | IC₅₀ (nM) in DC cell activation assay system | | | |
|---|---|---|---|---|
| | CD80 MFI | CD86 MFI | IL-12/23 p40 | TNFα |
| CFZ533 | 1.19 | 1.93 | 0.68 | 0.35 |
| 9E6-L4H2 | 3.44 | 3.72 | 1.85 | 0.7 |
| 2F12-L4H2 | 0.78 | 0.52 | 0.68 | 0.30 |

### Example 11. Agonistic Activity of Humanized Anti-CD40 Antibodies in B Cell Activation Assay System

CD40, which belongs to the TNF superfamily of receptors, mediates the specific and non-specific activation of downstream signaling pathways upon binding to the ligand CD40L or being cross-linked by antibodies. Therefore, the background agonist activity of CD40 antagonistic antibodies on B cells can be measured without adding CD40L.

Human PBMCs were plated at 2E5 cells/well and 100 µL/well into a 96-well cell culture plate (the medium was RPMI-1640 containing 10% FBS and 1% penicillin-streptomycin). Serially diluted test antibodies were added at 100 µL/well, and the cells were incubated overnight at 37 °C in 5% CO₂. The following day, the plate was centrifuged and the supernatant was removed, and the cells were washed twice with FACS buffer and stained at room temperature for 15 min with 100 µL of Fixable viability dye EF780 diluted at 1:1000. The cells were washed twice and then blocked at room temperature for 10 min with 100 µL of human Fc blocker diluted at 1:200. After centrifugation, the cells were incubated at 4 °C for 0.5 h with flow cytometry antibodies (PerCP/Cyanine5.5 anti-human CD19 (Biolegend, 302230), and APC anti-human CD69 (Biolegend, 310910)) diluted at 1:200. After centrifugation, the supernatant was removed, the cells were washed twice with FACS buffer and then resuspended in 200 µL of PBS, and the fluorescence intensity on the cell surface was measured by a flow cytometer (BD FACS Celesta).

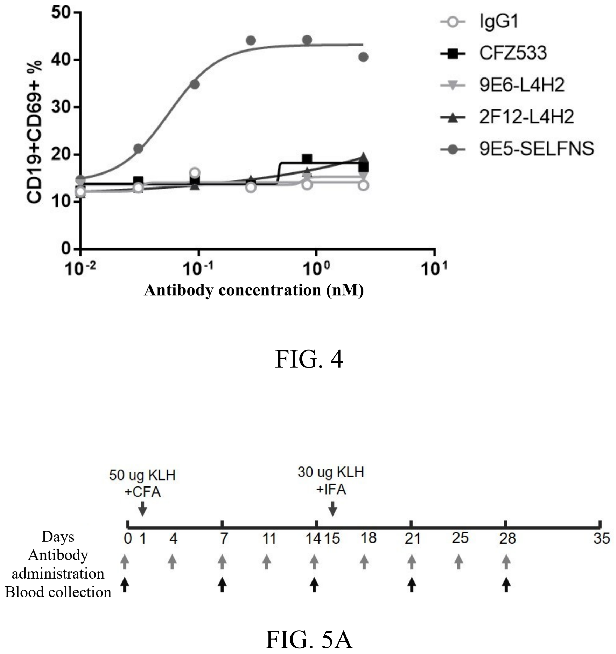

According to the results shown in FIG. 4, the CD40 agonistic antibody 9E5-SELFNS (WO2020108611A1) activated B cells in a dose-dependent manner, while 9E6-L4H2 and 2F12-L4H2 still showed no significant B cell agonistic activity at 2.5 nM.

### Example 12. Mouse T Cell-Dependent Humoral Immune Response (TDAR) Model

Female human CD40 transgenic mice, 6-7 weeks old, were purchased from Biocytogen Jiangsu Co., Ltd. Housing environment: SPF; production license: SCXK(Jiangsu)-2016-0004; human CD40 transgenic mouse certification number: 320726200100167773. After arrival, the animals were acclimatized for 7 days and randomly grouped. On day 0 of the experiment, one of the mice in each group was subjected to blood collection followed by intraperitoneal injection. On day 1, each mouse was intraperitoneally injected with 50 µg of KLH (KLH:complete Freund's adjuvant (CFA) = 1:1 emulsified immune complex) for immunization. On day 15 of the experiment, each mouse was intraperitoneally injected with 50 µg of KLH (KLH:incomplete Freund's adjuvant (IFA) = 1:1 emulsified immune complex) for a second immunization. Each group was intraperitoneally injected twice a week, and blood samples of about 150 µL were collected from the orbital venous plexus on days 7, 14, 21, and 28. Whole blood was left to stand at room temperature for 1-4 h and centrifuged at 7000 rpm at 4 °C for 10 min to separate serum, and the serum was stored at -80 °C for later use. The specific experimental process is shown in FIG. 5A.

The specific administration regimens are shown in Table 14. Mouse serum was separated weekly and assayed by ELISA for the anti-KLH specific IgG level.

**Table 14. Experimental administration regimens for mouse TDAR model**

| | Group | Administration | N | Dose | Frequency |
|---|---|---|---|---|---|
| hCD40 transgenic mouse | 1 | IgG1 | 5 | 1 mg/kg | BIW |
| | 2 | CFZ533 | 5 | 1 mg/kg | BIW |
| | 3 | CFZ533 | 5 | 0.3 mg/kg | BIW |
| | 4 | 9E6-L4H2 | 5 | 1 mg/kg | BIW |
| | 5 | 9E6-L4H2 | 5 | 0.3 mg/kg | BIW |
| | 6 | 2F12-L4H2 | 5 | 1 mg/kg | BIW |
| | 7 | 2F12-L4H2 | 5 | 0.3 mg/kg | BIW |

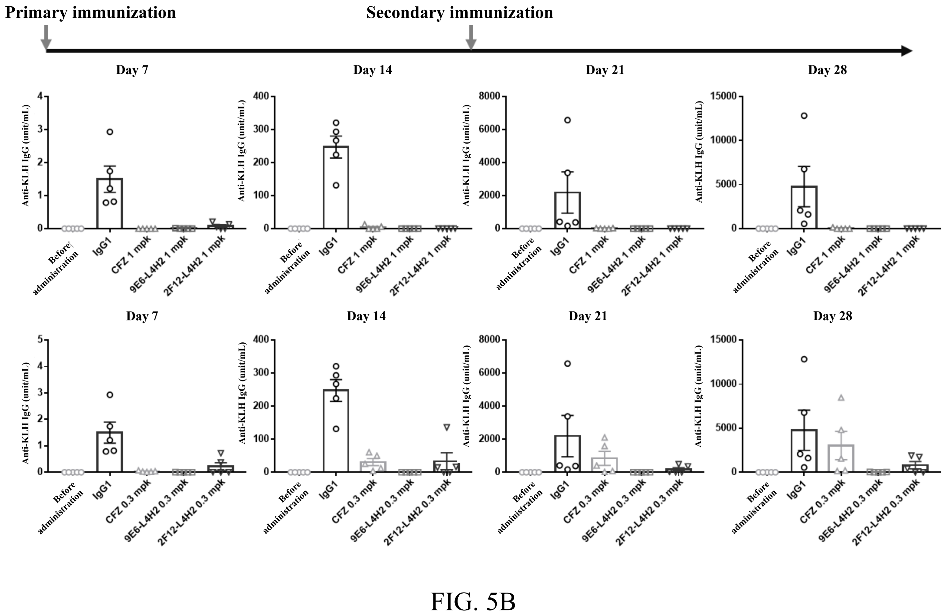

According to the results shown in FIG. 5B and Table 15, 1 mg/kg CD40 antagonistic antibodies significantly inhibited anti-KLH specific IgG production following two immunizations. The low doses (0.3 mg/kg) of 9E6-L4H2 and 2F12-L4H2 better inhibited anti-KLH IgG production than the same dose of CFZ533 following two immunizations. 2F12-L4H2 was another anti-CD40 antibody obtained by screening in the present application.

**Table 15. Inhibition of IgG production by anti-CD40 antibodies after immunization (0.3 mpk)**

| | Day 7 | Day 14 | Day 21 | Day 28 |
|---|---|---|---|---|
| Before administration | 0 | 0 | 0 | 0 |
| IgG1 | 1.5±0.4 | 247.4±33.1 | 2187±1248 | 4773±2280 |
| CFZ533 (0.3 mpk) | 0.04±0.01 | 30.9±10.8 | 845±423 | 3043±1600 |
| 9E6-L4H2 (0.3 mpk) | 0.002±0.001 | 0.02±0.01 | 0 | 18.25±8.9 |
| 2F12-L4H2 (0.3 mpk) | 0.22±0.14 | 33.4±25.8 | 175.5±103 | 805.7±415.6 |

### Example 13. Mouse Skin Graft Rejection Model

Male Balb/c mice, 6 weeks old, were purchased from the Laboratory Animal Management Department, Shanghai Institute of Planned Parenthood Research. Housing environment: SPF; certification number: 20180006023393.

Female human CD40 transgenic mice, 6-7 weeks old, were purchased from Biocytogen Jiangsu Co., Ltd. Housing environment: SPF; certification number: 320726200100179778.

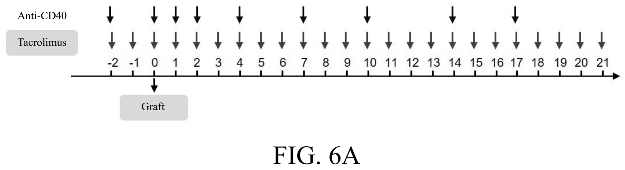

After arrival, the animals were acclimatized for 7 days and randomly grouped. On day -2 of the experiment, the mice were intraperitoneally injected with tacrolimus FK506 or CD40 antagonistic antibodies. On day 0 of the experiment, donor Balb/c mice and C57BL6/J mice were anesthetized with 4% chloral hydrate. The tails of the donor mice were removed, and full circles of skin, 1 cm in length, were isolated from the tails. The hair on the acceptor mice's back was removed, and an incision was made along the skin layer. An equal area of skin was removed while preserving the back fat and connective tissue. The donor skin was placed on the acceptor mice's incision site, and the incision was closed with glue. The mice were then placed back in cages to recover. The specific experimental process is shown in FIG. 6A.

The mice were dosed according to the administration regimens in Table 16. After 7 days of recovery, the mouse skin survival was observed daily and rejection scores were recorded. The scoring system is as follows: 3 - the skin does not turn red and is smooth; 2 - part of the skin turns red, loses its luster, and is dry; 1 - most of the skin turns red, does not have lines, and shrinks; 0 - graft rejection makes 80% of the skin necrotic.

**Table 16. Administration regimens for mouse skin graft rejection model**

| | Group | Administration | N | Dose | Route of administration |
|---|---|---|---|---|---|
| Donor: Balb/c | 1 | Control group (allograft) | 2 | IgG1 10 mg/kg | ip |
| | 2 | Model group (xenograft) | 7 | IgG1 10 mg/kg | ip |
| | 3 | CFZ533 | 7 | 3 mg/kg | ip |
| | 4 | CFZ533 | 7 | 10 mg/kg | ip |
| | 5 | 9E6-L4H2 | 7 | 3 mg/kg | ip |
| Acceptor: hCD40 transgenic mouse C57BL6/J | 6 | 9E6-L4H2 | 7 | 10 mg/kg | ip |
| | 7 | 2F12-L4H2 | 7 | 3 mg/kg | ip |
| | 8 | 2F12-L4H2 | 7 | 10 mg/kg | ip |
| | 9 | Tacrolimus | 6 | 3 mg/kg | ip |
| | 10 | 9E6-L4H2 + tacrolimus | 6 | 10 mg/kg + 3 mg/kg | ip |
| | 11 | 2F12-L4H2 + tacrolimus | 6 | 10 mg/kg + 3 mg/kg | ip |

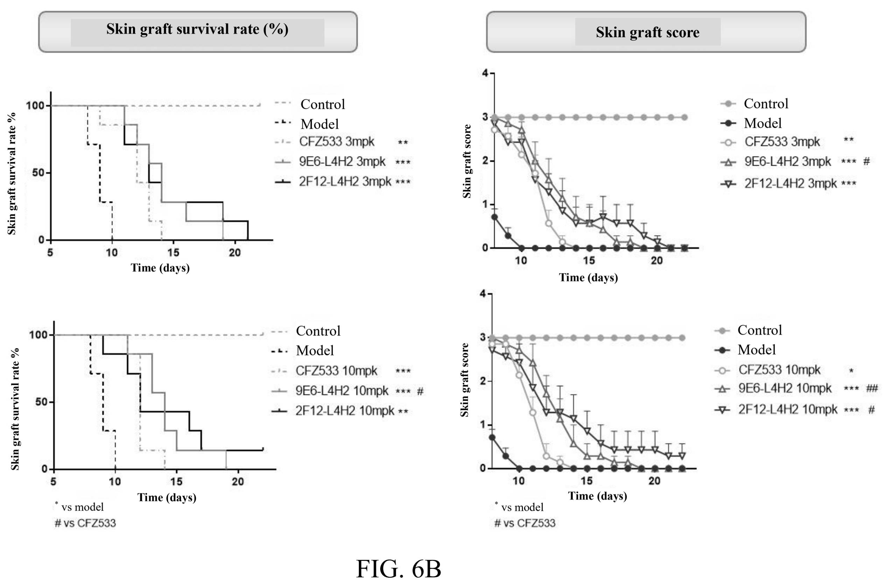

According to the results shown in FIG. 6B and Tables 17-19, both the CD40 antagonistic antibodies significantly improved mouse skin graft scores and prolonged skin graft survival time compared to the model group. Since it could not be determined before if there was rejection, skin graft scoring started from day 8. 9E6-L4H2 and 2F12-L4H2 showed better anti-graft rejection activity than the control antibody CFZ533. 2F12-L4H2 was another anti-CD40 antibody obtained by screening in the present application.

**Table 17. Day-15 skin graft survival rates (%) for anti-CD40 antibodies**

| | Skin graft survival rate (%) | | Skin graft survival rate (%) |
|---|---|---|---|
| Control | 100% | Control | 100% |
| Model | 0% | Model | 0% |
| CFZ533(3 mpk) | 0% | CFZ533(10 mpk) | 0% |
| 9E6-L4H2(3 mpk) | 28.5% | 9E6-L4H2(10 mpk) | 14.3% |
| 2F12-L4H2(3 mpk) | 28.5% | 2F12-L4H2(10 mpk) | 42.9% |

**Table 18. Skin graft scores for anti-CD40 antibodies (3 mpk)**

| | 8 days | 10 days | 15 days | 20 days |
|---|---|---|---|---|
| Control | 3 | 3 | 3 | 3 |
| Model | 0.7 | 0 | 0 | 0 |
| CFZ533(3 mpk) | 2.7 | 2.1 | 0 | 0 |
| 9E6-L4H2(3 mpk) | 3 | 2.7 | 0.6 | 0 |
| 2F12-L4H2(3 mpk) | 2.9 | 2.4 | 0.6 | 0.1 |

**Table 19. Skin graft scores for anti-CD40 antibodies (10 mpk)**

| | 8 days | 10 days | 15 days | 20 days |
|---|---|---|---|---|
| Control | 3 | 3 | 3 | 3 |
| Model | 0.7 | 0 | 0 | 0 |
| CFZ533(10 mpk) | 2.9 | 2.1 | 0 | 0 |
| 9E6-L4H2(10 mpk) | 3 | 2.7 | 0.3 | 0 |
| 2F12-L4H2(10 mpk) | 2.7 | 2.4 | 0.9 | 0.4 |

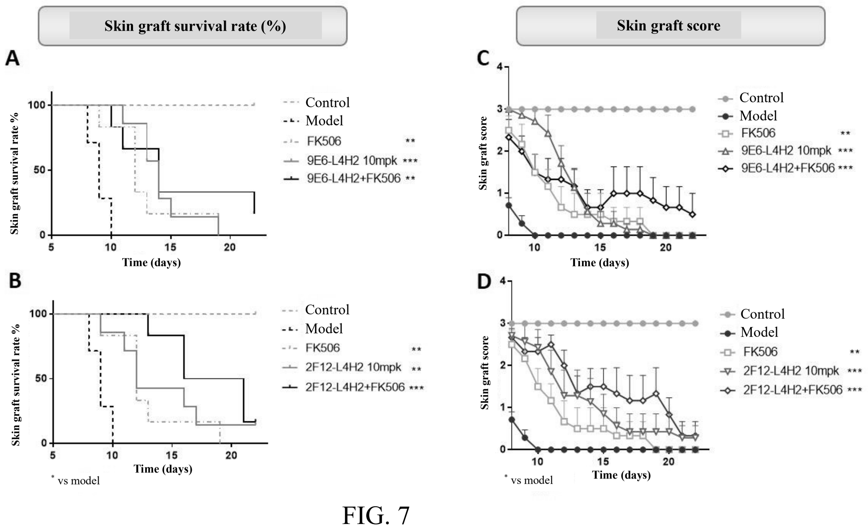

In addition, as shown in FIG. 7 and Tables 20-22, 9E6-L4H2 and 2F12-L4H2 further enhanced the resistance to mouse skin graft rejection after use in combination with tacrolimus.

**Table 20. Day-15 skin graft survival rates for anti-CD40 antibodies in combination with tacrolimus**

| | Skin graft survival rate (%) | | Skin graft survival rate (%) |
|---|---|---|---|
| Control | 100% | Control | 100% |
| Model | 0% | Model | 0% |
| FK506(3 mpk) | 16.7% | FK506(3 mpk) | 16.7% |
| 9E6-L4H2(10 mpk) | 14.3% | 2F12-L4H2(10 mpk) | 42.9% |
| 9E6-L4H2(10 mpk) +FK506 | 33.3% | 2F12-L4H2(10 mpk) +FK506 | 83.3% |

**Table 21. Skin graft scores for anti-CD40 antibodies in combination with tacrolimus**

| | 8 days | 10 days | 15 days | 20 days |
|---|---|---|---|---|
| Control | 3 | 3 | 3 | 3 |
| Model | 0.7 | 0 | 0 | 0 |
| FK506(3 mpk) | 2.5 | 1.5 | 0.5 | 0 |
| 9E6-L4H2(10 mpk) | 3 | 2.7 | 0.3 | 0 |
| 9E6-L4H2(10 mpk)+FK506(3 mpk) | 2.3 | 1.5 | 0.7 | 0.7 |

**Table 22. Skin graft scores for anti-CD40 antibodies in combination with tacrolimus**

| | 8 days | 10 days | 15 days | 20 days |
|---|---|---|---|---|
| Control | 3 | 3 | 3 | 3 |
| Model | 0.7 | 0 | 0 | 0 |
| FK506(3 mpk) | 2.5 | 1.5 | 0.5 | 0 |
| 2F12-L4H2(10 mpk) | 2.7 | 2.4 | 0.9 | 0.4 |
| 2f12-L4H2(10 mpk)+FK506(3 mpk) | 2.7 | 2.3 | 1.5 | 0.8 |

### Example 14. PK Assays of Humanized Anti-CD40 Antibodies in Human CD40 Transgenic Mice

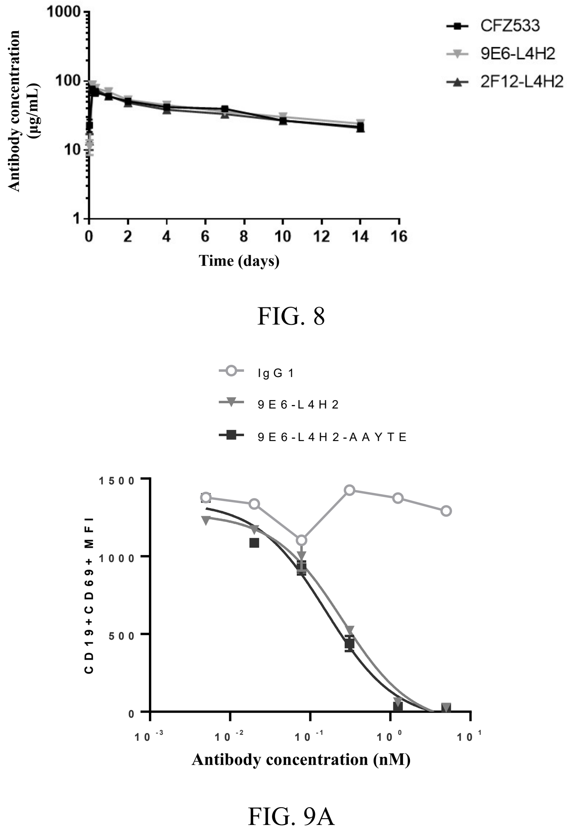

Female human CD40 transgenic mice, 6-7 weeks old, were purchased from Biocytogen Jiangsu Co., Ltd. Housing environment: SPF; production license: SCXK(Jiangsu)-2016-0004; human CD40 transgenic mouse certification number: 320726200100154632. After arrival, the animals were acclimatized for 7 days and randomly grouped. On day 0 of the experiment, each group of mice was intraperitoneally injected with the anti-CD40 antibody at 10 mg/kg. 100-150 µL of blood samples were collected at 15 min, 4 h, and 8 h after administration, and on days 1, 2, 4, 7, 10, and 14 after administration, anticoagulated with 10 µL of EDTA-K2 (0.1 M), and stored on ice. Mouse plasma was assayed by ELISA for antibody concentration at different time points. The assay is specifically as follows: A goat anti-human IgG Fc antibody (Rockland, Cat. # 609-101-017) was diluted to 2.5 µg/mL with PBS and added to a 96-well plate at 50 µL/well, and the plate was incubated overnight at 4 °C. After three washes with a washing buffer, 50 µL of blocking solution was added to each well, and the plate was incubated at 37 °C for 1 h. The mouse plasma and the standard curve of the test antibody were added, and the plate was incubated at 37 °C for 2 h. After three washes with the washing buffer, anti-hlgG Fab-HRP (Sigma, Cat. # A0293, 1:10,000) was added at 50 µL/well, and the plate was incubated at room temperature for 1 h. The plate was washed three times with the washing buffer. TMB was added at 100 µL/well, and the mixtures were left to react in the dark for 5 min. 0.16 M sulfuric acid was added at 100 µL/well. The OD value at 450 nm was measured with an Envision microplate reader, and the concentrations of CD40 antagonistic antibodies were calculated. According to the results shown in Table 23 and FIG. 8, 9E6-L4H2, 2F12-L4H2, and the control molecule CFZ533 have similar PK characteristics in human CD40 transgenic mice. 2F12-L4H2 was another anti-CD40 antibody obtained by screening in the present application.

**Table 23. PK data for CD40 antagonistic antibodies in hCD40 transgenic mice**

| | CFZ533 | 9E6-L4H2 | 2F12-L4H2 |
|---|---|---|---|
| T1/2 (h) | 214.8 | 226.8 | 257.1 |
| C max (mg/L) | 75.95 | 85.8 | 88.78 |
| AUC_{(0-d14)}((h)*(mg/L)) | 12655 | 13439 | 12014 |

### Example 15. Assays for Affinities of Humanized Anti-CD40 Antibodies for Human FcRn

The test antibody was affinity-captured on an anti-human Fab chip, and a series of concentrations of human FcRn antigen (purchased from AcroBiosystem) were allowed to flow over the chip surface. Reaction signals where the reactions were in a steady state were detected in real time using a Biacore instrument. The buffer used in the experiment was HBS-EP + 10× buffer solution (Cat. # BR-1006-69, GE) diluted to 1× (pH 7.4) with D. I. Water. Fitting was performed on the data obtained from the experiment using a steady state binding model to obtain affinity values. See Table 24. The anti-CD40 antibodies carrying the AAYTE mutation have a higher binding affinity for human FcRn than their parent antibodies.

**Table 24. Assays for affinities of humanized anti-CD40 antibodies for human FcRn**

| Mobile phase | Stationary phase | KD (M) |
|---|---|---|
| Human FcRn | 9E6-L4H2 | 2.93E-07 |
| | 9E6-L4H2-AAYTE | 3.98E-08 |

### Example 16. Inhibitory Activity of Anti-CD40 Antibody Carrying AAYTE Mutation in Fc in B Cell Activation Assay System

With reference to the method in Example 10, the inhibitory activity of the anti-CD40 antibody 9E6-L4H2-AAYTE carrying the AAYTE mutation in the Fc in the B cell activation assay system was measured. According to the results shown in FIG. 9A, the Fc-mutation anti-CD40 antibody has similar B cell inhibitory activity to its parent anti-CD40 antibody.

### Example 17. Inhibitory Activity of Anti-CD40 Antibody Carrying AAYTE Mutation in Fc in DC Cell Activation Assay System

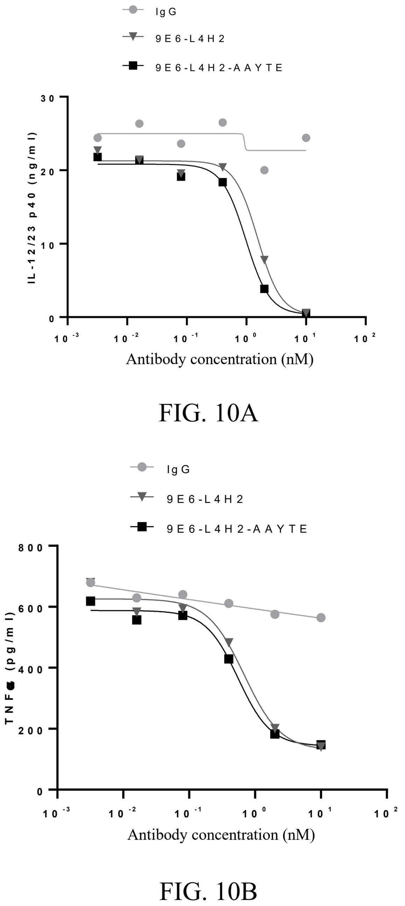

With reference to the method in Example 11, the inhibitory activity of the anti-CD40 antibody 9E6-L4H2-AAYTE carrying the AAYTE mutation in the Fc in the DC cell activation assay system was measured. According to the results shown in FIGs. 10A to 10B, the Fc-mutation anti-CD40 antibody has similar DC cell inhibitory activity to its parent anti-CD40 antibody.

### Example 18. Preparation of Antibody-Drug Conjugate ADC-2 (9E6-L4H2-Compound 2)

### 1. Preparation of tert-butyl (((9H-fluoren-9-yl)methoxy)carbonyl)glycylglycyl-L-phenylalaninate (compound 1b)

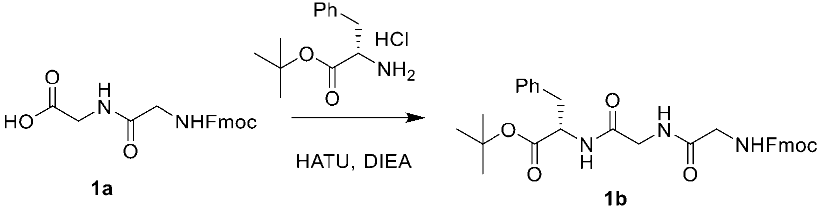

The starting materials **1a** (5.14 g, 20.0 mmol, 1.0 eq) and tert-butyl L-phenylalaninate hydrochloride (7.08 g, 20.0 mmol, 1.0 eq) were placed in a 250 mL three-necked flask and completely dissolved in anhydrous DMF (60 mL). The solution was cooled in an ice bath, and HATU (9.12 g, 24.0 mmol, 1.2 eq) and DIEA (7.74 g, 60.0 mmol, 3.0 eq) were then added. The mixture was left to react in the ice bath for 2 h. Water was added to the reaction mixture, and extraction was performed with ethyl acetate. The organic phase was washed with saturated brine, dried, and concentrated under reduced pressure to give white solid product **1b** (11.5 g, 100% yield). The crude product was directly used in the next step.

MS (ESI): m/z 580.3 [M+Na]⁺.

¹H NMR (400 MHz, DMSO-d6) δ 8.21-8.16(m, 1H), 8.12-8.05 (m, 1H), 7.91-7.87 (m, 2H), 7.72-7.68 (m, 2H), 7.64-7.59 (m, 1H), 7.44-7.38 (m, 2H), 7.36-7.25 (m, 4H), 7.23-7.18 (m, 3H), 4.41-4.18 (m, 4H), 3.76-3.72 (m, 2H), 3.68-3.61 (m, 2H), 2.98-2.89 (m, 2H), 2.69 (s, 2H), 1.30 (s, 9H).

### 2. Preparation of tert-butyl glycylglycyl-L-phenylalaninate (compound 1c)

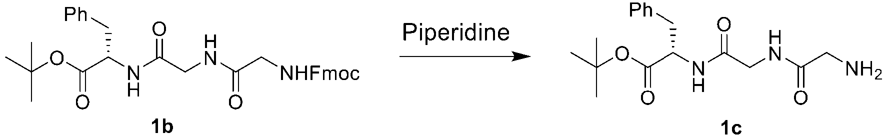

The starting material **1b** (5.57 g, 10.0 mmol, 1.0 eq) was placed in a 250 mL three-necked flask and completely dissolved in anhydrous DCM (60 mL). The solution was cooled in an ice bath. Piperidine (8.5 g, 100.0 mmol, 10.0 eq) was slowly added. After the addition, the mixture was stirred at room temperature for about 2 h. The reaction mixture was directly concentrated to dryness, and the crude product was purified by column chromatography to give product **1c** (2.70 g, 81% yield) as a pale yellow slurry. MS (ESI): m/z 358.3[M+H]⁺.

### 3. Preparation of tert-butyl (1-(9H-fluoren-9-yl)-3-oxo-2,7,10,13,16-pentaoxa-4-azanonadecan-19-oyl)glycylglycyl-L-phenylalaninate (compound 1d)

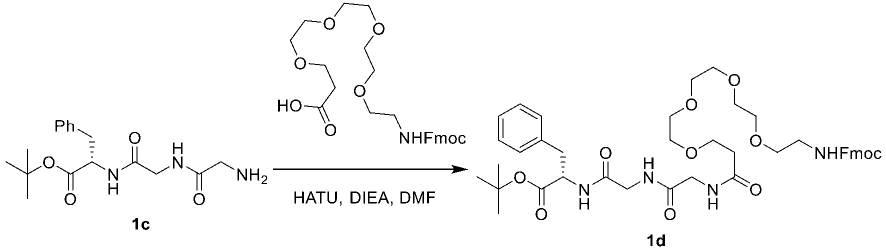

The starting materials 1-(9H-fluoren-9-yl)-3-oxo-2,7,10,13,16-pentaoxa-4-azanonadecan-19-oic acid (1.0 g, 2.05 mmol, 1.0 eq; from Tonglai Biochemical, Batch No.: P110114) and **1c** (0.69 g, 2.05 mmol, 1.0 eq) were added to a 100 mL three-necked flask and completely dissolved in anhydrous DMF (25 mL). The solution was cooled in an ice bath. HATU (1.01 g, 2.67 mmol, 1.3 eq) and DIEA (529 mg, 4.10 mmol, 2.0 eq) were added, and the mixture was left to react in the ice bath for 1 h. Water was added to the reaction mixture, and extraction was performed with ethyl acetate. The organic phase was washed with saturated brine, dried, and concentrated under reduced pressure to give **1d** (1.60 g, 97% yield) as a pale yellow oil.

MS (ESI): m/z 805.3 [M+H]⁺.

### 4. Preparation of (1-(9H-fluoren-9-yl)-3-oxo-2,7,10,13,16-pentaoxa-4-azanonadecan-19-oyl)glycylglycyl-L-phenylalanine (compound 1e)

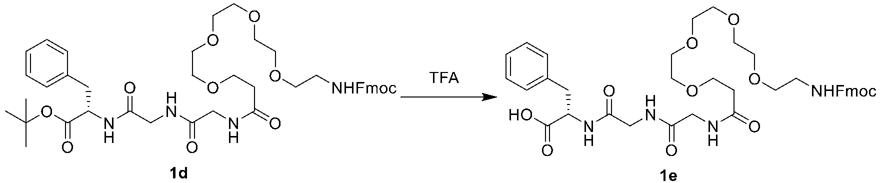

The starting material 1d (1.60 g, 1.99 mmol, 1.0 eq) was added to a 100 mL single-necked flask and dissolved in anhydrous dichloromethane (16 mL). Trifluoroacetic acid (8 mL) was added at room temperature, and the mixture was stirred for 2 h. The reaction mixture was directly concentrated to dryness to give a yellow slurry, and ethyl acetate was added to dissolve the slurry. The solution was washed with saturated brine, dried, and concentrated under reduced pressure to give **1e** (1.23 g, 83% yield) as a yellow oil;

MS (ESI): m/z 749.3 [M+H]⁺.

### 5. Preparation of (9H-fluoren-9-yl)methyl (2-(((2-((6aR,6bS,7S,8aS,8bS,11aR,12aS,12bS)-7-hydroxy-6a,8a-dimethyl-4-oxo-10-propyl-1,2,4,6a,6b,7,8,8a,11a,12,12a,12b-dodecahydro-8bH-naphtho[2',1':4,5]indeno[1,2-d][1,3]dioxol-8b-yl)-2-oxoethoxy)methyl)amino)-2-oxoethyl)carbamate (compound 1f)

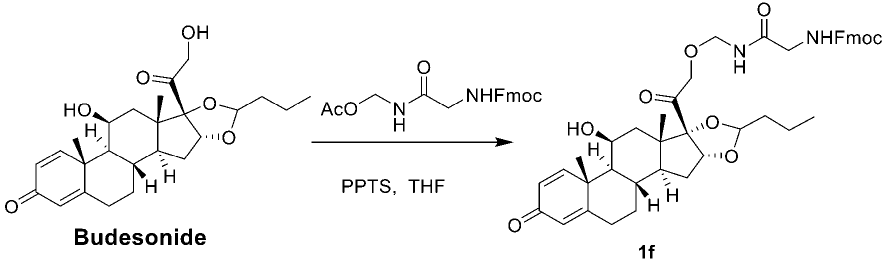

The starting materials budesonide (1.29 g, 3.0 mmol, 1.0 eq) and (2-((((9H-fluoren-9-yl)methoxy)carbonyl)amino)acetamido)methyl acetate (1.16 g, 3.15 mmol, 1.05 eq; prepared using the method in the document *"*Tetrahedron, 2018, 74(15), 1951-1956") and pyridinium p-toluenesulfonate (75 mg, 0.30 mmol, 0.1 eq) were added to a 100 mL three-necked flask. Anhydrous tetrahydrofuran (20 mL) was added at room temperature, and the mixture was heated at reflux for 4 h. The reaction mixture was directly concentrated to dryness, and the crude product was purified by column chromatography to give product **1f** (0.54 g, 24% yield) as a white solid.

MS (ESI): m/z 739.4 [M+H]⁺.

¹H NMR (400 MHz, DMSO-d6) δ 8.76-8.69 (m, 1H), 7.91-7.84 (m, 2H), 7.73-7.67 (m, 2H), 7.63-7.57 (m, 1H), 7.43-7.58 (m, 2H), 7.35-7.23 (m, 3H), 6.13 (d, *J* = 7.5 Hz, 1H), 5.91 (s, 2H), 5.15-4.97 (m, 1H), 4.73-4.45 (m, 5H), 4.30-4.09 (m, 5H), 3.65-3.59 (m, 2H), 2.29-2.21 (m, 1H), 2.11-1.87 (m, 2H), 1.74-1.21 (m, 11H), 1.09-0.77 (m, 8H).

### 6. Preparation of 2-amino-N-((2-((6aR,6bS,7S,8aS,8bS,11aR,12aS,12bS)-7-hydroxy-6a,8a-dimethyl-4-oxo-10-propyl-1,2,4,6a,6b,7,8,8a,11a,12,12a,12b-dodecahydro-8bH-naphtho[2',1':4,5]indeno[1,2-d][1,3]dioxol-8b-yl)-2-oxoethoxy)methyl)acetamide (compound 1g)

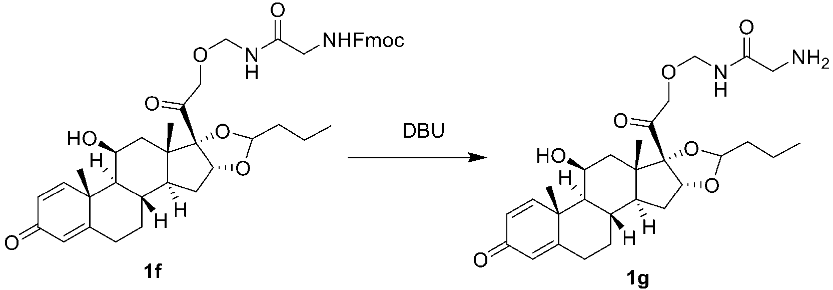

The starting material **1f** (540 mg, 0.73 mmol, 1.0 eq) was placed in a 25 mL single-necked flask and dissolved in anhydrous dichloromethane (10 mL). The solution was cooled in an ice bath, and DBU (167 mg, 1.10 mmol, 1.5 eq) was added. The mixture was stirred for 30 min. Water was added, liquid separation was performed, and the aqueous phase was extracted twice with dichloromethane. The organic phase was washed with saturated brine and concentrated under reduced pressure to give **1g** (450 mg of crude product containing fluorene, 100% yield) as a pale yellow semi-solid. The crude product was directly used in the next step.

MS (ESI): m/z 517.4 [M+H]⁺.

### 7. Preparation of (9H-fluoren-9-yl)methyl ((10S)-10-benzyl-1-((6aR,6bS,7S,8aS,8bS,11aR,12aS, 12bS)-7-hydroxy-6a,8a-dimethyl-4-oxo-10-propyl-1,2,4,6a,6b,7,8,8a,11a,12,12a,12b-dodecahydro-8bH-naphtho[2',1':4,5]indeno[1,2-d][1,3]dioxol-8b-yl)-1,6,9,12,15,18-hexaoxo-3,21,24,27,30-pentaoxa-5,8,11,14,17-pentaazadotriacontan-32-yl)carbamate (compound 1h)

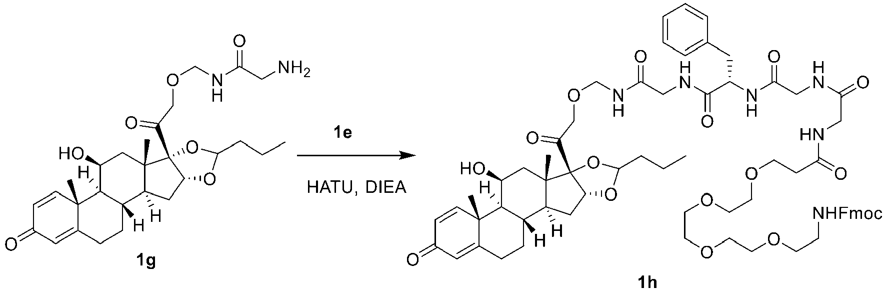

The starting materials **1g** (0.45 g of crude product, equivalent to 0.73 mmol, 1.0 eq) and 1e (0.55 g, 0.73 mmol, 1.0 eq) were placed in a 100 mL three-necked flask and completely dissolved in anhydrous DMF (9 mL). The solution was cooled in an ice bath, and HATU (361 mg, 0.95 mmol, 1.3 eq) and DIEA (189 mg, 1.46 mmol, 2.0 eq) were then added sequentially. The mixture was stirred for 1 h. Water was added to the reaction mixture, and extraction was performed twice with dichloromethane. The organic phases were combined, washed once with saturated brine, dried, and concentrated under reduced pressure to give a yellow oil-solid crude product. The solid crude product was purified by slurrying in a mixed solvent of petroleum ether and ethyl acetate to give product **1h** (710 mg, 78% yield) as a pale yellow solid.

MS (ESI): m/z 1269.7 [M+Na]⁺.

### 8. Preparation of 1-amino-N-((10S)-10-benzyl-1-((6aR,6bS,7S,8aS,8bS,11aR,12aS,12bS)-7-hydroxy-6a,8a-dimethyl-4-oxo-10-propyl-1,2,4,6a,6b,7,8,8a,11a,12,12a,12b-dodecahydro-8bH-naphtho[2',1':4,5]indeno[1,2-d][1,3]dioxol-8b-yl)-1,6,9,12,15-pentaoxo-3-oxa-5,8,11,14-tetraazahexadecan-16-yl)-3,6,9,12-tetraoxapentadecan-15-amide (compound 1i)

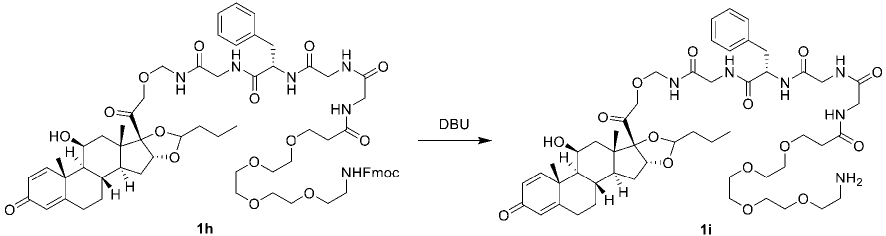

The starting material 1h (350 mg, 0.28 mmol, 1.0 eq) was placed in a 25 mL single-necked flask and completely dissolved in anhydrous dichloromethane (10 mL). The solution was cooled in an ice bath, and DBU (64 mg, 0.42 mmol, 1.5 eq) was added. The mixture was stirred for 1 h. The reaction mixture was directly concentrated to dryness to give an oily crude product, and the crude product was purified by column chromatography to give product **1i** (289 mg, 89% yield) as a pale yellow foamy solid. MS (ESI): m/z 1025.6 [M+H]⁺.

### 9. Preparation of N-((10S)-10-benzyl-1-((6aR,6bS,7S,8aS,8bS,11aR,12aS,12bS)-7-hydroxy-6a,8a-dimethyl-4-oxo-10-propyl-1,2,4,6a,6b,7,8,8a,11a,12,12a,12b-dodecahydro-8bH-naphtho[2',1':4,5]indeno[1,2-d][1,3]dioxol-8b-yl)-1,6,9,12,15-pentaoxo-3-oxa-5,8,11,14-tetraazahexadecan-16-yl)-1-(2-bromoacetamido)-3,6,9,12-tetraoxapentadecan-15-amide (compound 2)

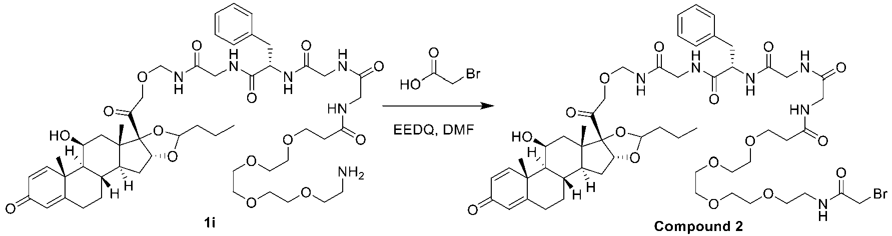

The solid bromoacetic acid (35.2 mg, 0.25 mmol, 2.0 eq) and EEDQ (63 mg, 0.25 mmol, 2.0 eq) were placed in a 25 mL Schlenk-Tube and completely dissolved in anhydrous DMF (5 mL). The solution was stirred at room temperature for 1 h. The starting material 1i (130 mg, 0.127 mmol, 1.0 eq) was dissolved in DMF (2 mL), and the resulting solution was added dropwise to the active ester solution prepared above. After the addition, the mixture was left to react at room temperature for 2 h.

Water was added to the reaction system, and extraction was performed twice with ethyl acetate. The organic phases were combined, washed with saturated brine, dried, and concentrated under reduced pressure to give an oily crude product, and the crude product was then purified by column chromatography to give **compound 2** (39 mg, 27% yield) as a pale yellow solid.

MS (ESI): m/z 1145.5/1147.6 [M+1]⁺.

¹H NMR (400 MHz, CDCl₃) δ 8.62-8.57 (m, 1H), 8.34-8.28 (m, 2H), 8.19-8.11(m, 2H), 8.03-7.99(m, 1H), 7.30-7.17(m, 6H), 6.15 (d, *J* = 9.6 Hz, 1H), 5.92 (s, 2H), 5.18-5.03 (m, 1H), 4.72-4.47 (m, 5H), 4.29-4.15 (m, 2H), 3.78-3.42 (m, 27H), 3.27-3.21 (m, 2H), 3.06-2.84 (m, 2H), 2.41-2.28 (m, 3H), 2.06-1.73 (m, 2H), 1.60-1.24 (m, 10H), 1.01-0.82 (m, 8H).

### 10. Preparation of ADC-2 (9E6-L4H2-compound 2)

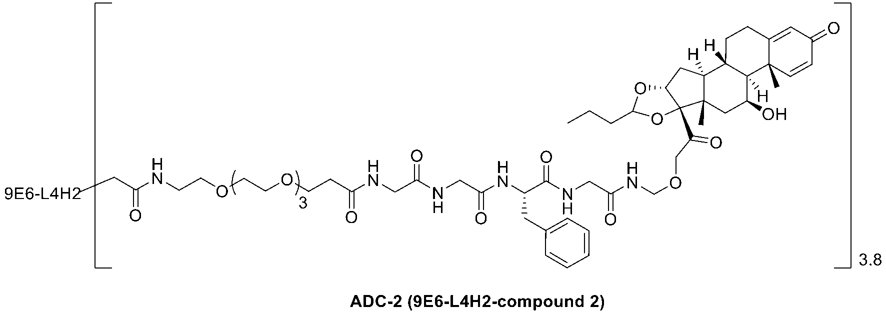

A prepared aqueous solution of tris(2-carboxyethyl)phosphine hydrochloride (TCEP.HCl) (2.5 mM, 170.3 µL, 0.42 mmol) was added to an aqueous buffer solution of antibody 9E6-L4H2 in buffer A (a pH 6.3, 0.05 M aqueous buffer solution; 15.0 mg/mL, 1.5 mL, 0.10 mmol) at 37 °C. The mixture was left to react on a water-bath shaker at 37 °C for 3 h, and then the reaction was stopped. The reaction mixture was cooled to 25 °C in a water bath. 1.0 M Tris buffer at a pH of 8.30 (210 µL) was added. Compound 2 (1.16 mg, 1.0 mmol) was dissolved in 75 µL of DMSO, and the solution was added to the above reaction mixture. The mixture was left to react on a water-bath shaker at 25 °C for 3 h, and then the reaction was stopped. The reaction mixture was desalted and purified using a Sephadex G25 gel column (eluent phase: buffer A) and concentrated using an ultrafiltration tube to give the title product ADC-2 (9E6-L4H2-compound 2) in buffer A buffer (5.14 mg/mL, 2.48 mL, 85% yield). The product was refrigerated at 4 °C. The theoretical DAR value was 4.0, and the found DAR value was 3.8.

### Example 19. Assays for Affinities of Anti-CD40-ADC for Human CD40 and Cynomolgus Monkey CD40

The test antibody and anti-CD40-ADC were affinity-captured on anti-human Fc chips, and a series of concentrations of His-tagged human or cynomolgus monkey CD40 antigen were allowed to flow over the chip surface. Reaction signals were detected in real time using a Biacore instrument, and thus association and dissociation curves were obtained. The buffer used in the experiment was HBS-EP + 10× buffer solution (Cat. # BR-1006-69, GE) diluted to 1× (pH 7.4) with D. I. Water. Fitting was performed on the data obtained from the experiment using a (1:1) Binding model to obtain affinity values. See Table 25.

**Table 25. SPR affinities of anti-CD40-ADC for CD40 of different species**

| Mobile phase | Stationary phase | Ka (1/Ms) | Kd (1/s) | KD (M) |
|---|---|---|---|---|
| Human CD40 | 9E6-L4H2 | 4.07E+05 | 3.77E-04 | 9.26E-10 |
| | ADC-2 | 2.78E+05 | 2.16E-04 | 7.76E-10 |
| Cynomolgus monkey CD40 | 9E6-L4H2 | 4.68E+05 | 1.38E-04 | 2.94E-10 |
| | ADC-2 | 3.30E+05 | 2.40E-04 | 7.29E-10 |

### Example 20. Inhibitory Activity of Anti-CD40-ADC in B Cell Activation Assay System

CD40 is highly expressed in B cells, and CD40L, upon binding to CD40, can induce B cell activation and up-regulate the expression of a series of activation markers. CD40 antagonistic antibodies block the binding of CD40L to CD40, thereby eliminating the immune activation process of B cells.

Human PBMCs were plated at 2E5 cells/well and 50 µL/well into a 96-well cell culture plate (the medium was RPMI-1640 containing 10% FBS and 1% penicillin-streptomycin). The serially diluted test antibody or ADC was added at 50 µL/well, and the cells were co-incubated at 37 °C in 5% CO₂ for 0.5 h. The pre-incubated CD40L-his and anti-His antibody were added at 50 µL/well for overnight stimulation. The following day, the plate was centrifuged, the supernatant was removed, and the cells were washed twice with FACS buffer and stained at room temperature for 15 min with 100 µL of Fixable viability dye EF780 (Invitrogen, 65086514) diluted at 1:1000. The cells were washed twice and then blocked at room temperature for 10 min with 100 µL of human Fc blocker (BD, 564220) diluted at 1:200. After centrifugation, the cells were incubated at 4 °C for 0.5 h with 100 µL of flow cytometry antibodies (PerCP/Cyanine5.5 anti-human CD19 (Biolegend, 302230, diluted at 1:100), and APC anti-human CD69 (Biolegend, 310910, diluted at 1:200)). After centrifugation, the supernatant was removed, the cells were washed twice with FACS buffer and then resuspended in 200 µL of FACS buffer, and the fluorescence intensity on the cell surface was measured by a flow cytometer (BD FACS Celesta).

According to the results shown in Table 26, ADC-1 and ADC-2 have similar B cell inhibitory activity to the monoclonal antibody 9E6-L4H2.

**Table 26. Inhibitory activity of anti-CD40-ADC in B cell activation assay system**

| Drug No. | CD 19+CD69+% | | | |
|---|---|---|---|---|
| | Donor 1 | | Donor 2 | |
| | IC₅₀ (nM) | Maximum inhibition rate | IC₅₀ (nM) | Maximum inhibition rate |
| 9E6-L4H2 | 0.44 | 95% | 0.52 | 104% |
| ADC-2 | 0.63 | 90% | 0.48 | 98% |

After the antibody or ADC was added, the CD19+CD69+ signal was lower than the background, probably because the background activation of B cells was inhibited to some extent (in addition to the inhibition of the CD40L-induced signal).

### Example 21. Mouse Skin Graft Rejection Model

Male Balb/c mice, 6 weeks old, were purchased from the Laboratory Animal Management Department, Shanghai Institute of Planned Parenthood Research. Housing environment: SPF; production license SCXK (Shanghai) 2018-0006; Balb/c mouse certification number: 20180006023393.

Female human CD40 transgenic mice, 6-7 weeks old, were purchased from Biocytogen Jiangsu Co., Ltd. Housing environment: SPF; production license: SCXK(Jiangsu)-2016-0004; human CD40 transgenic mouse certification number: 320726200100179778.

After arrival, the animals were acclimatized for 7 days and randomly grouped. On day -2 of the experiment, the mice were intraperitoneally injected with the CD40 antagonistic antibody, anti-CD40-ADC, or glucocorticoid. On day 0 of the experiment, donor Balb/c mice and C57BL6/J mice were anesthetized with 4% chloral hydrate. The tails of the donor mice were removed, and full circles of skin, 1 cm in length, were isolated from the tails. The hair on the acceptor mice's back was removed, and an incision was made along the skin layer. An equal area of skin was removed while preserving the back fat and connective tissue. The donor skin was placed on the acceptor mice's incision site, and the incision was closed with glue. The mice were then placed back in cages to recover. The specific experimental process is shown in FIG. 13A.

The mice were dosed according to the administration regimens in Table 27. After 7 days of recovery, the mouse skin survival was observed daily and rejection scores were recorded. The scoring system is as follows: 3 - the skin does not turn red and is smooth; 2 - part of the skin turns red, loses its luster, and is dry; 1 - most of the skin turns red, does not have lines, and shrinks; 0 - graft rejection makes 80% of the skin necrotic.

**Table 27. Administration regimens for mouse skin graft rejection model**

| | Group | Administration | N | Dose | Route of administration |
|---|---|---|---|---|---|
| Donor: Balb/c | 1 | Control group (allograft) | 3 | IgG1 10 mg/kg | ip |
| | 2 | Model group (xenograft) | 7 | IgG1 10 mg/kg | ip |
| Acceptor: hCD40 transgenic mouse C57BL6/J | 3 | 9E6-L4H2 | 7 | 10 mg/kg | ip |
| | 4 | ADC-2 | 7 | 10 mg/kg | ip |
| | 5 | 9E6-L4H2 + budesonide | 7 | 10 mg/kg+0.12 mg/kg(QD) | ip |
| | 6 | Dexamethasone | 7 | 20 mg/kg(QD) | ip |

According to the results shown in Tables 28-29, ADC-2 has significantly better anti-graft rejection activity than the CD40 antagonist monoclonal antibody 9E6-L4H2 and the monoclonal antibody used in combination with glucocorticoid, and slightly better than high-dose dexamethasone.

**Table 28. Skin graft survival rates (%) for anti-CD40-ADC**

| | 10 days | 15 days | 20 days |
|---|---|---|---|
| Control | 100% | 100% | 100% |
| Model | 33% | 0% | 0% |
| 9E6-L4H2 (10 mpk) | 100% | 44.4% | 11% |
| ADC-1 (10 mpk) | 100% | 87.5% | 75% |
| ADC-2 (10 mpk) | 100% | 88.9% | 88.9% |
| 9E6-L4H2 + budesonide | 100% | 44.4% | 33.3% |
| Dexamethasone (20 mpk, QD) | 88.9% | 88.9% | 66.7% |

**Table 29. Skin graft scores for anti-CD40-ADC**

| | 10 days | 15 days | 20 days |
|---|---|---|---|
| Control | 3 | 3 | 3 |
| Model | 0.44 | 0 | 0 |
| 9E6-L4H2 (10 mpk) | 2.78 | 1 | 0.22 |
| ADC-1 (10 mpk) | 2.78 | 2.38 | 1.88 |
| ADC-2 (10 mpk) | 2.56 | 2 | 2 |
| 9E6-L4H2 + budesonide | 2.33 | 1.11 | 0.78 |
| Dexamethasone (20 mpk, QD) | 2.33 | 2 | 1.56 |

### Example 22. pH and Buffer Screening for Anti-CD40 Antibody Formulations

20 mM acetate, 20 mM citrate, 20 mM histidine salt, and 20 mM phosphate buffer systems, as well as pH values of 4.5, 5.0, 5.5, 6.0, 6.5, 6.6, 7.0, and 7.4 were selected to form 12 combinations of different pH values and buffers, and a total of 12 groups of formula antibody formulations with the concentration of the anti-CD40 antibody (antibody No. 9E6(L4H2), with heavy and light chains set forth in SEQ ID NOs: 20 and 21, respectively; the same applied hereinafter) being 20.0 mg/mL were

prepared. The melting temperature (Tm) and aggregation temperature (Tagg) of the antibody in the above formulations were measured. Meanwhile, by detecting the SEC purity, NRCE purity, icIEF purity, and the like in the formulations, 12 groups of formulas were investigated for sample stability under the conditions of storage at 2-8 °C for 2 weeks, storage at 25 °C for 1 week and 2 weeks, storage at 40 °C for 1 week and 2 weeks, and 5 freeze-thaw cycles (-35 °C/room temperature). The following are formulas 1)-12):
1) 20 mM acetic acid-sodium acetate, pH 4.5
2) 20 mM acetic acid-sodium acetate, pH 5.0
3) 20 mM acetic acid-sodium acetate, pH 5.5
4) 20 mM citric acid-sodium citrate, pH 5.0
5) 20 mM citric acid-sodium citrate, pH 5.5
6) 20 mM citric acid-sodium citrate, pH 6.0
7) 20 mM histidine-histidine hydrochloride, pH 5.5
8) 20 mM histidine-histidine hydrochloride, pH 6.0
9) 20 mM histidine-histidine hydrochloride, pH 6.5
10) 20 mM sodium dihydrogen phosphate-disodium hydrogen phosphate, pH 6.6
11) 20 mM sodium dihydrogen phosphate-disodium hydrogen phosphate, pH 7.0
12) 20 mM sodium dihydrogen phosphate-disodium hydrogen phosphate, pH 7.4

The anti-CD40 antibody was found to have relatively poor solubility in the buffer systems of formula 4 and formula 5 during the study, and thus the stability of these two groups of buffer systems was not investigated.

**Table 30. Results 1 of pH and buffer screening for anti-CD40 antibody formulations**

| Formula No. | T ml (°C) | Tagg266 (°C) |
|---|---|---|
| 1 | 53.39 | 75.17 |
| 2 | 56.27 | 79.79 |
| 3 | 60.02 | 80.24 |
| 6 | 59.30 | 79.06 |
| 7 | 56.87 | 81.57 |
| 8 | 60.33 | 81.18 |
| 9 | 62.89 | 81.01 |
| 10 | 61.58 | 80.24 |
| 11 | 61.70 | 77.77 |
| 12 | 61.60 | 77.90 |

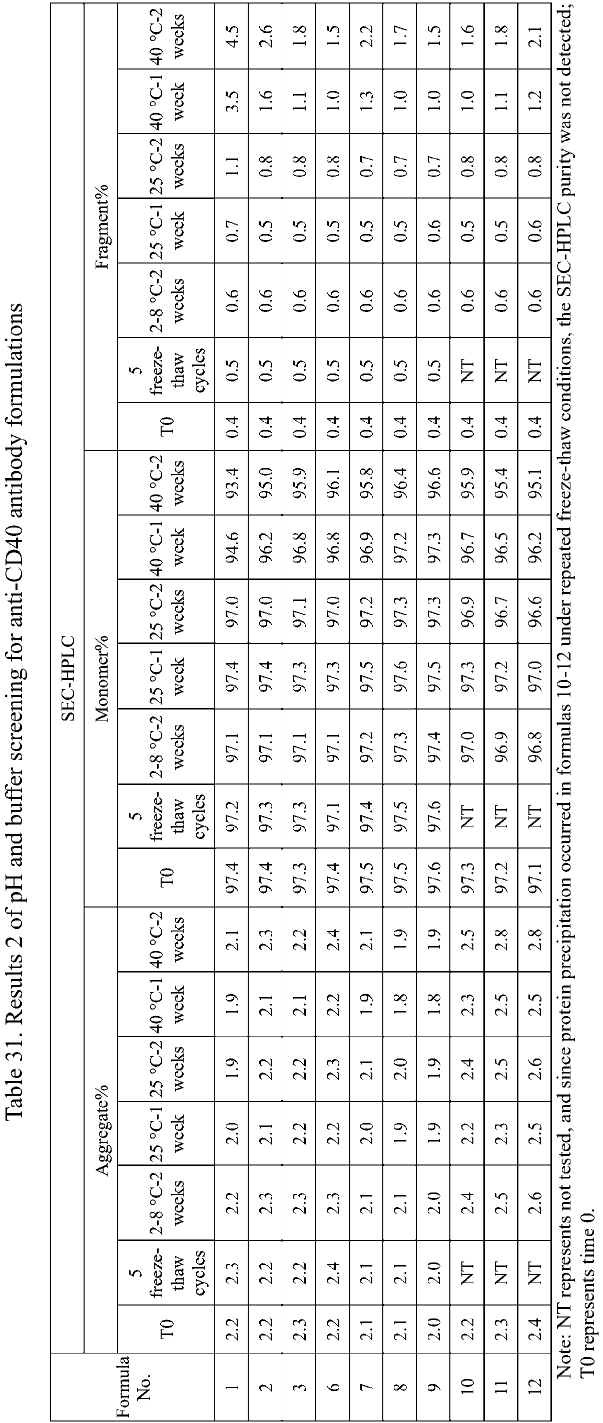

**Table 32. Results 3 of pH and buffer screening for anti-CD40 antibody formulations**

| Formula No. | NRCE | | | | | | | | | | | | | |
|---|---|---|---|---|---|---|---|---|---|---|---|---|---|---|
| | Fragment% | | | | | | | Main peak% | | | | | | |
| | T0 | 5 freeze-thaw cycles | 2-8 °C-2 weeks | 25 °C-1 week | 25 °C-2 weeks | 40 °C-1 week | 40 °C-2 weeks | T0 | 5 freeze-thaw cycles | 2-8 °C-2 weeks | 25 °C-1 week | 25 °C-2 weeks | 40 °C-1 week | 40 °C-2 weeks |
| 1 | 4.4 | 3.9 | 4.7 | 3.8 | 4.8 | 6.0 | 9.9 | 95.6 | 96.1 | 95.2 | 96.2 | 95.1 | 94.9 | 90.0 |
| 2 | 4.1 | 3.9 | 4.4 | 3.9 | 4.7 | 5.1 | 7.3 | 95.9 | 96.1 | 95.5 | 96.1 | 95.2 | 95.8 | 92.6 |
| 3 | 4.6 | 3.9 | 5.0 | 3.9 | 4.5 | 4.2 | 5.8 | 95.4 | 96.1 | 94.9 | 96.1 | 95.4 | 95.1 | 94.0 |
| 6 | 4.3 | 3.8 | 4.6 | 3.6 | 4.5 | 4.1 | 5.4 | 95.7 | 96.2 | 95.3 | 96.4 | 95.4 | 95.8 | 94.5 |
| 7 | 4.3 | 3.7 | 4.6 | 3.7 | 4.6 | 4.2 | 5.9 | 95.7 | 96.3 | 95.3 | 96.3 | 95.3 | 95.8 | 94.0 |
| 8 | 4.3 | 3.7 | 4.4 | 3.7 | 4.6 | 4.2 | 5.5 | 95.7 | 96.3 | 95.5 | 96.4 | 95.3 | 95.9 | 94.4 |
| 9 | 4.4 | 3.6 | 4.5 | 3.7 | 4.4 | 4.1 | 5.7 | 95.6 | 96.4 | 95.3 | 96.3 | 95.5 | 95.3 | 94.2 |
| 10 | 4.6 | NT | 4.2 | 3.8 | 4.5 | 4.7 | 6.3 | 95.4 | NT | 95.7 | 96.2 | 95.3 | 95.0 | 93.4 |
| 11 | 4.4 | NT | 4.3 | 3.9 | 4.6 | 5.0 | 7.3 | 95.6 | NT | 95.6 | 96.1 | 95.2 | 94.2 | 92.5 |
| 12 | 4.5 | NT | 4.3 | 3.8 | 4.7 | 5.8 | 8.8 | 95.5 | NT | 95.5 | 96.2 | 95.1 | 94.9 | 90.9 |

| | | | | | | | | | | | | | | |
|---|---|---|---|---|---|---|---|---|---|---|---|---|---|---|
| Note: NT represents not tested, and since protein precipitation occurred in formulas 10-12 under repeated freeze-thaw conditions, the NRCE purity was not detected; T0 represents time 0. | | | | | | | | | | | | | | |

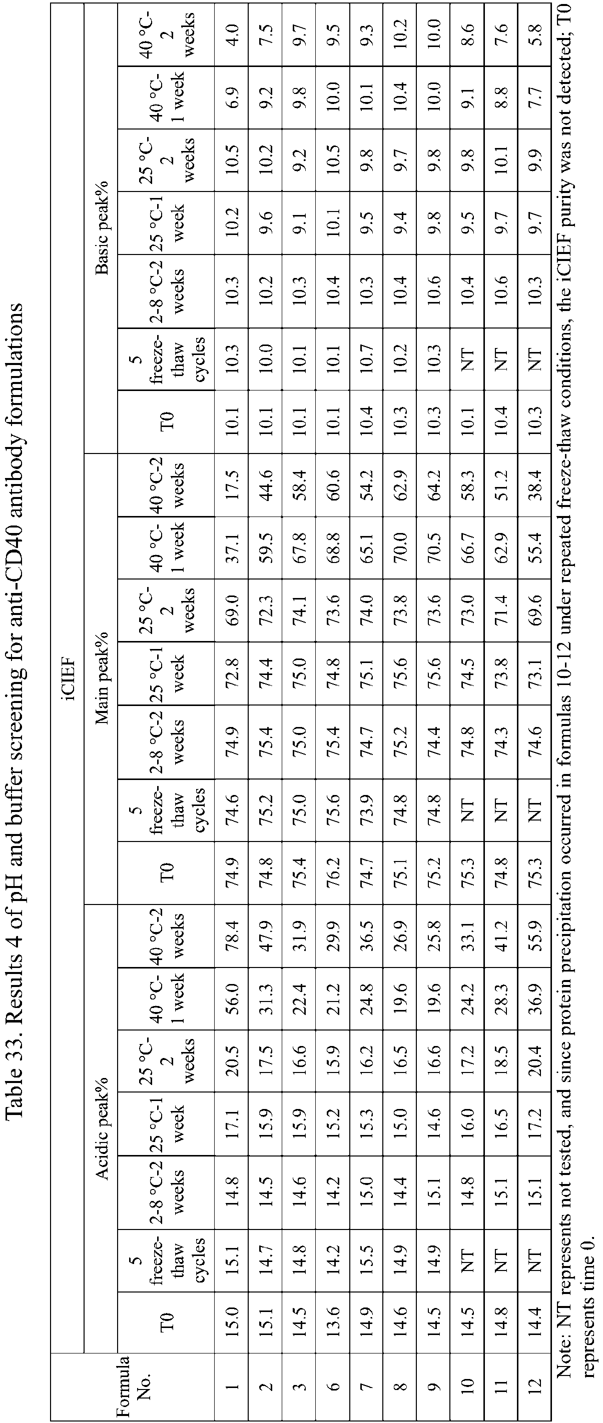

The results show that: the Tm values and Tagg values of the anti-CD40 antibody in the histidine salt buffer systems (formula 7-formula 9) were relatively high, which indicates that the antibody has relatively good conformation stability and colloidal stability in the histidine buffer system; after investigation for 2 weeks at the high temperature (40 °C), the SEC-HPLC purity, NRCE purity, and icIEF purity of the anti-CD40 antibody in histidine pH 6.0 and pH 6.5 systems were higher than those of the other systems, which indicates that the anti-CD40 antibody has better stability in histidine pH 6.0 and histidine pH 6.5 systems. Therefore, histidine systems in the pH range of pH 6.0-pH 6.6 were selected for the subsequent study.

### Example 23. Screening for Anti-CD40 Antibody Concentration, and Type and Concentration of Excipients

Two groups of buffer systems of 20 mM histidine-histidine hydrochloride at pH 6.0 and pH 6.6 were selected. 8% (w/v) sucrose and 5% (w/v) sucrose + 50 mM arginine hydrochloride were used as stabilizers, and 0.02% (w/v) polysorbate 80 was used as a surfactant. The concentrations of the anti-CD40 antibody were 50 mg/mL and 80 mg/mL. 6 groups of formulas were prepared. By detecting the appearance, pH, SEC-HPLC, NRCE, iCIEF purity, and the like of samples, the following 6 groups of formulas were investigated for stability under the conditions of storage at a high temperature (40 °C) for 2 weeks and 4 weeks, storage under the light exposure (5000±500 lx, 25 °C) for 5 days and 10 days, shaking (300 rpm/room temperature) for 2 days, repeated freeze-thaw cycles (-35 °C and room temperature) for 3 times and 5 times, and the like.
13) 20 mM histidine-histidine hydrochloride, pH 6.0, 8% (w/v) sucrose, 0.02% (w/v) polysorbate 80, 50 mg/mL anti-CD40 antibody
14) 20 mM histidine-histidine hydrochloride, pH 6.0, 5% (w/v) sucrose, 50 mM arginine hydrochloride, 0.02% (w/v) polysorbate 80, 50 mg/mL anti-CD40 antibody
15) 20 mM histidine-histidine hydrochloride, pH 6.0, 8% (w/v) sucrose, 0.02% (w/v) polysorbate 80, 80 mg/mL anti-CD40 antibody
16) 20 mM histidine-histidine hydrochloride, pH 6.6, 8% (w/v) sucrose, 0.02% (w/v) polysorbate 80, 50 mg/mL anti-CD40 antibody
17) 20 mM histidine-histidine hydrochloride, pH 6.6, 5% (w/v) sucrose, 50 mM arginine hydrochloride, 0.02% (w/v) polysorbate 80, 50 mg/mL anti-CD40 antibody
18) 20 mM histidine-histidine hydrochloride, pH 6.6, 8% (w/v) sucrose, 0.02% (w/v) polysorbate 80, 80 mg/mL anti-CD40 antibody

**Table 34. Results 1 of screening for protein concentration of anti-CD40 antibody and excipients**

| No. | Appearance |
|---|---|
| | All conditions |
| 13 | Colorless, slightly opalescent, free of visible protein particles |
| 14 | Colorless, significantly opalescent, free of visible protein particles |
| 15 | Colorless, slightly opalescent, free of visible protein particles |
| 16 | Colorless, slightly opalescent, free of visible protein particles |
| 17 | Colorless, significantly opalescent, free of visible protein particles |
| 18 | Colorless, slightly opalescent, free of visible protein particles |

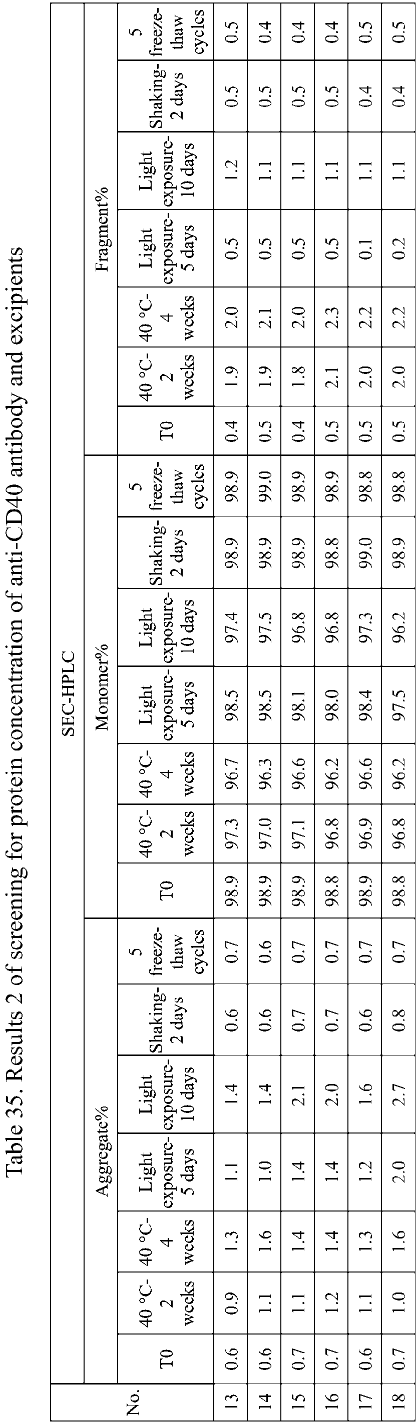

**Table 36. Results 3 of screening for protein concentration of anti-CD40 antibody and excipients**

| No. | NRCE | | | | | | | |
|---|---|---|---|---|---|---|---|---|
| | Main peak% | | | | Fragment% | | | |
| | T0 | 40 °C-4 weeks | Light exposure-5 days | Light exposure-10 days | T0 | 40 °C-4 weeks | Light exposure-5 days | Light exposure-10 days |
| 13 | 96.8 | 93.8 | 96.0 | 95.7 | 3.2 | 6.0 | 3.5 | 3.7 |
| 14 | 96.6 | 93.9 | 96.2 | 95.6 | 3.4 | 5.8 | 3.5 | 3.8 |
| 15 | 96.5 | 93.4 | 95.9 | 95.2 | 3.5 | 6.0 | 3.6 | 3.8 |
| 16 | 96.6 | 93.3 | 95.8 | 95.1 | 3.4 | 6.2 | 3.6 | 3.9 |
| 17 | 96.7 | 93.7 | 96.1 | 95.2 | 3.3 | 6.0 | 3.4 | 4.0 |
| 18 | 96.8 | 93.0 | 95.8 | 94.3 | 3.2 | 6.3 | 3.5 | 4.1 |

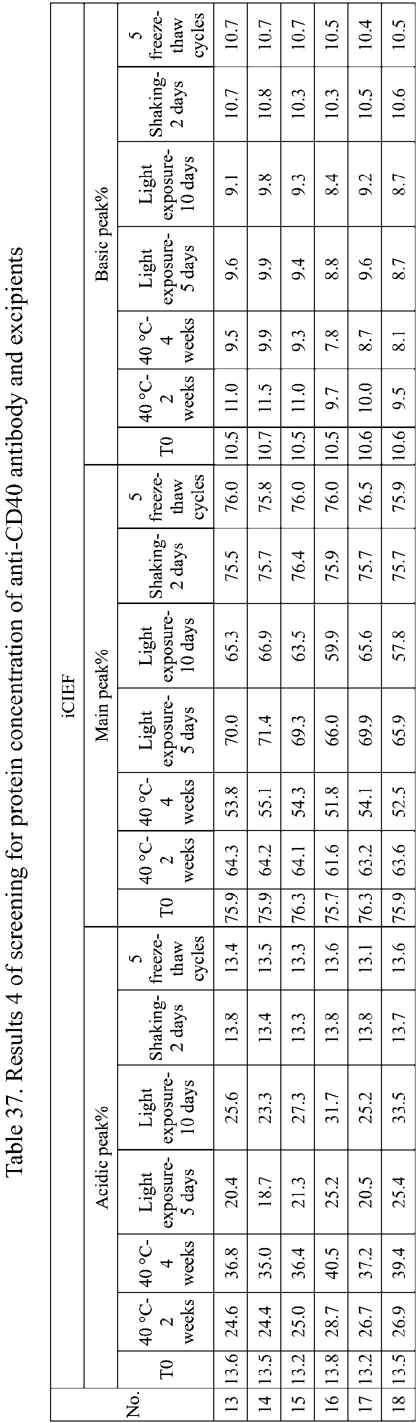

The results show that in terms of appearance, the formulas containing arginine hydrochloride were significantly opalescent. In terms of purity, the SEC purity of all formulas was slightly reduced only under the high temperature and light exposure conditions, and compared with T0, the increase proportion of aggregates was no more than 2%, with relatively small overall difference; under the high temperature condition, NRCE fragments were slightly increased, and the difference among the groups was not significant; the iCIEF purity changed under the high temperature and light exposure conditions, wherein under the high temperature condition, formula 1 exhibited slightly higher acidic peak content than formula 2, and formula 4 exhibited slightly higher acidic peak content than formula 5, with minor differences. In addition, different protein concentrations had no significant effect on the purity, indicating that 50 mg/mL-80 mg/mL anti-CD40 antibody has relatively good stability in the formulas of the 10 mM histidine-histidine hydrochloride, pH 6.0-pH 6.6 buffer systems, 8% (w/v) sucrose as a stabilizer, and 0.02% (w/v) polysorbate 80 as a surfactant.

### Example 24. Stabilizer Concentration Confirmation for Anti-CD40 Antibody Formulation

7.5% (w/v) sucrose, 20 mM histidine-histidine hydrochloride, and 0.02% (w/v) polysorbate 80 were selected to prepare an antibody formulation with an anti-CD40 antibody content of 80 mg/mL and a pH of 6.3, and stability studies were performed under the conditions of a high temperature of 40 °C and repeated freeze-thaw (-35 °C/room temperature) for 5 times.

19) 20 mM histidine-histidine hydrochloride, pH 6.3, 7.5% (w/v) sucrose, 0.02% (w/v) polysorbate 80, 80 mg/mL anti-CD40 antibody

**Table 38. confirmation results 1 of target pH and stabilizer concentration for anti-CD40 antibody formulation**

| No. | Condition | Sampling point | Appearance | pH |
|---|---|---|---|---|
| 19 | T0 | | Colorless, clear, free of visible protein particles | 6.32 |
| | Freeze-thaw | 5 cycles | Colorless, clear, free of visible protein particles | 6.40 |
| | 40°C | 2 weeks | Colorless, slightly opalescent, free of visible protein particles | 6.31 |
| | | 4 weeks | Colorless, slightly opalescent, free of visible protein particles | 6.32 |

**Table 39. confirmation results 2 of target pH and stabilizer concentration for anti-CD40 antibody formulation**

| No. | Condition | Sampling point | SEC | | | NRCE | | | iCIEF | | |
|---|---|---|---|---|---|---|---|---|---|---|---|
| | | | Monomer% | Aggregate% | Fragment% | Monomer% | Fragment% | Aggregate% | Acidic peak% | Main peak% | Basic peak% |
| 19 | T0 | | 98.9 | 1.1 | 0 | 96.3 | 3.7 | 0 | 23.9 | 67.3 | 8.8 |
| | Freeze-thaw | 5 cycles | 98.9 | 1.0 | 0 | NT | NT | NT | NT | NT | NT |
| | 40°C | 2 weeks | 96.5 | 1.6 | 1.9 | 94.7 | 5.3 | 0 | 34.3 | 57.0 | 8.7 |
| | | 4 weeks | 94.8 | 2.0 | 3.1 | 93.5 | 6.5 | 0 | 40.9 | 47.3 | 7.6 |

| | | | | | | | | | | | |
|---|---|---|---|---|---|---|---|---|---|---|---|
| Note: NT represents not tested, and since the repeated freeze-thaw temperature was relatively low and the time was relatively short, which had relatively little effect on the purity of NRCE and iCIEF, the purity of the NRCE and iCIEF was not tested; T0 represents time 0. | | | | | | | | | | | |

The results show that the formula exhibited no significant change in the appearance after 5 freeze-thaw cycles, and the clarity was changed from clarity to slight opalescence under the high temperature condition. The pH did not change significantly under all conditions. In terms of purity, after 5 freeze-thaw cycles, the SEC-HPLC purity was not significantly changed under the high temperature condition, and the SEC-HPLC aggregates were slightly increased, the NRCE fragments were increased by about 2.8% compared with T0, and the increase of the iCIEF acidic peak was within an acceptable range, indicating that the formula has good freeze-thaw stability and thermal stability.

### Example 25. pH Confirmation for Anti-CD40 Antibody Formulation

The 20 mM histidine-histidine hydrochloride, pH 6.1 buffer system, 7.5% (w/v) sucrose, and 0.02% (w/v) polysorbate 80 were selected to prepare an antibody formulation with an anti-CD40 antibody content of 80 mg/mL, and stability studies were performed under the conditions of a high temperature of 40 °C, repeated freeze-thaw (-35 °C/room temperature) for 5 times, and the like.

20) 20 mM histidine-histidine hydrochloride, pH 6.1, 7.5% (w/v) sucrose, 0.02% (w/v) polysorbate 80, 80 mg/mL anti-CD40 antibody

**Table 40. pH confirmation results 1 for anti-CD40 antibody formulation**

| No. | Condition | Sampling point | pH | Appearance |
|---|---|---|---|---|
| 20 | T0 | | 6.14 | Colorless, slightly opalescent, free of visible protein particles |
| | Freeze-thaw | 5 cycles | 6.15 | Colorless, slightly opalescent, free of visible protein particles |
| | 40°C | 2 weeks | 6.09 | Colorless, slightly opalescent, free of visible protein particles |
| | | 1 month | 6.10 | Colorless, slightly opalescent, free of visible protein particles |

**Table 41. pH confirmation results 2 for anti-CD40 antibody formulation**

| No. | Conditi on | Sampling point | SEC | | NRCE | | iCIEF | | |
|---|---|---|---|---|---|---|---|---|---|
| | | | Monomer % | Aggregate % | Monomer % | Fragment % | Acidic peak% | Main peak% | Basic peak% |
| 20 | T0 | | 98.7 | 1.2 | 97.5 | 2.5 | 24.0 | 69.3 | 6.7 |
| | Freeze-thaw | 3 cycles | 98.8 | 1.2 | 97.5 | 2.5 | 24.5 | 69.1 | 6.4 |
| | | 5 cycles | 98.8 | 1.2 | 97.6 | 2.4 | 24.3 | 69.3 | 6.5 |
| | 40°C | 2 weeks | 96.9 | 1.7 | 97.3 | 2.7 | 33.8 | 60.4 | 5.8 |
| | | 4 weeks | 94.6 | 2.3 | 91.8 | 8.2 | 54.9 | 40.6 | 4.5 |

| | | | | | | | | | |
|---|---|---|---|---|---|---|---|---|---|
| Note: NT represents not tested, and T0 represents time 0. | | | | | | | | | |

The results show that the formula had no significant change in pH and appearance after 5 freeze-thaw cycles and storage at the high temperature for 1 month. In terms of purity, after 5 freeze-thaw cycles, all the purities had no significant change; after storage at the high temperature for 4 weeks, the SEC-HPLC aggregates were slightly increased compared with T0, the NRCE purity was more than 91%, and the iCIEF main peak was still more than 40%, indicating that the pH formula has good freeze-thaw stability and thermal stability.

### Example 26. Anti-CD40 Antibody Concentration Screening

The 20 mM histidine-histidine hydrochloride, pH 6.1 buffer system, 7.5% (w/v) sucrose, and 0.02% (w/v) polysorbate 80 were selected to prepare different concentrations of anti-CD40 antibody formulations, and the viscosity was measured.

**Table 42. Viscosity results of anti-CD40 antibodies**

| No. | Actual protein concentration | Viscosity |
|---|---|---|
| 21 | 81.6 mg/mL | NT |
| 22 | 131.2 mg/mL | 19.6cp |
| 23 | 189.0 mg/mL | 114.0cp |

| | | |
|---|---|---|
| Note: NT represents not tested | | |

The results show that the higher the protein concentration, the higher the formula viscosity, and the viscosity approached 20 cp when the protein concentration reached 131.2 mg/mL by using sucrose as a stabilizer.

### Example 27. Anti-CD40 Antibody Concentration Screening

The 20 mM histidine-histidine hydrochloride, pH 6.1 buffer system, 2.5% (w/v) proline, and 0.02% (w/v) polysorbate 80 were selected to prepare different concentrations of anti-CD40 antibody formulations, and the viscosity was measured.

**Table 43. Viscosity results of anti-CD40 antibodies**

| No. | Actual protein concentration | Viscosity |
|---|---|---|
| 24 | 187 mg/mL | 18.6cp |
| 25 | 207 mg/mL | 40.9cp |

The results show that the higher the protein concentration, the higher the formula viscosity, and the viscosity still did not exceed 20 cp when the protein concentration reached 187 mg/mL by using proline as a stabilizer.

### Example 28. Excipient Type Screening

The 20 mM histidine-histidine hydrochloride, pH 6.1 buffer system, 0.02% (w/v) polysorbate 80 were selected and 7.5% (w/v) sucrose or 2.5% (w/v) proline was used as a stabilizer to prepare antibody formulations with anti-CD40 antibody contents of 80, 120, and 150 mg/mL respectively, and the stability was investigated under the conditions of storage at a high temperature (40 °C) for 2 weeks and 4 weeks, storage under the light exposure (4500±500 lx/25 °C) for 5 days, shaking (300 rpm/25 °C) for 2 days, 5 freeze-thaw cycles (-35 °C/room temperature), storage at 2-8 °C for 4 weeks, and freezing (-35 °C) for 4 weeks.
26) 20 mM histidine-histidine hydrochloride, pH 6.1, 7.5% (w/v) sucrose, 0.02% (w/v) polysorbate 80, 80 mg/mL anti-CD40 antibody
27) 20 mM histidine-histidine hydrochloride, pH 6.1, 7.5% (w/v) sucrose, 0.02% (w/v) polysorbate 80, 120 mg/mL anti-CD40 antibody
28) 20 mM histidine-histidine hydrochloride, pH 6.1, 2.5% (w/v) proline, 0.02% (w/v) polysorbate 80, 150 mg/mL anti-CD40 antibody

**Table 44. Results 1 of screening for anti-CD40 antibody excipients**

| No. | Appearance (all investigation conditions) | pH (time 0) |
|---|---|---|
| 26 | Pale yellow, slightly opalescent, free of visible protein particles | 6.1 |
| 27 | Pale yellow, slightly opalescent, free of visible protein particles | 6.2 |
| 28 | Pale yellow, slightly opalescent, free of visible protein particles | 6.2 |

**Table 45. Results 2 of screening for anti-CD40 antibody excipients**

| No. | Insoluble microparticles (particle/mL) | | | | | | | | | | | |
|---|---|---|---|---|---|---|---|---|---|---|---|---|
| | Time 0 | | | | 5 freeze-thaw cycles | | | | Shaking for 2 days | | | |
| | 2-5 µm | 5-10 µm | 10-25 µm | > 25 µm | 2-5 µm | 5-10 µm | 10-25 µm | > 25 µm | 2-5 µm | 5-10 µm | 10-25 µm | > 25 µm |
| 26 | 56 | 4 | 6 | 2 | 112 | 11 | 7 | 2 | 249 | 29 | 6 | 4 |
| 27 | 24 | 16 | 12 | 2 | 99 | 16 | 17 | 0 | 376 | 38 | 4 | 2 |
| 28 | 829 | 82 | 20 | 4 | 1376 | 92 | 5 | 2 | 1030 | 122 | 15 | 0 |

**Table 46. Results 3 of screening for anti-CD40 antibody excipients**

| No. | SEC-HPLC | | | | | | | | | | | |
|---|---|---|---|---|---|---|---|---|---|---|---|---|
| | Aggregate% | | | | Monomer% | | | | Fragment% | | | |
| | Time 0 | 5 freeze-thaw cycles | Shaking for 2 days | Light exposure for 5 days | Time 0 | 5 freeze-thaw cycles | Shaking for 2 days | Light exposure for 5 days | Time 0 | 5 freeze-thaw cycles | Shaking for 2 days | Light exposure for 5 days |
| 26 | 2.0 | 2.0 | 2.0 | 2.4 | 97.2 | 97.2 | 97.2 | 96.9 | 0.8 | 0.9 | 0.8 | 0.7 |
| 27 | 2.1 | 2.1 | 2.3 | 3.3 | 97.1 | 97.2 | 97.0 | 95.8 | 0.7 | 0.7 | 0.7 | 0.8 |
| 28 | 2.1 | 2.1 | 2.2 | 3.5 | 97.1 | 97.1 | 97.2 | 95.7 | 0.7 | 0.7 | 0.7 | 0.8 |

**Table 47. Results 4 of screening for anti-CD40 antibody excipients**

| No. | SEC-HPLC | | | | | | | | | | | |
|---|---|---|---|---|---|---|---|---|---|---|---|---|
| | Aggregate% | | | | Monomer% | | | | Fragment% | | | |
| | Time 0 | 40 °C-4 weeks | 2-8 °C-4 weeks | -35 °C-4 weeks | Time 0 | 40 °C-4 weeks | 2-8 °C-4 weeks | -35 °C-4 weeks | Time 0 | 40 °C-4 weeks | 2-8 °C-4 weeks | -35 °C-4 weeks |
| 26 | 2.0 | 2.6 | 2.0 | 1.9 | 97.2 | 95.2 | 97.3 | 97.4 | 0.8 | 2.2 | 0.8 | 0.7 |
| 27 | 2.1 | 3.0 | 2.1 | 2.0 | 97.1 | 95.0 | 97.2 | 97.3 | 0.7 | 2.1 | 0.7 | 0.8 |
| 28 | 2.1 | 3.2 | 2.1 | 2.0 | 97.1 | 94.6 | 97.2 | 97.4 | 0.7 | 2.1 | 0.7 | 0.6 |

**Table 48. Results 5 of screening for anti-CD40 antibody excipients**

| No. | iCIEF | | | | | | | | |
|---|---|---|---|---|---|---|---|---|---|
| | Acidic peak% | | | Main peak% | | | Basic peak% | | |
| | Time 0 | Light exposure for 5 days | 40 °C-4 weeks | Time 0 | Light exposure for 5 days | 40 °C-4 weeks | Time 0 | Light exposure for 5 days | 40 °C-4 weeks |
| 26 | 22.4 | 29.9 | 42.9 | 71.8 | 64.6 | 51.4 | 5.8 | 5.5 | 5.8 |
| 27 | 22.0 | 30.2 | 41.6 | 72.2 | 64.6 | 52.8 | 5.8 | 5.2 | 5.6 |
| 28 | 22.0 | 29.2 | 40.8 | 72.2 | 65.5 | 53.7 | 5.8 | 5.3 | 5.6 |

**Table 49. Results 6 of screening for anti-CD40 antibody excipients**

| No. | NRCE | | | | | | RCE | | |
|---|---|---|---|---|---|---|---|---|---|
| | Main peak% | | | Fragment% | | | LC+HC% | | |
| | Time 0 | Light exposure for 5 days | 40 °C-4 weeks | Time 0 | Light exposure for 5 days | 40 °C-4 weeks | Time 0 | Light exposure for 5 days | 40 °C-4 weeks |
| 26 | 96.9 | 95.6 | 94.7 | 3.1 | 4.4 | 5.3 | 99.4 | 98.4 | 97.7 |
| 27 | 97.1 | 96.4 | 95.0 | 2.9 | 3.4 | 5.0 | 99.5 | 98.3 | 98.2 |
| 28 | 96.9 | 96.1 | 94.3 | 3.1 | 3.4 | 5.7 | 99.5 | 98.3 | 98.4 |

**Table 50. Results 7 of screening for anti-CD40 antibody excipients**

| No. | Binding activity% | | |
|---|---|---|---|
| | Time 0 | Light exposure for 5 days | 40 °C-4 weeks |
| 26 | 102 | 99 | 101 |
| 27 | 94 | 95 | 95 |
| 28 | 97 | 98 | 102 |

The results show that under all conditions, all formulas exhibited a pale yellow appearance and slight opalescence and were free of visible protein particles, and the binding activities were all within the range of 60%-140%; no significant difference was observed among the groups in terms of insoluble microparticles, SEC purity, NRCE, and RCE purity groups; in terms of iCIEF purity, under the high temperature condition, formula 28 was optimal, followed by formulas 27 and 26, and the three formulas had no significant difference, indicating that the above 3 formulas have good appearance and good stability under the conditions of high temperature, light exposure, shaking, freeze-thaw, and freezing.

### Example 29. Alternative Anti-CD40 Antibody Formulas

The present disclosure provides anti-CD40 antibody pharmaceutical formulations with a formula "50-150 mg/mL anti-CD40 antibody, 7.5%-8% (w/v) sucrose or 2.5% (w/v) proline, 0.02% (w/v) polysorbate 80, 20 mM histidine-histidine hydrochloride, pH 5.6-6.6", including but not limited to:
(1) 80 mg/mL anti-CD40 antibody, 7.5% (w/v) sucrose, 0.02% (w/v) polysorbate 80, 20 mM histidine-histidine hydrochloride, pH 6.3;
(2) 50 mg/mL anti-CD40 antibody, 7.5% (w/v) sucrose, 0.02% (w/v) polysorbate 80, 20 mM histidine-histidine hydrochloride, pH 6.3;
(3) 80 mg/mL anti-CD40 antibody, 8% (w/v) sucrose, 0.02% (w/v) polysorbate 80, 20 mM histidine-histidine hydrochloride, pH 6.3;
(4) 50 mg/mL anti-CD40 antibody, 8% (w/v) sucrose, 0.02% (w/v) polysorbate 80, 20 mM histidine-histidine hydrochloride, pH 6.3;
(5) 80 mg/mL anti-CD40 antibody, 7.5% (w/v) sucrose, 0.02% (w/v) polysorbate 80, 20 mM histidine-histidine hydrochloride, pH 6.0;
(6) 80 mg/mL anti-CD40 antibody, 7.5% (w/v) sucrose, 0.02% (w/v) polysorbate 80, 20 mM histidine-histidine hydrochloride, pH 6.6;
(7) 50 mg/mL anti-CD40 antibody, 7.5% (w/v) sucrose, 0.02% (w/v) polysorbate 80, 20 mM histidine-histidine hydrochloride, pH 6.0;
(8) 50 mg/mL anti-CD40 antibody, 7.5% (w/v) sucrose, 0.02% (w/v) polysorbate 80, 20 mM histidine-histidine hydrochloride, pH 6.6;
(9) 80 mg/mL anti-CD40 antibody, 8% (w/v) sucrose, 0.02% (w/v) polysorbate 80, 20 mM histidine-histidine hydrochloride, pH 6.0;
(10) 80 mg/mL anti-CD40 antibody, 8% (w/v) sucrose, 0.02% (w/v) polysorbate 80, 20 mM histidine-histidine hydrochloride, pH 6.6;
(11) 50 mg/mL anti-CD40 antibody, 8% (w/v) sucrose, 0.02% (w/v) polysorbate 80, 20 mM histidine-histidine hydrochloride, pH 6.0;
(12) 50 mg/mL anti-CD40 antibody, 8% (w/v) sucrose, 0.02% (w/v) polysorbate 80, 20 mM histidine-histidine hydrochloride, pH 6.6;
(13) 80 mg/mL anti-CD40 antibody, 7.5% (w/v) sucrose, 0.02% (w/v) polysorbate 80, 20 mM histidine-histidine hydrochloride, pH 6.1;
(14) 50 mg/mL anti-CD40 antibody, 7.5% (w/v) sucrose, 0.02% (w/v) polysorbate 80, 20 mM histidine-histidine hydrochloride, pH 6.1;
(15) 80 mg/mL anti-CD40 antibody, 8% (w/v) sucrose, 0.02% (w/v) polysorbate 80, 20 mM histidine-histidine hydrochloride, pH 6.1;
(16) 50 mg/mL anti-CD40 antibody, 8% (w/v) sucrose, 0.02% (w/v) polysorbate 80, 20 mM histidine-histidine hydrochloride, pH 6.1;
(17) 120 mg/mL anti-CD40 antibody, 7.5% (w/v) sucrose, 0.02% (w/v) polysorbate 80, 20 mM histidine-histidine hydrochloride, pH 6.1;
(18) 120 mg/mL anti-CD40 antibody, 7.5% (w/v) sucrose, 0.02% (w/v) polysorbate 80, 20 mM histidine-histidine hydrochloride, pH 6.2;
(19) 120 mg/mL anti-CD40 antibody, 7.5% (w/v) sucrose, 0.02% (w/v) polysorbate 80, 20 mM histidine-histidine hydrochloride, pH 6.3;
(20) 150 mg/mL anti-CD40 antibody, 2.5% (w/v) proline, 0.02% (w/v) polysorbate 80, 20 mM histidine-histidine hydrochloride, pH 6.1;
(21) 150 mg/mL anti-CD40 antibody, 2.5% (w/v) proline, 0.02% (w/v) polysorbate 80, 20 mM histidine-histidine hydrochloride, pH 6.2;
(22) 150 mg/mL anti-CD40 antibody, 2.5% (w/v) proline, 0.02% (w/v) polysorbate 80, 20 mM histidine-histidine hydrochloride, pH 6.3.

The experimental results show that anti-CD40 antibody formulations of the above formulas have good stability; the above formulas can be applied to the preparation of anti-CD40 antibody drugs.

### Example 30. Formula Investigation for Anti-CD40-ADC Formulation

The 20 mM histidine-histidine hydrochloride, pH 6.1 buffer system, 7.5% (w/v) sucrose, and 0.02% (w/v) polysorbate 80 were selected to prepare a formula of CD40-ADC (the ADC-2 in Example 18 of the present disclosure, the same as in the following examples) with the concentration of 50 mg/mL. The stability of the liquid formulation was investigated under the conditions of different temperatures (-35 °C, 2-8 °C, 25 °C, and 40 °C), light exposure (5000±500 lx, 25 °C), shaking (300 rpm, 25 °C), and repeated freeze-thaw (-35 °C/room temperature). The liquid formulation was lyophilized, and the stability of the freeze-dried powder was investigated under the condition of 50 °C.

29) 20 mM histidine-histidine hydrochloride, pH 6.1 buffer system, 7.5% (w/v) sucrose, 0.02% (w/v) polysorbate 80, 50 mg/mL anti-CD40-ADC

**Table 51. Anti-CD40-ADC formula investigation results 1**

| No. | Appearance | | | | |
|---|---|---|---|---|---|
| | T0 | 25 °C-2 weeks/4 weeks | 40 °C-2 weeks/4 weeks | 2-8 °C-2 weeks/4 weeks | -35 °C-4 weeks |
| 29 | Colorless, slightly opalescent, free of visible protein particles | Colorless, slightly opalescent, free of visible protein particles | Colorless, slightly opalescent, free of visible protein particles | Colorless, slightly opalescent, free of visible protein particles | Colorless, slightly opalescent, free of visible protein particles |

| No. | Appearance | | | | |
|---|---|---|---|---|---|
| | Light exposure-10 days | Shaking-3 days | 3 or 5 freeze-thaw cycles | Lyo-T0 | Lyo-50 °C-2 weeks/4 weeks (after reconstitution) |
| 29 | Colorless, slightly opalescent, free of visible protein particles | Colorless, slightly opalescent, free of visible protein particles | Colorless, slightly opalescent, free of visible protein particles | Colorless, slightly opalescent, free of visible protein particles | Colorless, slightly opalescent, free of visible protein particles |

| No. | Appearance of freeze-dried powder (Lyo-T0, 50 °C-2 weeks/4 weeks) | | | | |
|---|---|---|---|---|---|
| 29 | Off-white block, full without collapses | | | | |

| | | | | | |
|---|---|---|---|---|---|
| Note: Lyo T0 represents a sample at time 0 after lyophilization. | | | | | |

**Table 52. Anti-CD40-ADC formula investigation results 2**

| No. | SEC purity | | | | | | | | | | | | | | |
|---|---|---|---|---|---|---|---|---|---|---|---|---|---|---|---|
| | Aggregate% | | | | | Monomer% | | | | | Fragment% | | | | |
| | T0 | Shaking-3 days | Light exposure-10 days | 3 freeze-thaw cycles | 5 freeze-thaw cycles | T0 | Shaking-3 days | Light exposure-10 days | 3 freeze-thaw cycles | 5 freeze-thaw cycles | T0 | Shaking-3 days | Light exposure-10 days | 3 freeze-thaw cycles | 5 freeze-thaw cycles |
| 29 | 2.0 | 2.2 | 3.7 | 1.7 | 2.0 | 97.3 | 97.0 | 95.5 | 97.2 | 97.1 | 0.7 | 0.8 | 0.8 | 1.1 | 0.9 |

| No. | Aggregate% | | | | | Monomer% | | | | | Fragment% | | | | |
|---|---|---|---|---|---|---|---|---|---|---|---|---|---|---|---|
| | 2-8 °C-2 weeks | 2-8 °c-4 weeks | 25 °C-2 weeks | 25 °C-4 weeks | 40 °C-2 weeks | 2-8 °C-2 weeks | 2-8 °C-4 weeks | 25 °C-2 weeks | 25 °C-4 weeks | 40 °C-2 weeks | 2-8 °C-2 weeks | 2-8 °C-4 weeks | 25 °C-2 weeks | 25 °C-4 weeks | 40 °C-2 weeks |
| 29 | 2.2 | 2.2 | 2.6 | 2.6 | 3.9 | 97.1 | 97.0 | 96.7 | 96.4 | 94.7 | 0.7 | 0.8 | 0.8 | 0.9 | 1.5 |

| 29 No. | SEC purity | | | | | | | | | | | | | | |
|---|---|---|---|---|---|---|---|---|---|---|---|---|---|---|---|
| | Aggregate% | | | | | Monomer% | | | | | Fragment% | | | | |
| | 40 °C-4 weeks | -35 °C-4 weeks | Lyo-T0 | Lyo-50 °C-2 weeks | Lyo-50 °C-4 weeks | 40 °C-4 weeks | -35 °C-4 weeks | Lyo-T0 | Lyo-50 °C-2 weeks | Lyo-50 °C-4 weeks | 40 °C-4 weeks | -35 °C-4 weeks | Lyo-T0 | Lyo-50 °C-2 weeks | Lyo-50 °C-4 weeks |
| 2 | 4.5 | 1.9 | 2.0 | 2.7 | 2.9 | 93.6 | 97.3 | 97.3 | 96.6 | 96.3 | 1.9 | 0.8 | 0.7 | 0.7 | 0.7 |

**Table 53. Anti-CD40-ADC formula investigation results 3**

| No. | NRCE purity | | | | | | | | | | |
|---|---|---|---|---|---|---|---|---|---|---|---|
| | T0 | 3 freeze-thaw cycles | 5 freeze-thaw cycles | -35 °C-4 weeks | 2-8 °C-4 weeks | 25 °C-4 weeks | 40 °C-4 weeks | -35 °C-8 weeks | Lyo-T0 | Lyo-50 °C-2 weeks | Lyo-50 °C-4 weeks |
| 2 | 98.2 | 98.0 | 98.0 | 98.1 | 97.7 | 97.4 | 96.4 | 97.8 | 98.1 | 98.0 | 98.1 |

| | | | | | | | | | | | |
|---|---|---|---|---|---|---|---|---|---|---|---|
| Note: Lyo T0 represents a sample at time 0 after lyophilization. | | | | | | | | | | | |

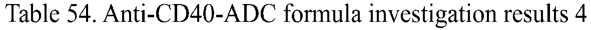

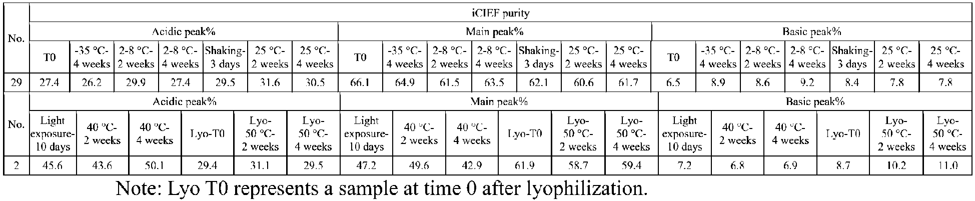

**Table 55. Anti-CD40-ADC formula investigation results 5**

| Group | DAR | | | | | | | | | |
|---|---|---|---|---|---|---|---|---|---|---|
| | T0 | 3 freeze-thaw cycles | 5 freeze-thaw cycles | 2-8 °C-4 weeks | 25 °C-4 weeks | 40 °C-4 weeks | -35 °C-4 weeks | Lyo-T0 | Lyo-50 °C-2 weeks | Lyo-50 °C-4 weeks |
| 2 | 4.1 | 4.1 | 4.1 | 4.1 | 4.1 | 4.1 | 4.1 | 4.1 | 4.1 | 4.1 |

The results show that the solution and the freeze-dried powder of the anti-CD40-ADC formula have good stability under different stability investigation conditions.

### Example 31. Alternative Anti-CD40-ADC Formulas

The present disclosure provides anti-CD40-ADC formulations with a formula "30-50 mg/mL anti-CD40-ADC, 7.5%-9% (w/v) sucrose or 7.5% (w/v) trehalose, 0.02% (w/v) polysorbate 80, 20 mM histidine-histidine hydrochloride, pH 6.0-6.6", including but not limited to:
(1) 50 mg/mL anti-CD40 ADC, 7.5% (w/v) sucrose, 0.02% (w/v) polysorbate 80, 20 mM histidine-histidine hydrochloride, pH 6.3;
(2) 50 mg/mL anti-CD40 ADC, 7.5% (w/v) sucrose, 0.02% (w/v) polysorbate 80, 20 mM histidine-histidine hydrochloride, pH 6.0;
(3) 50 mg/mL anti-CD40 ADC, 7.5% (w/v) sucrose, 0.02% (w/v) polysorbate 80, 20 mM histidine-histidine hydrochloride, pH 6.6;
(4) 30 mg/mL anti-CD40 ADC, 7.5% (w/v) sucrose, 0.02% (w/v) polysorbate 80, 20 mM histidine-histidine hydrochloride, pH 6.3;
(5) 50 mg/mL anti-CD40 ADC, 9% (w/v) sucrose, 0.02% (w/v) polysorbate 80, 20 mM histidine-histidine hydrochloride, pH 6.3;
(6) 50 mg/mL anti-CD40 ADC, 7.5% (w/v) trehalose, 0.02% (w/v) polysorbate 80, 20 mM histidine-histidine hydrochloride, pH 6.3;

The experimental results show that anti-CD40 ADC formulations of the above formulas have good stability; the above formulas can be applied to the preparation of anti-CD40-ADC drugs.

Although specific embodiments of the present disclosure have been described above, it will be appreciated by those skilled in the art that these embodiments are merely illustrative and that many changes or modifications can be made to these embodiments without departing from the principles and spirit of the present disclosure.

## Claims

1. A pharmaceutical composition, comprising a CD40-binding molecule and a buffer, wherein the buffer is selected from the group consisting of an acetate buffer, a citrate buffer, a histidine salt buffer, and a phosphate buffer; preferably, the buffer is a histidine salt buffer; more preferably, the buffer is a histidine-hydrochloride buffer or a histidineacetate buffer; most preferably, the buffer is a histidine-histidine hydrochloride buffer.

2. The pharmaceutical composition according to claim 1, wherein the CD40-binding molecule is an anti-CD40 antibody or an antigen-binding fragment thereof.

3. The pharmaceutical composition according to claim 2, wherein the anti-CD40 antibody or the antigen-binding fragment thereof comprises a heavy chain variable region (VH) and a light chain variable region (VL), wherein:
the VH comprises a HCDR1, a HCDR2, and a HCDR3 in a VH set forth in SEQ ID NO: 1, and the VL comprises a LCDR1, a LCDR2, and a LCDR3 in a VL set forth in SEQ ID NO: 2;
wherein the CDRs are defined according to the Kabat, IMGT, Chothia, AbM, or Contact numbering scheme, preferably the Kabat numbering scheme.

4. The pharmaceutical composition according to claim 2 or 3, wherein the anti-CD40 antibody or the antigen-binding fragment thereof comprises:
a heavy chain HCDR1, a heavy chain HCDR2, and a heavy chain HCDR3, comprising the sequences set forth in SEQ ID NOs: 3, 4, and 5, respectively; and a light chain LCDR1, a light chain LCDR2, and a light chain LCDR3, comprising the sequences set forth in SEQ ID NOs: 6, 7, and 19, respectively;
preferably, the anti-CD40 antibody or the antigen-binding fragment thereof comprises: a heavy chain HCDR1, a heavy chain HCDR2, and a heavy chain HCDR3, comprising the sequences set forth in SEQ ID NOs: 3, 4, and 5, respectively; a light chain LCDR1, comprising the sequence set forth in SEQ ID NO: 6; a light chain LCDR2, comprising the sequence set forth in SEQ ID NO: 7; and a light chain LCDR3, comprising the sequence set forth in any one of SEQ ID NOs: 15-18.

5. The pharmaceutical composition according to any one of claims 2 to 4, wherein the anti-CD40 antibody or the antigen-binding fragment thereof is a recombinant antibody, a rabbit antibody, a chimeric antibody, a humanized antibody, a fully human antibody, or an antigen-binding fragment thereof.

6. The pharmaceutical composition according to claim 5, wherein:
heavy chain framework regions of the humanized antibody or the antigen-binding fragment thereof are derived from IGHV2-26*01, IGHV4-30-4*02, IGHV4-4*08, and IGHJ1*01, and/or light chain framework regions are derived from IGkV1-13*02, IGkV1-9*01, IGkV1-6*01, and IGKJ4*01;
preferably, heavy chain framework regions FR1-FR3 are derived from IGHV2-26*01, IGHV4-30-4*02, or IGHV4-4*08, and a heavy chain framework region FR4 is derived from IGHJ1*01; light chain framework regions FR1-FR3 are derived from IGkV1-13*02, IGkV1-9*01, or IGkV1-6*01, and a light chain framework region FR4 is derived from IGKJ4*01.

7. The pharmaceutical composition according to any one of claims 2 to 6, wherein the anti-CD40 antibody or the antigen-binding fragment thereof comprises:
A-1) the amino acid sequence of the VH being set forth in SEQ ID NO: 13 or having at least 90% identity thereto, and the amino acid sequence of the VL being set forth in any one of SEQ ID NOs: 9-12 or having at least 90% identity thereto;
A-2) the amino acid sequence of the VH being set forth in SEQ ID NO: 14 or having at least 90% identity thereto, and the amino acid sequence of the VL being set forth in any one of SEQ ID NOs: 9-12 or having at least 90% identity thereto;
A-3) the amino acid sequence of the VH being set forth in SEQ ID NO: 1 or having at least 90% identity thereto, and the amino acid sequence of the VL being set forth in SEQ ID NO: 2 or having at least 90% identity thereto.

8. The pharmaceutical composition according to any one of claims 2 to 7, wherein the anti-CD40 antibody or the antigen-binding fragment thereof further comprises an Fc region of an IgG antibody; preferably, the IgG antibody is an IgG1, IgG2, or IgG4 antibody; more preferably, the Fc region is an Fc region of IgG1 comprising mutation N297A, or an Fc region of IgG1 comprising any one or more of mutations L234A, L235A, M252Y, S254T, and T256E.

9. The pharmaceutical composition according to any one of claims 2 to 8, wherein the antigen-binding fragment is an scFv, Fv, Fab, or Fab' fragment.

10. The pharmaceutical composition according to any one of claims 2 to 9, wherein the anti-CD40 antibody or the antigen-binding fragment thereof comprises:
the full-length amino acid sequence of a heavy chain being set forth in SEQ ID NO: 20 or 22 or having at least 90% identity thereto, and the full-length amino acid sequence of a light chain being set forth in SEQ ID NO: 21 or having at least 90% identity thereto.

11. The pharmaceutical composition according to claim 1, wherein the CD40-binding molecule is an antibody-drug conjugate comprising the anti-CD40 antibody or the antigen-binding fragment thereof according to any one of claims 2 to 10.

12. The pharmaceutical composition according to claim 11, wherein the antibody-drug conjugate has a structure represented by formula (I):
Ab-(L-D)ₖ (I);
wherein Ab is the anti-CD40 antibody or the antigen-binding fragment thereof according to any one of claims 2 to 8; D is selected from the group consisting of a glucocorticoid receptor agonist, a glucocorticoid, a calcineurin inhibitor, and a target of rapamycin (mTOR) kinase inhibitor; L is a linker covalently linking Ab to D; k is an integer or decimal selected from the group consisting of 1-20.

13. The pharmaceutical composition according to claim 12, wherein D in the antibody-drug conjugate is:

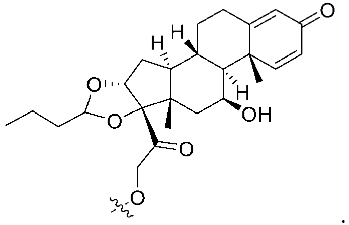

14. The pharmaceutical composition according to any one of claims 11 to 13, wherein the antibody-drug conjugate has the following structure: 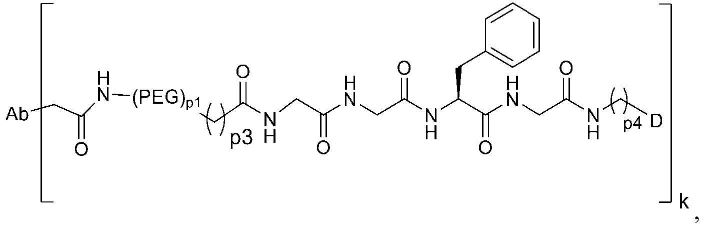
wherein k is an integer or decimal selected from the group consisting of 1-10; p1 is selected from the group consisting of 2, 4, 6, and 8; p3 and p4 are each independently selected from the group consisting of 0, 1, and 2;
preferably, the antibody-drug conjugate has the following structure: 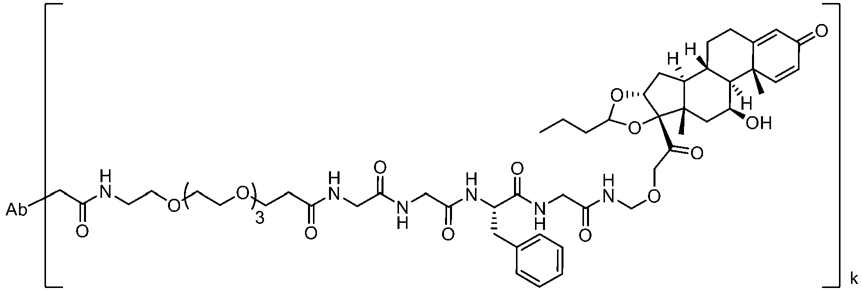
wherein k is an integer or decimal selected from the group consisting of 1-10; more preferably, the antibody-drug conjugate has the following structure: 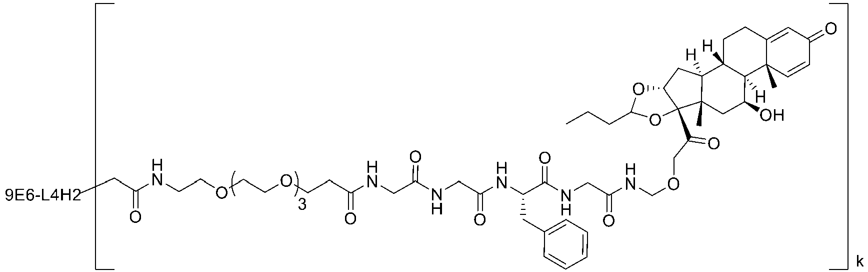
wherein 9E6-L4H2 is an anti-CD40 antibody comprising: a heavy chain and a light chain comprising the amino acid sequences set forth in SEQ ID NOs: 20 and 21, respectively; k is an integer or decimal selected from the group consisting of 1-10.

15. The pharmaceutical composition according to any one of claims 1 to 14, wherein the composition has a pH of 4.5-7.5, preferably 5-7.2, more preferably 5.5-7, and most preferably 5.6-6.6.

16. The pharmaceutical composition according to any one of claims 1 to 15, wherein the buffer is at a concentration of 1-50 mM, preferably 5-40 mM, more preferably 10-30 mM, and most preferably 15-25 mM.

17. The pharmaceutical composition according to any one of claims 1 to 16, further comprising a stabilizer selected from the group consisting of one or more of an amino acid or a pharmaceutically acceptable salt thereof and a sugar, wherein preferably, the amino acid is selected from the group consisting of one or more of arginine and proline or pharmaceutically acceptable salts thereof, and the sugar is selected from the group consisting of one or more of sucrose and trehalose; more preferably, the stabilizer is selected from the group consisting of one or more of sucrose, trehalose, arginine or a pharmaceutically acceptable salt thereof, and proline or a pharmaceutically acceptable salt thereof; most preferably, the stabilizer is sucrose, trehalose, or proline or a pharmaceutically acceptable salt thereof.

18. The pharmaceutical composition according to claim 17, wherein sucrose or trehalose is at a concentration of 0.1%-30% w/v, preferably 0.5%-20% w/v, more preferably 1%-15% w/v, and most preferably 5%-10% w/v; or proline or the pharmaceutically acceptable salt thereof is at a concentration of 0.01%-20% w/v, preferably 0.1%-15% w/v, more preferably 0.5%-10% w/v, and most preferably 1%-5% w/v.

19. The pharmaceutical composition according to any one of claims 1 to 18, further comprising a surfactant, wherein the surfactant is preferably a polysorbate, more preferably one or more of polysorbate 20 and polysorbate 80, and most preferably polysorbate 80.

20. The pharmaceutical composition according to claim 19, wherein the surfactant is at a concentration of 0.001%-1% w/v, preferably 0.005%-1% w/v, more preferably 0.008%-0.2% w/v, and most preferably 0.01%-0.1% w/v.

21. The pharmaceutical composition according to any one of claims 2 to 10 and 15 to 20, wherein the anti-CD40 antibody or the antigen-binding fragment thereof is at a concentration of 0.1-500 mg/mL, preferably 1-300 mg/mL, more preferably 10-250 mg/mL, and most preferably 20-200 mg/mL.

22. The pharmaceutical composition according to any one of claims 11 to 20, wherein the antibody-drug conjugate is at a concentration of 0.1-300 mg/mL, preferably 1-150 mg/mL, more preferably 5-120 mg/mL, and most preferably 10-100 mg/mL.

23. A pharmaceutical composition, comprising any one of the following groups:
A) the anti-CD40 antibody or the antigen-binding fragment thereof according to any one of claims 2 to 10, or the antibody-drug conjugate according to any one of claims 11 to 14; a histidine-hydrochloride buffer, preferably histidine-histidine hydrochloride;
a polysorbate, preferably polysorbate 80;
sucrose, trehalose, and/or arginine or a pharmaceutically acceptable salt thereof;
B) the anti-CD40 antibody or the antigen-binding fragment thereof according to any one of claims 2 to 10, or the antibody-drug conjugate according to any one of claims 11 to 14; a histidine-hydrochloride buffer, preferably histidine-histidine hydrochloride;
a polysorbate, preferably polysorbate 80;
proline or a pharmaceutically acceptable salt thereof;
C) the anti-CD40 antibody or the antigen-binding fragment thereof according to any one of claims 2 to 10, or the antibody-drug conjugate according to any one of claims 11 to 14; a histidine-hydrochloride buffer, preferably histidine-histidine hydrochloride;
a polysorbate, preferably polysorbate 80;
sucrose;
D) the antibody-drug conjugate according to any one of claims 11 to 14;
a histidine-hydrochloride buffer, preferably histidine-histidine hydrochloride;
a polysorbate, preferably polysorbate 80;
trehalose.

24. A pharmaceutical composition, comprising any one of the following groups:
1) 0.1-300 mg/mL said anti-CD40 antibody or said antigen-binding fragment thereof according to any one of claims 2 to 10;
1-50 mM histidine-hydrochloride buffer, preferably histidine-histidine hydrochloride;
0.001%-1% w/v polysorbate, preferably polysorbate 80;
0.1%-30% w/v sucrose;
and the composition having a pH of 4.5-7.5;
2) 1-200 mg/mL said anti-CD40 antibody or said antigen-binding fragment thereof according to any one of claims 2 to 10;
5-40 mM histidine-hydrochloride buffer, preferably histidine-histidine hydrochloride;
0.005%-1% w/v polysorbate, preferably polysorbate 80;
0.5%-20% w/v sucrose;
and the composition having a pH of 5-7.2;
3) 10-150 mg/mL said anti-CD40 antibody or said antigen-binding fragment thereof according to any one of claims 2 to 10;
10-30 mM histidine-hydrochloride buffer, preferably histidine-histidine hydrochloride;
0.008%-0.2% w/v polysorbate, preferably polysorbate 80;
1%-15% w/v sucrose;
and the composition having a pH of 5.5-7;
4) 15-132 mg/mL or 20-100 mg/mL said anti-CD40 antibody or said antigen-binding fragment thereof according to any one of claims 2 to 10;
15-25 mM histidine-hydrochloride buffer, preferably histidine-histidine hydrochloride;
0.01%-0.1% w/v polysorbate, preferably polysorbate 80;
5%-10% w/v sucrose;
and the composition having a pH of 5.6-6.6;
5) 0.1-500 mg/mL said anti-CD40 antibody or said antigen-binding fragment thereof according to any one of claims 2 to 10;
1-50 mM histidine-hydrochloride buffer, preferably histidine-histidine hydrochloride;
0.001%-1% w/v polysorbate, preferably polysorbate 80;
0.01%-20% w/v proline or a pharmaceutically acceptable salt thereof;
and the composition having a pH of 4.5-7.5;
6) 1-300 mg/mL said anti-CD40 antibody or said antigen-binding fragment thereof according to any one of claims 2 to 10;
5-40 mM histidine-hydrochloride buffer, preferably histidine-histidine hydrochloride;
0.005%-1% w/v polysorbate, preferably polysorbate 80;
0.1%-15% w/v proline or a pharmaceutically acceptable salt thereof;
and the composition having a pH of 5-7.2;
7) 10-250 mg/mL said anti-CD40 antibody or said antigen-binding fragment thereof according to any one of claims 2 to 10;
10-30 mM histidine-hydrochloride buffer, preferably histidine-histidine hydrochloride;
0.008%-0.2% w/v polysorbate, preferably polysorbate 80;
0.5%-10% w/v proline or a pharmaceutically acceptable salt thereof;
and the composition having a pH of 5.5-7;
8) 20-200 mg/mL said anti-CD40 antibody or said antigen-binding fragment thereof according to any one of claims 2 to 10;
15-25 mM histidine-hydrochloride buffer, preferably histidine-histidine hydrochloride;
0.01%-0.1% w/v polysorbate, preferably polysorbate 80;
1%-5% w/v proline or a pharmaceutically acceptable salt thereof;
and the composition having a pH of 5.6-6.6.

25. A pharmaceutical composition, comprising any one of the following groups:
1) 0.1-300 mg/mL said antibody-drug conjugate according to any one of claims 11 to 14;
1-50 mM histidine-hydrochloride buffer, preferably histidine-histidine hydrochloride;
0.001%-1% w/v polysorbate, preferably polysorbate 80;
0.1%-30% w/v sucrose or trehalose;
and the composition having a pH of 4.5-7.5;
2) 1-150 mg/mL said antibody-drug conjugate according to any one of claims 11 to 14;
5-40 mM histidine-hydrochloride buffer, preferably histidine-histidine hydrochloride;
0.005%-1% w/v polysorbate, preferably polysorbate 80;
0.5%-20% w/v sucrose or trehalose;
and the composition having a pH of 5-7.2;
3) 5-120 mg/mL said antibody-drug conjugate according to any one of claims 11 to 14;
10-30 mM histidine-hydrochloride buffer, preferably histidine-histidine hydrochloride;
0.008%-0.2% w/v polysorbate, preferably polysorbate 80;
1%-15% w/v sucrose or trehalose;
and the composition having a pH of 5.5-7;
4) 10-100 mg/mL said antibody-drug conjugate according to any one of claims 11 to 14;
15-25 mM histidine-hydrochloride buffer, preferably histidine-histidine hydrochloride;
0.01%-0.1% w/v polysorbate, preferably polysorbate 80;
5%-10% w/v sucrose or trehalose;
and the composition having a pH of 5.6-6.6.

26. The pharmaceutical composition according to claim 23 or 24, comprising any one of the following groups:
1) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 7.5% w/v sucrose;
and the composition having a pH of about 6.0;
2) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 7.5% w/v sucrose;
and the composition having a pH of about 6.1;
3) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 7.5% w/v sucrose;
and the composition having a pH of about 6.2;
4) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 7.5% w/v sucrose;
and the composition having a pH of about 6.3;
5) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 7.5% w/v sucrose;
and the composition having a pH of about 6.5;
6) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 7.5% w/v sucrose;
and the composition having a pH of about 6.6;
7) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 8% w/v sucrose;
and the composition having a pH of about 6.0;
8) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 8% w/v sucrose;
and the composition having a pH of about 6.1;
9) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 8% w/v sucrose;
and the composition having a pH of about 6.2;
10) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 8% w/v sucrose;
and the composition having a pH of about 6.3;
11) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 8% w/v sucrose;
and the composition having a pH of about 6.5;
12) about 50 mg/mL, about 70 mg/mL, about 80 mg/mL, about 90 mg/mL, about 100 mg/mL, about 110 mg/mL, about 120 mg/mL, or about 130 mg/mL said anti-CD40 antibody or said antigen-binding fragment thereof according to any one of claims 2 to 10; about 20 mM histidine-hydrochloride buffer, preferably histidine-histidine hydrochloride;
about 0.02% w/v polysorbate 80;
about 8% w/v sucrose;
and the composition having a pH of about 6.6;
13) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL said anti-CD40 antibody or said antigen-binding fragment thereof according to any one of claims 2 to 10;
about 20 mM histidine-hydrochloride buffer, preferably histidine-histidine hydrochloride;
about 0.02% w/v polysorbate 80;
about 2.5% w/v proline;
and the composition having a pH of about 6.0;
14) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL said anti-CD40 antibody or said antigen-binding fragment thereof according to any one of claims 2 to 10;
about 20 mM histidine-hydrochloride buffer, preferably histidine-histidine hydrochloride;
about 0.02% w/v polysorbate 80;
about 2.5% w/v proline;
and the composition having a pH of about 6.1;
15) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL said anti-CD40 antibody or said antigen-binding fragment thereof according to any one of claims 2 to 10;
about 20 mM histidine-hydrochloride buffer, preferably histidine-histidine hydrochloride;
about 0.02% w/v polysorbate 80;
about 2.5% w/v proline;
and the composition having a pH of about 6.2;
16) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL said anti-CD40 antibody or said antigen-binding fragment thereof according to any one of claims 2 to 10;
about 20 mM histidine-hydrochloride buffer, preferably histidine-histidine hydrochloride;
about 0.02% w/v polysorbate 80;
about 2.5% w/v proline;
and the composition having a pH of about 6.3;
17) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL said anti-CD40 antibody or said antigen-binding fragment thereof according to any one of claims 2 to 10;
about 20 mM histidine-hydrochloride buffer, preferably histidine-histidine hydrochloride;
about 0.02% w/v polysorbate 80;
about 2.5% w/v proline;
and the composition having a pH of about 6.5;
18) about 50 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL said anti-CD40 antibody or said antigen-binding fragment thereof according to any one of claims 2 to 10;
about 20 mM histidine-hydrochloride buffer, preferably histidine-histidine hydrochloride;
about 0.02% w/v polysorbate 80;
about 2.5% w/v proline;
and the composition having a pH of about 6.6.

27. The pharmaceutical composition according to claim 23 or 25, comprising any one of the following groups:
1) about 20 mg/mL, about 30 mg/mL, about 40 mg/mL, about 50 mg/mL, about 60 mg/mL, about 70 mg/mL, or about 80 mg/mL said antibody-drug conjugate according to any one of claims 11 to 14;
about 20 mM histidine-hydrochloride buffer, preferably histidine-histidine hydrochloride;
about 0.02% w/v polysorbate 80;
about 7.5% w/v or about 9% w/v sucrose;
and the composition having a pH of about 6.0;
2) about 20 mg/mL, about 30 mg/mL, about 40 mg/mL, about 50 mg/mL, about 60 mg/mL, about 70 mg/mL, or about 80 mg/mL said antibody-drug conjugate according to any one of claims 11 to 14;
about 20 mM histidine-hydrochloride buffer, preferably histidine-histidine hydrochloride;
about 0.02% w/v polysorbate 80;
about 7.5% w/v or about 9% w/v sucrose;
and the composition having a pH of about 6.3;
3) about 20 mg/mL, about 30 mg/mL, about 40 mg/mL, about 50 mg/mL, about 60 mg/mL, about 70 mg/mL, or about 80 mg/mL said antibody-drug conjugate according to any one of claims 11 to 14;
about 20 mM histidine-hydrochloride buffer, preferably histidine-histidine hydrochloride;
about 0.02% w/v polysorbate 80;
about 7.5% w/v or about 9% w/v sucrose;
and the composition having a pH of about 6.6;
4) about 20 mg/mL, about 30 mg/mL, about 40 mg/mL, about 50 mg/mL, about 60 mg/mL, about 70 mg/mL, or about 80 mg/mL said antibody-drug conjugate according to any one of claims 11 to 14;
about 20 mM histidine-hydrochloride buffer, preferably histidine-histidine hydrochloride;
about 0.02% w/v polysorbate 80;
about 7.5% w/v trehalose;
and the composition having a pH of about 6.0;
5) about 20 mg/mL, about 30 mg/mL, about 40 mg/mL, about 50 mg/mL, about 60 mg/mL, about 70 mg/mL, or about 80 mg/mL said antibody-drug conjugate according to any one of claims 11 to 14;
about 20 mM histidine-hydrochloride buffer, preferably histidine-histidine hydrochloride;
about 0.02% w/v polysorbate 80;
about 7.5% w/v trehalose;
and the composition having a pH of about 6.3;
6) about 20 mg/mL, about 30 mg/mL, about 40 mg/mL, about 50 mg/mL, about 60 mg/mL, about 70 mg/mL, or about 80 mg/mL said antibody-drug conjugate according to any one of claims 11 to 14;
about 20 mM histidine-hydrochloride buffer, preferably histidine-histidine hydrochloride;
about 0.02% w/v polysorbate 80;
about 7.5% w/v trehalose;
and the composition having a pH of about 6.6.

28. A freeze-dried formulation, wherein the freeze-dried formulation is capable of forming the pharmaceutical composition according to any one of claims 1 to 27 upon reconstitution, or the freeze-dried formulation is obtained by lyophilizing the pharmaceutical composition according to any one of claims 1 to 27.

29. A reconstituted solution, wherein the reconstituted solution is prepared by reconstituting the freeze-dried formulation according to claim 28.

30. An article of manufacture, comprising a container, wherein the container contains the pharmaceutical composition according to any one of claims 1 to 27, the freeze-dried formulation according to claim 28, or the reconstituted solution according to claim 29.

31. Use of the pharmaceutical composition according to any one of claims 1 to 27, the freeze-dried formulation according to claim 28, or the reconstituted solution according to claim 29 in the manufacture of a medicament for treating an autoimmune disease, wherein the autoimmune disease is preferably Sjögren's syndrome, multiple sclerosis, or lupus erythematosus, more preferably systemic lupus erythematosus.

32. Use of the pharmaceutical composition according to any one of claims 1 to 27, the freeze-dried formulation according to claim 28, or the reconstituted solution according to claim 29 in the manufacture of a medicament for treating graft-versus-host disease or alleviating graft rejection, wherein
the graft is preferably a solid organ graft, more preferably a liver, kidney, heart, or lung graft.
